# EUROPEAN PATENT APPLICATION

(11) **EP 4 309 666 A1**
(43) Date of publication of application: **24.01.2024**
(21) Application number: 22186231.1
(22) Date of filing: 21.07.2022
(51) Int. Cl.: A61K 38/19, A61K 38/20, A61P 31/12, A61K 35/28

(54) **M-CSF FOR USE IN THE PROPHYLAXIS AND/OR TREATMENT OF VIRAL INFECTIONS IN STATES OF IMMUNOSUPPRESSION**

(71) Applicant: Technische Universität Dresden, 01069 Dresden (DE); Centre National de la Recherche Scientifique (CNRS), 75016 Paris (FR); Université d'Aix-Marseille, 13007 Marseille (FR); Institut National de la Santé et de la Recherche Médicale (INSERM), 75013 Paris (FR)
(72) Inventor: SIEWEKE, Michael, 01307 Dresden (DE); KANDALLA, Prashanth Kumar, 517325 Madanapalle (IN); ENGLMEIER, Ludwig, 83080 Oberaudorf (DE)
(74) Representative: Maikowski & Ninnemann Patentanwälte Partnerschaft mbB

(57) **Abstract**

The present invention relates to an agonist of the colony stimulating factor 1 receptor (CSF1R) for use in the prophylaxis and/or treatment of viral infections in states of immunosuppression in a subject and to pharmaceutical compositions comprising said CSF1R agonist. According to the invention, said subject is in a state of immunosuppression, when in said subject the absolute number of CD14+, CD16+ und HLA-DR+ expressing monocytes is reduced when compared to a healthy control subject. The invention is based on the finding that CSF1R agonist treatment, such as M-CSF treatment, induces an integrated multistep differentiation cascade that culminated in increased NK cell activation protecting HCT recipients from a viral infection. A mechanism of action by which M-CSF-induced myelopoiesis can rapidly reconstitute antiviral activity under immunosuppressed conditions was identified, which provides a general paradigm to boost innate antiviral immune response.

## Description

### Field of the invention

The present invention relates to an agonist of the colony stimulating factor 1 receptor (CSF1R) for use in the prophylaxis and/or treatment of viral infections in states of immunosuppression in a subject and to pharmaceutical compositions comprising said CSF1R agonist. The invention is based on the finding that CSF1R agonist treatment, such as M-CSF treatment, induces an integrated multistep differentiation cascade that culminates in increased NK cell activation, protecting individuals in states of immunosuppression from a viral infection, for example leukopenic subjects after hematopoietic cell transplantation. A mechanism of action by which M-CSF-induced myelopoiesis can rapidly reconstitute antiviral activity under immunosuppressed conditions was identified, which provides a general paradigm to boost innate antiviral immune response.

### Background of the invention

Immunosuppressed patients are highly susceptible to viral infections. Therapies reconstituting autologous antiviral immune competence could therefore represent an important protective measure. Viral infections, such as reactivation of latent cytomegalovirus (CMV), are a major cause of morbidity and mortality in leukopenic patients, for example after hematopoietic stem cell transplantation (HCT).

Allogenic HCT is carried out in about 16000 patients in Europe / year. Despite treatment and clinical protocol improvements about 15% of patients still risk to die from infections after HCT. Even if not fatal, the treatment of infections incurs enormous extra costs for the health care system. For example, in the case of CMV infection, antiviral medication and longer hospitalization result in $58,000 to $74,000 spent per patient within the first 6 months after HCT (Jain et al, 2014).

The outcome and severity of disease during a viral infection depends to a large extent on the immune status of the infected individual. Immunosuppressed patients have a high risk of viral infection and virus-induced morbidity, in particular under conditions of leukopenia. A prominent and clinically important example is the immune ablation occurring during HCT (Arber et al.). Viral infection is a particular serious complication of this procedure, for which effective treatments are lacking (Ljungman et al.; Locatelli et al.). For HCT recipients, no effective vaccines are available against predominant opportunistic viruses causing severe disease, such as Cytomegalovirus (CMV) (Plotkin). Moreover, current antiviral treatments based on inhibition of viral replication can have significant bone marrow toxicity that impedes donor cell engraftment and hematopoietic reconstitution (Ahmed; Boeckh and Ljungman; Cho et al.; El Chaer et al.). Biologics stimulating antiviral immunity of the patient are currently not available. Myeloid cytokines, such as G-CSF, have been used to improve neutropenia by stimulating myeloid progenitor proliferation and activating mature myeloid cells to improve neutropenia (Heuser et al.). However, no effects of such treatments on antiviral immunity have been reported. This appeared consistent with the fact that antiviral immunity is mainly mediated by natural killer (NK) cell and T-cell mediated cytotoxicity rather than by myeloid cells. Viruses that cause frequent viral complications after HCT are herpesviridae, such as Cytomegalovirus (CMV) and Epstein-Barr virus (EBV), adenovirus (ADV) and respiratory viruses (Ogonek et al.).

In addition the following table, published by Chatzidimitriou et al, gives an overview on further viral infections in HCT and the so far limited therapy options.

**TABLE I. Characteristics of Emerging Viral Infections in Hematopoietic Transplant Recipients**

| Virus | Incidence | Clinical manifestations | Laboratory diagnosis | Antiviral agents |
|---|---|---|---|---|
| HMPV (paramyxovirus) | Up to 5% in HCT recipients | URI, pneumonia | PCR techniques; culture/FA rapid culture: research | Ribavirin |
| HBoV (parvovirus) | Limited data; ≈ 5% in immunocompromised patients | Limited data; fever, pneumonia | PCR techniques; culture: research | Not available |
| HCoV-NL63 HCoV-HKU1 (coronaviruses) | Limited data; 8.8% in immunocompromised patients | Limited data; pneumonia | PCR techniques; culture: research | Not available |
| HHV-6 (betaherpesvirus) | 33-48% in HCT recipients | Skin rash, fever, acute GVHD, myelosuppression, pneumonitis, encephalitis | PCR techniques; antigen-antibody detection tests: available | Foscarnet, ganciclovir, cidofovir |
| HHV-7 (betaherpesvirus) | 10-57% in HCT recipients | Limited data; GVHD,CNS disease | PCR techniques, antigen detection tests | Foscarnet, ganciclovir |
| BK virus (polyomavirus) | Viremia: 30% in HCT recipients | Hemorrhagic cystitis, nephropathy | PCR techniques, culture, electron microscopy, immunohistochemistry, cytology, histology | Cidofovir, leflunomide |
| JC virus (polyomavirus) | No data available | PML (14 cases in HCT recipients) | PCR techniques, histology | Cidofovir |
| KI virus/WU virus (polyomaviruses) | Limited data; KIV: 8%; WUV: 1% in immunocopromised patients | Respiratory pathogens? | PCR techniques | Not available |

| | | | | |
|---|---|---|---|---|
| HMPV, human metapneumovirus; URI, upper respiratory infection; HCT, hematopoietic cell transplantation; FA, fluorescent antibody; PCR, polymerase chain reaction; HBoV, human boca virus; HCoV, human coronavirus; HHV-6, human herpes virus-6; GVHD, graft versus host disease; HHV-7, human herpes virus-7; PML, progressive multifocal leukoencephalopathy, CNS; central nervous system. | | | | |

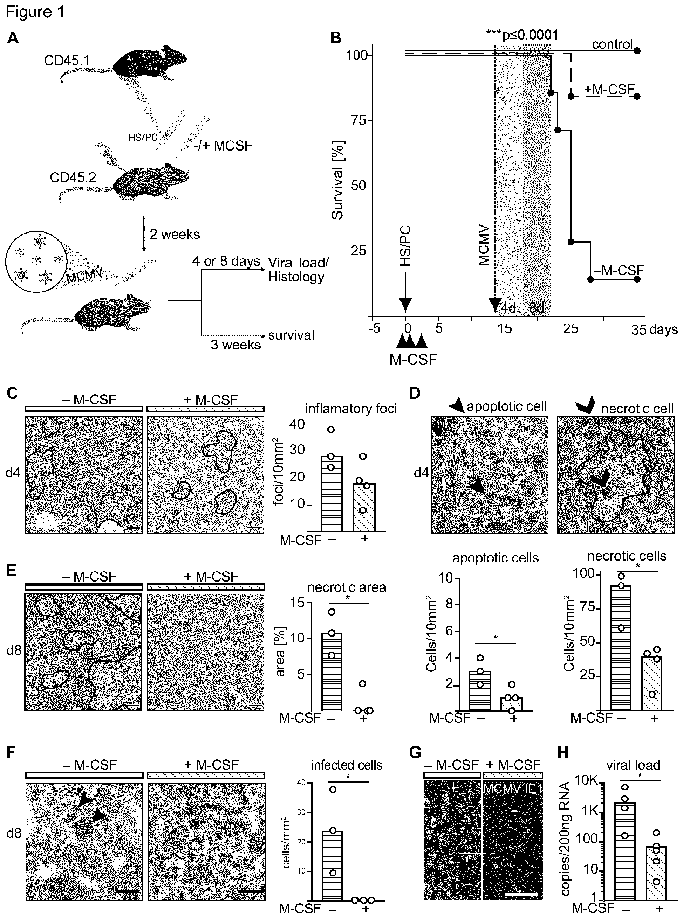

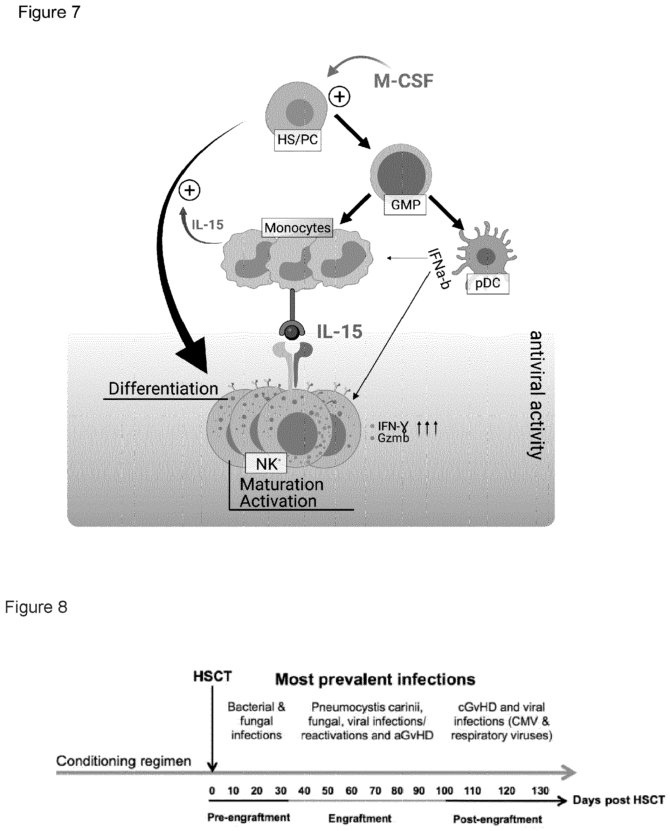

Moreover, Ogonek et al. report a time line of complications after allogeneic HCT. Figure 8 (Fig. 1 in Ogonek et al.) shows the most prevalent complications after hematopoietic stem cell transplantation (HCT), which occur according to the three phases of engraftment. Concomitant infectious complications consisting of bacterial, fungal, and viral infections are shown according to their occurrence as well as association with acute and chronic graft-versus-host disease (GvHD) during different phases of follow-up: (1) pre-engraftment, (2) engraftment, and (3) post-engraftment phase. The infections encountered during the pre-engraftment phase consist primarily of bacterial and fungal infections that are reasonably well controlled by medications given for prophylaxis and treatment, albeit at the risk of emerging multidrug resistance of nosocomial strains and bone marrow toxicity of antifungals. The first 100 days after HCT (engraftment phase) are characterized by cellular immunodeficiencies due to a reduced number of natural killer (NK) cells of the innate immune system and T cells of the adaptive immune system. This renders patients especially susceptible to reactivation of latent viruses including cytomegalovirus (CMV) and Epstein-Barr virus (EBV) as well as to new viral infections.

Abbreviations: CMV, cytomegalovirus; aGvHD, acute graft-versus-host disease; cGvHD, chronic graft-versus-host disease.

CMV belongs to the family of herpesviruses and is one among the viral pathogens that can reactivate after HCT. It reactivates in about 60-70% of CMV-seropositive patients, and a primary infection affects 20-30% of CMV seronegative recipients transplanted from CMV-seropositive donors. Uncontrolled CMV reactivations and infections can lead to a life-threatening, multi-organ CMV disease such as retinitis, gastroenteritis, or pneumonia. Early reconstitution of antiviral immunity remains an essential issue for the control of CMC reactivations after HCT (Ogonek et al.).

CMV leads to a diverse range of infection-associated pathologies in humans (Tabeta et al.). Mouse CMV (MCMV) is a closely related natural pathogen in mice with similar cellular tropism and kinetics (Alexandre et al.; Krmpotic et al.). It is therefore broadly used as a model for studying immune responses against CMV. The spleen is an early site for filtering blood-borne virus and initiating immune responses, and there is viral replication in the organ. The liver is a key site of viral infection, and viral replication at this site can be observed at times after its decline in the spleen (Hsu et al.). Type I interferons (Baranek et al.) constitute a first line of defense against CMV, with plasmacytoid dendritic cells (pDCs) being the major producer (Dalod et al.; Zucchini et al.). On the cellular level, antiviral defense is mediated by lymphoid cells, with NK cells and T-cells coming in as a critical second and third wave of the immune response that can block viral replication by killing infected cells (Orange and Biron).

Cytokines released during an infection can massively alter hematopoietic output (Boettcher and Manz 2017). For example, M-CSF is such a cytokine that increases during infections (Cheers and Stanley; Roth et al.; Mossadegh-Keller et al.) and can induce myelopoiesis by promoting bone marrow progenitor cells to proliferate and differentiate into mature monocytes and macrophages (Metcalf; Motoyoshi; Ushach and Zlotnik, 2016). Furthermore, it was reported previously that M-CSF acts directly on hematopoietic stem cells (HSC) to induce emergency hematopoiesis and increased production of myeloid cells (Mossadegh-Keller et al.). Importantly, in concert with the myeloid transcription factor MafB, M-CSF selectively controls asymmetric myeloid commitment division (Sarrazin et al.; Sarrazin and Sieweke). As a consequence, M-CSF stimulates myeloid cell production without exhausting HSC numbers (Mossadegh-Keller et al.) (Kandalla et al.). It was further demonstrated that this property had therapeutic benefit in improving protection against bacterial and fungal infections after transplantation of hematopoietic stem and progenitor cells (HCT) (Kandalla et al.). Antiviral activities of M-CSF, however, have not been reported so far. This is not surprising, because fungal and bacterial infections occur at an earlier stage in the timeline of infections after allogenic HCT than viral infections (Fig. 8 and Ogonek et al.) and the components of the immune system involved in pathogen defense in these stages are different.

M-CSF activates the CSF-1 receptor (CSF1R). Another activator of the CSF1R is interleukin-34 (IL-34). Accordingly, M-CSF and IL-34 are agonists of CSF1R. The CSF1R plays important roles in development and in innate immunity by regulating the development, homeostasis and fucntion of macrophages including specialized tissue macrophage populations. Owing to this expression pattern in macrophages and its progenitors and the broad presence of macrophages in essentially all tissues including in the central nervous system and tumors, it plays a central role in neoplastic, inflammatory, and neurological diseases. The evolution, structure, and regulation of expression of the CSF1R gene, the structures of CSF-1, IL-34, and the CSF1R as well as the mechanism of ligand binding to and activation of the receptor are known. There are pathways regulating macrophage survival, proliferation, differentiation, and chemotaxis downstream from the CSF1R (Stanley and Chitu).

### Summary of the invention

As discussed above, immunouppressed patients have a high risk of viral infection and virus-induced morbidity, in particular under conditions of leukopenia. A prominent and clinically important example is the immune ablation occurring during HCT (Arber et al.). Viral infection is a particular serious complication of this procedure, for which effective treatments are lacking or have adverse side effects.

Accordingly, there is a great need for such treatments which can preferably be used in the prophylaxis of viral infections in an immunosuppressed subject. It is thus the problem of the invention to provide effective agents for the prophylaxis and/or treatment of viral infections in states of immunosuppression.

The invention solves this problem by providing a CSF1R agonist for use in the prophylaxis and/or treatment of viral infections in states of immunosuppression.

Said viral infection may be an activation of a latent virus in a subject. Furthermore, said viral infection may be an accidental infection, for example an accidential infection with a respiratory virus. Furthermore, said viral infection may be derived from the transfer of donor material to a subject, e.g. by from transfer of cells during HCT, in particular, from a donor that had been diagnosed as having a latent virus infection.

The inventors have specifically investigated the role of emergency hematopoiesis on CMV infection under leukopenic conditions and have surprisingly found that the CSF1R agonist M-CSF induced myelopoiesis promoted rapid reconstitution of antiviral activity and protection from CMV infection. Using a model of HCT (hematopoietic stem cells/progenitor cells) transplantation and infection with lethal doses of CMV in mice, it was observed that M-CSF treatment had anti-viral activity and resulted in substantially improved survival and pathogen clearance in infected mice. Dissecting the mechanism underlying this M-CSF mediated protection against murine CMV infection, a multistep differentiation cascade was identified, in which M-CSF-induced myelopoiesis further stimulated NK cell differentiation and activation via IL-15 and IFN-I mediators.

The invention further provides a pharmaceutical composition for use in the prophylaxis and/or treatment of viral infections in states of immunosuppression, comprising a CSFR1 agonist.

### Brief description of the drawings

**Figure 1** shows that M-CSF protects HCT graft recipients from CMV viremia and mortality.
A) Leukopenia model to study CMV viremia. Lethally irradiated CD45.2 mice were transplanted with 3,000 CD45.1 HCT, treated by repeated intravenous injections of control PBS or M-CSF during transplantation (-1h, +5h, +18h), challenged after 2 weeks by intra-peritoneal (i.p.) injection of 5,000 PFU MCMV and analyzed for viral load or for survival.
B) Survival of mice after HCT transplantation (arrow), CMV infection (stippled arrow) and treatment (arrowheads) with control PBS (-M-CSF; n=12) or three doses of 10µg mouse recombinant M-CSF (+M-CSF; n=11). Transplanted, not infected mice (n=5) are shown as control.
C-E) Histopathology of CMV induced hepatitis.
C) Assessment of inflammatory foci in livers 4 days after infection of transplanted mice treated with M-CSF or control PBS. Example of Hematoxylin and eosin (H&E)-staining and inflammatory foci (n=4)
D) Examples of apoptotic (arrowheads) and necrotic (arrows) hepatocytes and quantification as median cell numbers per area (n=4).
E) Assessment of necrotic area in 8 days after infection of transplanted mice treated with M-CSF or control PBS. Example of Hematoxylin and eosin (H&E)-staining and percentage of affected areas (n=4).
F-H) Assessment of viral load in the liver 8 days after CMV infection of transplanted mice treated with M-CSF or control PBS.
F) Histological analysis of H&E staining. Examples of infected hepatocytes indicated by arrowheads and quantification by median cell numbers per area (n=4).
G) Immunofluorescence staining of MCMV E1 protein.
H) RT-qPCR quantitation of viral mRNA per 200ng RNA (n=5).
^{∗∗∗}P=<0.0001 by Mantel-Cox test (B). ^{∗}P=<0.05 by two-tailed non-parametric Mann-Whitney U-test (C-G). All data are representative of at least two independent experiments.

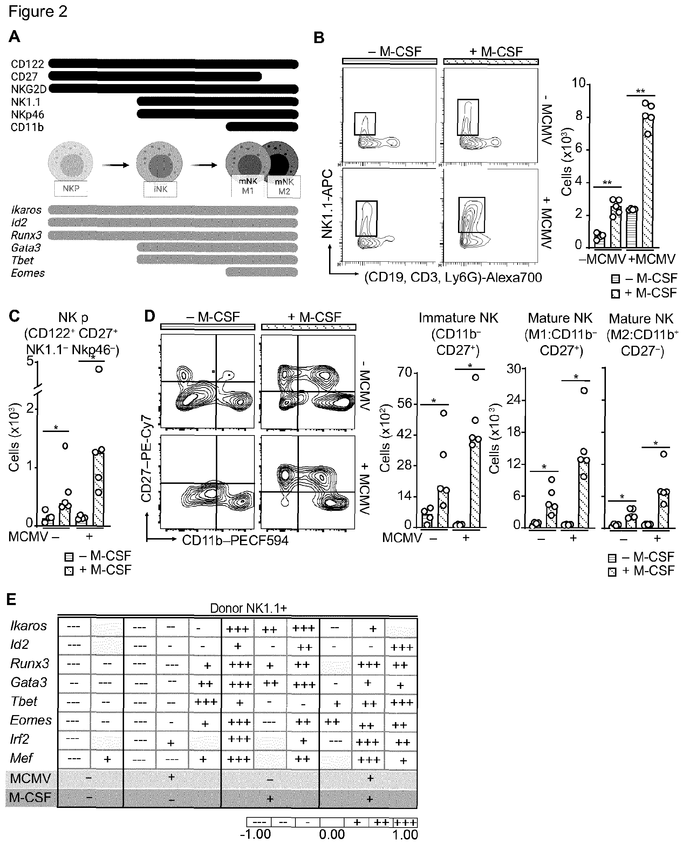

**Figure 2** shows that M-CSF treatment increases NK cell production, differentiation and activation. Analysis of spleen NK cell populations without or 1.5 days after CMV infection and 2 weeks after HCT transplantation and PBS control or M-CSF treatment.
A) Markers specific to differentiation and maturation stages of NK cells used in this analysis.
B) FACS examples and median of absolute number of total NK cells (CD19⁻, CD3⁻, Ly6G⁻ , NK1.1⁺)
C) Median of absolute number of donor-derived NK progenitor cells (CD122⁺, CD27⁺, NK1.1⁻, Nkp46⁻, CD45.1⁺)
D) FACS examples and median of absolute numbers of donor-derived immature NK cells, donor-derived mature NK M1 cells (CD11b⁺ CD27⁺) and donor-derived mature NK M2 cells (CD11b⁺ CD27⁻).
E) Gene expression analysis of NK cell expressed transcription factors in FACS sorted donor-derived NK1.1⁺ NK cells (definitions of Fig.2A) by nanofluidic Fluidigm array real-time PCR.
^{∗}P=<0.05, ^{∗∗}P=<0.01 by two-tailed non-parametric Mann-Whitney U-test. All data are representative of two independent experiments.

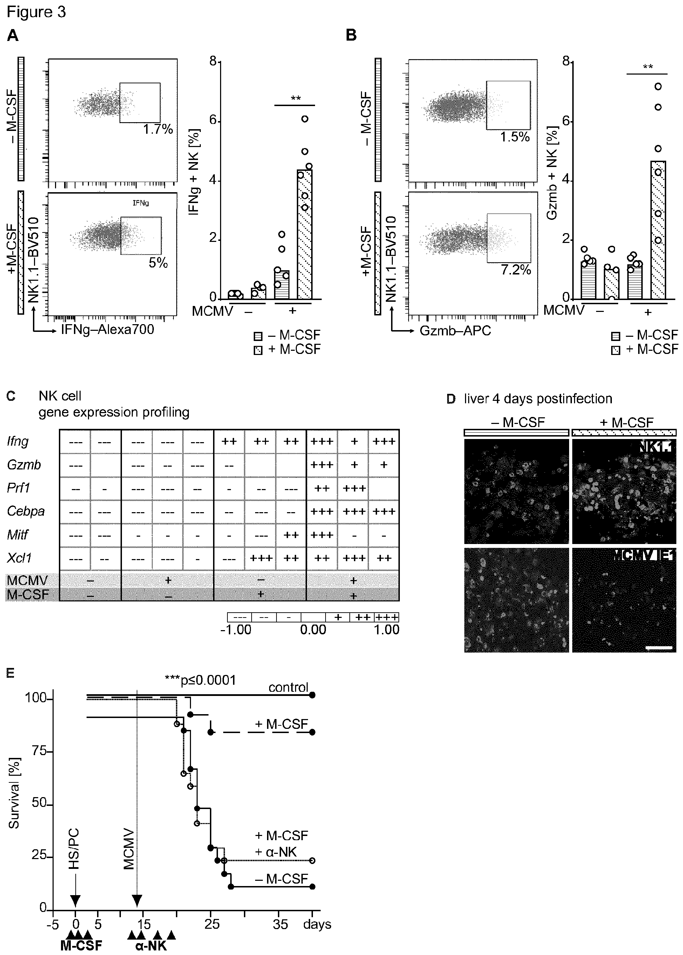

**Figure 3** shows that NK cell activity is required for the antiviral effect of M-CSF.
A-C) Analysis of NK cell activity in the spleen without or 1.5 days after CMV infection and 2 weeks after HCT transplantation and PBS control or M-CSF treatment.
A) FACS examples and median of percentage of donor-derived NK cells producing IFN-γ.
B) FACS examples and median of percentage of donor-derived NK cells producing Granzyme B.
C) Gene expression analysis of activation and maturation related factors in FACS sorted donor derived NK1.1⁺ NK cells by real-time PCR.
D) Immunofluorescence analysis with anti-NK1.1 and anti-MCMV IE1 antibodies in liver of HCT and M-CSF or control PBS treated mice 4 days after CMV infection.
E) Analysis of NK cell requirement for M-CSF antiviral effect. Survival of PBS control (n=15), M-CSF and control IgG treated (n=12), M-CSF and anti-NK1.1 treated (n=17) or transplanted, non-infected control mice (n=4). Mice were HCT transplanted (solid arrow), control PBS or M-CSF treated (black arrowheads), infected with MCMV (stippled arrow) as described in Fig.1A,B and repeatedly treated with anti-NK1.1 antibody before and after infection (-1d, +1d, +3d, +5d).
^{∗∗}P=<0.01 by two-tailed non-parametric Mann-Whitney U-test (A, B), ^{∗∗∗}P=<0.0001 by Mantel-Cox test (E). All data are representative of two independent experiments.

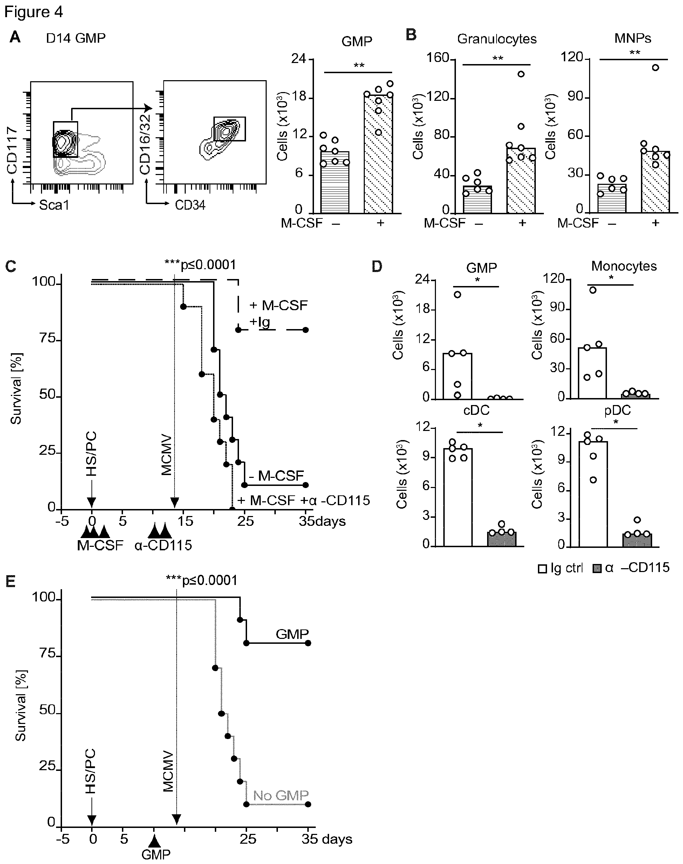

**Figure 4** shows that M-CSF induced myelopoiesis is required for its antiviral effect.
A) FACS example and median of absolute number of granulocyte monocyte/macrophage progenitors (GMP) in the spleen of PBS control or M-CSF-treated uninfected recipient mice 14 days after transplantation.
B) Median of absolute number of granulocytes (Ly6G⁺, CD11b⁺) and mononuclear phagocytes (Ly6G⁻, CD11b⁺) in the spleen of PBS control or M-CSF-treated uninfected recipient mice 14 days after transplantation.
C) Analysis of requirement of M-CSF dependent myeloid cells for antiviral effect. Survival curve of MCMV infected and PBS control (n=10), M-CSF and IgG control Ab treated (n=9) or M-CSF and anti-CD115 antibody treated mice (n=10). Mice were HCT transplanted (solid arrow), control PBS or M-CSF treated (black arrowheads), infected with MCMV (stippled arrow) as described in Fig.1A, B and treated twice with anti-CD115 antibody before infection (-2d, -1d,).
D) Median of absolute number of GMP, monocytes, cDC and pDC in the spleen of uninfected Ig control- or anti-CD 115 antibody-treated recipient mice two days after first treatment. ^{∗}P=<0.05 by two-tailed non-parametric Mann-Whitney U-test.
E) Analysis of GMP derived myeloid cells in antiviral effect. Survival of MCMV infected control (n=10) or GMP-transplanted mice (n=10). Mice were HCT transplanted (solid arrow), infected with MCMV (stippled arrow) as described in Fig.1A, B and GMP transplanted 10 days after HCT transplantation.
^{∗∗}P=<0.01 by two-tailed non-parametric Mann-Whitney U-test (A). ^{∗∗∗} P=<0.0001 by Mantel-Cox test (B, D). All data are representative of two independent experiments.

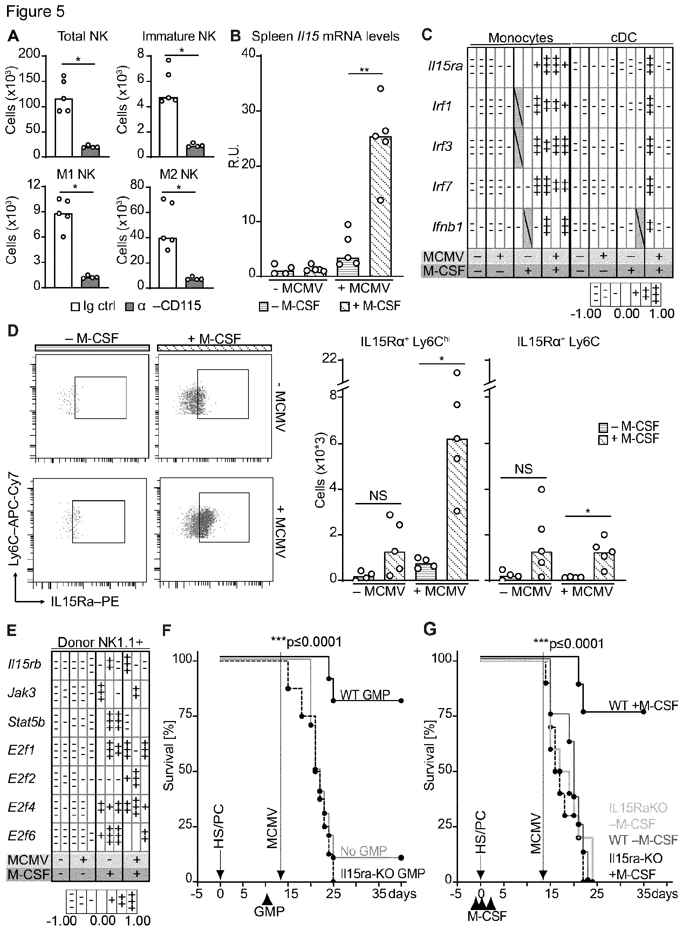

**Figure 5** shows that myeloid IL-15 trans-presentation is required for antiviral activity of M-CSF.
A) Median of absolute number of total NK1.1+, immature and mature (M1 and M2) NK cells in the spleen of uninfected Ig control or anti-CD115 antibody-treated recipient mice two days after Ab treatment and 2 weeks after HCT transplantation and control or M-CSF treatment.
B) Spleen *Il15* mRNA levels by RT-qPCR two weeks after HCT transplantation and control or M-CSF treatment without or 1.5 days after CMV infection.
C) Gene expression analysis of FACS-sorted donor-derived spleen monocytes and cDC (gating strategy Fig.S3C), two weeks after HCT transplantation and control or M-CSF treatment, without or 1.5 days after MCMV infection, for genes related to IL-15 induction and presentation by nanofluidic Fluidigm array RT-qPCR.
D) Representative FACS profiles and median of absolute number of IL-15Rα expressing donor-derived spleen Ly6C^{hi} or Ly6C^{low} monocytes two weeks after HCT transplantation and control or M-CSF treatment, without or 1.5 days after MCMV infection.
E) Gene expression analysis of IL-15 signaling and response genes in donor-derived spleen NK cells, two weeks after HCT transplantation and control or M-CSF treatment, without or 1.5 days after MCMV infection by nanofluidic Fluidigm array real-time PCR.
F) Analysis of myeloid IL-15 presentation in antiviral effect. Survival of MCMV infected control (n=10), WT GMP (n=10) or *Il15ra*-KO GMP-transplanted mice (n=9). Mice were HCT transplanted (solid arrow), infected with MCMV (stippled arrow) as described in Fig.1A, B and GMP transplanted 10 days after HCT transplantation (arrowhead).
G) Analysis of requirement of IL-15 presentation for M-CSF antiviral effect. Survival of WT HCT transplanted, PBS control treated (n=8), WT HCT transplanted, M-CSF treated (n=8) or *Il15ra*-KO HCT transplanted, PBS control treated (n=10) or *Il15ra-*KO HCT transplanted, M-CSF treated mice(n=10). Mice were transplanted with WT HCT control or *Il15ra*-KO HCT HCT (solid arrow), control PBS or M-CSF treated (black arrowheads), and infected with MCMV (stippled arrow) as described in Fig.1A, B.
^{∗}P=<0.05; ^{∗∗}P=<0.01 by two-tailed non-parametric Mann-Whitney U-test (A-D). ^{∗∗∗}P=<0.0001 by Mantel-Cox test (F-G). All data are representative of two independent experiments.

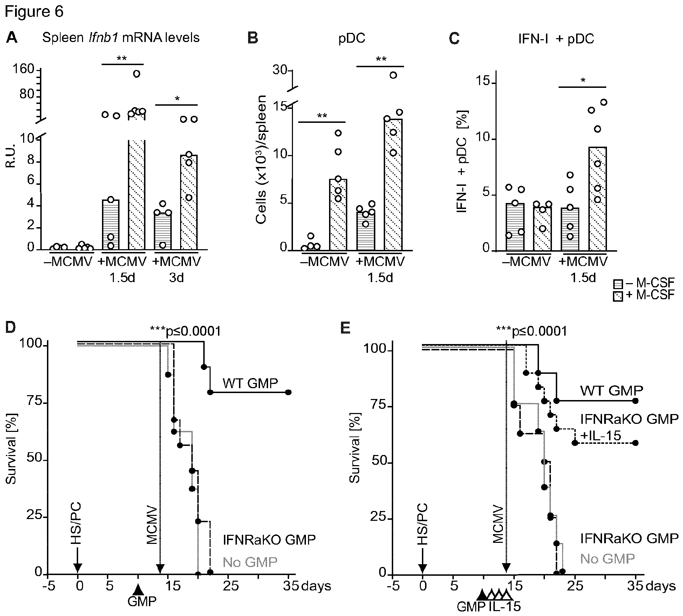

**Figure 6** shows that M-CSF-induced IFN-I production stimulates IL-15-dependent antiviral effect.
A) Spleen *Ifnb1* mRNA levels of control or M-CSF-treated recipient mice without or 1.5 and 3 days after MCMV infection measured by RT-qPCR.
B, C) Median of total numbers (B) and % IFN-β⁺ (C) of donor-derived spleen Lin⁻ CD11c^{lo} BST2^{hi} pDC (gating strategy Fig.S3) of transplanted PBS control or M-CSF-treated recipient mice 2 weeks after transplantation, without or at 1.5 days after MCMV infection.
D) Analysis of requirement for myeloid I-IFN response in antiviral effect. Survival of MCMV infected, no GMP control (n=8), WT GMP (n=9) or *Ifnarl*-KO GMP-transplanted mice (n=9). Mice were HCT transplanted (solid arrow), infected with MCMV (stippled arrow) as described in Fig.1A, B and GMP transplanted 10 days after HCT transplantation (arrowhead). P=<0.0001 by Mantel-Cox test.
E) Analysis of requirement for myeloid I-IFN response in IL-15 mediated antiviral effect. Survival of MCMV infected, no GMP control (n=8), WT GMP (n=8) or *Ifnarl*-KO GMP-transplanted mice without (n=8) or with IL-15 rescue treatment (n=16). Mice were HCT transplanted (solid arrow), infected with MCMV (stippled arrow) as described in Fig.1A, B, GMP transplanted 10 days after HCT transplantation (blue arrowhead) and treated or not with 0.5 µg IL-15 on day 12, 13 and 14 (black arrowheads).
^{∗∗}P=<0.01, ^{∗}P=<0.05 by two-tailed non-parametric Mann-Whitney U-test (A-D). ^{∗∗∗}P=<0.0001 by Mantel-Cox test (E-F). All data are representative of two independent experiments.

**Figure 7** shows a scheme of M-CSF-induced coordinated myeloid and NK cell differentiation and activation cascade in anti-viral defense.

**Figure 8** is disclosed in Ogonek et al and shows a time line of complications after allogeneic HCT. The figure shows the most prevalent complications after HCT according to the three phases of engraftment. Concomitant infectious complications consisting of bacterial, fungal, and viral infections are shown according to their occurrence as well as association with acute and chronic GvHD during different phases of follow-up: (1) pre-engraftment, (2) engraftment, and (3) post-engraftment phase. Abbreviations: CMV, cytomegalovirus; aGvHD, acute graft-versus-host disease; cGvHD, chronic graft-versus-host disease.

### Detailed description of the invention

### Definitions

"Activation," and "stimulation", as it applies to cells or to receptors, may have the same meaning, e.g., activation or stimulation of a cell or receptor with a ligand, agonist or antagonist unless indicated otherwise by the context or explicitly.

"Activation" can refer to cell activation as regulated by internal mechanisms as well as by external or environmental factors.

"Ligand" encompasses natural and synthetic (artificial) ligands, e.g., cytokines, cytokine variants, analogues, muteins, and binding compositions derived from antibodies. "Ligand" also encompasses small molecules, e.g., peptide mimetics of cytokines, peptide mimetics of antibodies, nucleic acids and nucleic acid mimetics.

An "agonist" is a chemical, agent or ligand that binds to a receptor and activates the receptor to produce a biological response. Whereas an agonist causes an action, an antagonist blocks the action of the agonist and an inverse agonist causes an action opposite to that of the agonist.

"Activity" of a molecule may describe or refer to the binding of the molecule to a ligand or to a receptor, to catalytic activity; to the ability to stimulate gene expression or cell signalling, differentiation, or maturation; to antigenic activity, to the modulation of activities of other molecules, and the like. "Activity" of a molecule may also refer to activity in modulating or maintaining cell-to-cell interactions, e.g., adhesion, or activity in maintaining a structure of a cell, e.g., cell membranes or cytoskeleton.

"Administration" and "treatment," as it applies to treatment of a human subject, research subject, veterinary subject or animal, refers to exposure of the human subject, research subject, veterinary subject or animal to a pharmaceutical, therapeutic, diagnostic agent or composition.

"Prophylaxis" means the prevention of or protective treatment of a disease or disorder. "Prophylactic treatment" means a treatment to prevent the outbreak of a disease or disorder.

"Disorder" or "disease" refers to a pathological state, or a condition that is correlated with or predisposes to a pathological state. In particular, "disorder" or "disease" is an impairment of the normal state of the living animal or human body or one of its parts that interrupts or modifies the performance of the vital functions, is typically manifested by distinguishing signs and symptoms, and is a response to environmental factors (as malnutrition, industrial hazards, or climate), to specific infective agents (as worms, bacteria, fungi or viruses), to inherent defects of the organism (as genetic anomalies or impaired functionality of the immune system), or to combinations of these factors.

"Infectious disorder" or "infectious diseases" refers, e.g., to a disorder resulting from a microbe, bacterium, parasite, pathogenic fungus, viruses and the like, as well as to an inappropriate, ineffective, or pathological immune response to the disorder.

"Effective amount" means, e.g., an amount of a CSF1R agonist, antagonist, or binding compound or composition sufficient to prevent or ameliorate a symptom or sign of a disorder, condition, or pathological state in a tissue system, animal or human being sought by a researcher, veterinarian, medical doctor or other clinician.

"Expression" refers to the presence of a measureable amount of mRNA or polypeptide encoded by a specific gene. Units of expression may be a measure of, e.g., the number of molecules of mRNA or polypeptide/mg protein in a cell or tissue, or in a cell extract or tissue extract. The units of expression may be relative, e.g., a comparison of signal from control and experimental mammals or a comparison of signals with a reagent that is specific for the mRNA or polypeptide versus a reagent that is non-specific.

A "marker" relates to the phenotype of a cell, tissue, organ, animal, e.g., of a mouse, or human subject. A cell surface marker refers to a molecule that is located on the plasma membrane of a specific cell type or even a limited number of cell types. An intracellular marker refers to a molecule that is located inside the cell of specific cell type or even a limited number of cell types. They are normally used in identification of cell types. Markers are used to detect cells, e.g., during cell purification, quantitation, migration, activation, maturation, or development, and may be used for both *in vitro* and *in vivo* studies. An activation marker is a marker that is associated with cell activation.

A "primary cell" is a cell that is directly derived from the human or animal body.

A "gene" encompasses the coding region for a mRNA and/or polypeptide and regulatory sequences, e.g., promoters, operators, introns, splice acceptor and donor sites, translational and transcriptional start and stop signals. The coding region may comprise one, continuous exon, or it may comprise more than one exon, i.e., it may be interrupted by one or more introns. A "gene" can encompass one or more open reading frames (ORF).

If the CSF1R agonists of the subject method or medical use will be small molecules, they will have e.g., molecular weights of 1000 g/mole or less, 500 g/mole or less, preferably of 400 g/mole or less, and even more preferably of 350 g/mole or less and even of 300 g/mole or less.

The term "subject" as used herein, refers to an animal, preferably a mammal, most preferably a human, who has been the object of treatment, observation or experiment and/or is suspected of being afflicted with a disease and/or condition as defined in the items.

A viral infection as used herein includes an infection with a virus that is extrinsic to the subject's body as well as the activation of one or more intrinsic viruses, for example the reactivation of a latent virus infection. A "latent virus infection" as used herein means that a subject is carrying the respective virus, but not suffering from acute symptoms of the respective viral disease. A subject which is IgG seropositive for one or more protein(s) specific for the respective virus, wherein the virus is known to typically remain withing the subject's body in a latent state after an acute infection, is, within the present invention, having a latent virus infection. The skilled person is aware that there are FDA-approved assays for determining seropositivity due to the latent infection with a specific virus, for example the FDA-approved assays having the STN number BK200542, BK200476, BK180247, BK950029 and BK070030, respectively, for the detection of antibodies against CMV. For EBV the presence of antibodies to both, the viral capsid antigen and the EBV nuclear antigen, indicate a past acute infection with EBV and thus a state of latent infection (see https://www.cdc.gov/epstein-barr/laboratory-testing.html).

"Immunosuppression" as used herein is a reduction in the capacity of the immune system to respond effectively to foreign antigens, pathogen associated patterns (PAMPs) or danger associated patterns (DAMPs), including surface antigens or abnormal patterns on tumour, diseased and infected cells. Immunosuppression can result from a numerical reduction of immune effector cells or a reduction of the functional capacity of immune effector cells, such as mounting an inflammatory response, or such as killing, eliminating, or otherwise neutralising pathogens, or infected, cancerous and diseased cells. This can result, for example, from blockage or active repression of intracellular pathways essential for antigen recognition or for mounting immune effector functions or from the inability to engage other elements of the immune response.

States of immunosuppression are often characterized by a numerical reduction of blood monocytes or from a reduction of the blood monocytes' capability to mount an inflammatory response.

In some disorders characterized by a state of immunosuppression the absolute number of CD14+ and HLA-DR+ expressing monocytes is reduced during at least one phase of the disorder, such as reduced to at most 80 % compared to the absolute number of CD14+ and HLA-DR+ expressing monocytes in a cohort of healthy, matched control subjects (mathematically that is ≤0.8x the absolute number of CD14+ and HLA-DR+ expressing monocytes in a cohort of healthy, matched control subjects), such as reduced to at most 60 % compared to the absolute number of CD14+ and und HLA-DR+ expressing monocytes in a cohort of healthy, matched control subjects.

This is, for example, the case in the disorder hematopoietic stem cell transplantation, where the absolute number of CD14+ and HLA-DR+ expressing monocytes is reduced because the overall number of monocytes is reduced (in the case of HCT as a consequence of depleting the recipients own hematopoietic stem cells in preparation of the HCT).

In some other disorders characterized by a state of immunosuppression, for example in the disorder sepsis, the functional anergy of a subject's blood monocyte compartment appears to be due to reduced HLA-DR expression levels for the overall population of blood monocytes during at least one phase of the disorder. In such a situation, the overall number of blood monocytes may be unaffected in comparison to a healthy matched control cohort or even slightly higher, while the average level of HLA-DR expression within the blood monocyte population is decreased. In the practice of clinical medicine, the level of HLA-DR expression on blood monocytes is often measured using Becton Dickinsons Quantbrite^{™} assay, such as "Anti-Human-HLA-DR PE/Monocyte PerCP Cy5.5, Catalogue number 340827, which determines the number of antibodies bound per CD14+ monocyte in blood (the "ABC" value). The data sheet 23-3949-02 dated 5/2014, which is herein incorporated by reference. A mean value of at most 15000 anti-HLA-DR antibodies bound per CD14+ monocyte is indicative of an immunosuppressed state, in particular a value of at most 12000 ABC, such as at most 10000ABC (see, for example, Quadrini et al. Cytometry 2021; 100:103-114).

Specifically, a "state of immunosuppression" can occur, for example, when a subject receives immunosuppressive drugs due to an autoimmune disease or in preparation for or after an organ or tissue transplantation or after chemotherapy in the context of a cancer treatment. In these disorders a state of immunosuppression can be induced as part of a treatment regimen. A state of immunosuppression can also during disorders, which are characterized by (a) period(s) of increased DAMP (damage associated molecular pattern) and/or PAMP (pathogen associated molecular pattern) concentrations in the blood. An example is the disorder sepsis, where PAMPs in the blood, such as bacterial lipopolysaccharide, can cause post-sepsis immunosuppression. Other examples characterized by at least transiently increased DAMP and/or PAMP concentrations in the blood are, for example, the disorder severe burn and, for example, the disorder severe trauma.

An immunosuppressed state may occur as a phase of a disorder, like sepsis, or it may be induced. For example, an immunosuppressed state is induced prior to HCT, when the recipient is conditioned for stem cell transplantation by chemotherapy and/or irradiation. An immunosuppressed state is, for example, also induced by chemotherapy and/or irradiation, for example, for the treatment of cancer patients or for organ transplantation. In such cases, when it is intended to induce an immunosuppressed state in a subject is as part of a planned therapeutic treatment of a disorder, the present invention suggests to assay for the presence of a latent virus in the subject to be treated, and, preferably if such a virus is present, the administration of a CSF1R agonist, such as M-CSF, for preventing the reactivation of the latent virus.

"HLA-DR" is an MHC class II cell surface receptor encoded by the human leukocyte antigen complex on chromosome 6 region 6p21.31. The complex of HLA-DR (Human Leukocyte Antigen - DR isotype ) and peptide, generally between 9 and 30 amino acids in length, constitutes a ligand for the T-cell receptor (TCR). HLA (human leukocyte antigens) were originally defined as cell surface antigens that mediate graft-versus-host disease. Identification of these antigens has led to greater success and longevity in organ transplant. Antigens most responsible for graft loss are HLA-DR (first six months), HLA-B (first two years), and HLA-A (long-term survival). Good matching of these antigens between host and donor is most critical for achieving graft survival. HLA-DR is also involved in several autoimmune conditions, disease susceptibility and disease resistance. HLA-DR molecules are upregulated in response to signaling. In the instance of an infection, the peptide (e.g the staphylococcal enterotoxin I peptide) is bound into a DR molecule and presented to a few of a great many T-cell receptors found on T-helper cells. These cells then bind to antigens on the surface of B-cells stimulating B-cell proliferation.

"CD14" (cluster of differentiation 14) is a human protein made mostly by macrophages as part of the innate immune system.[5][6] It helps to detect bacteria in the body by binding lipopolysaccharide (LPS), a pathogen-associated molecular pattern (PAMP).

"CD16", also known as FcyRIII, is a cluster of differentiation molecule found on the surface of natural killer cells, neutrophils, monocytes, macrophages, and certain T cells. CD16 has been identified as Fc receptors FcγRIIIa (CD16a) and FcyRIIIb (CD16b), which participate in signal transduction. The most well-researched membrane receptor implicated in triggering lysis by NK cells, CD16 is a molecule of the immunoglobulin superfamily (IgSF) involved in antibody-dependent cellular cytotoxicity (ADCC). It can be used to isolate populations of specific immune cells through fluorescent-activated cell sorting (FACS) or magnetic-activated cell sorting, using antibodies directed towards CD16.

CD27 as used herein is described in detail as gene ID 939 in the NCBI Gene database. The protein encoded by this gene is a member of the TNF-receptor superfamily. This receptor is required for generation and long-term maintenance of T cell immunity. It binds to ligand CD70, and plays a key role in regulating B-cell activation and immunoglobulin synthesis. This receptor transduces signals that lead to the activation of NF-kappaB and MAPK8/JNK. Adaptor proteins TRAF2 and TRAF5 have been shown to mediate the signaling process of this receptor. CD27-binding protein (SIVA), a proapoptotic protein, can bind to this receptor and is thought to play an important role in the apoptosis induced by this receptor. It exists a corresponding murine gene.

The type-I interferons ("IFN-I") are cytokines which play essential roles in inflammation, immunoregulation, tumor cells recognition, and T-cell responses. In the human genome, a cluster of thirteen functional IFN genes is located at the 9p21.3 cytoband over approximately 400 kb including coding genes for IFNα (*IFNA1, IFNA2, IFNA4, IFNA5, IFNA6, IFNA7, IFNA8, IFNA10, IFNA13, IFNA14, IFNA16, IFNA17* and *IFNA21*), IFNω (*IFNW1*)*,* IFNε (*IFNE*), IFNκ (*IFNK*) and IFNβ (*IFNB1*), plus 11 IFN pseudogenes.

As used herein, the term "agonist of the M-CSF receptor" or "CSF1R" agonist refers to any compound synthetic or natural (for instance a polypeptide, a chemical, an agent or ligand) capable of binding to the M-CSF receptor (also known as CD 115 or CSF1R, which is a cell-surface protein encoded, in humans, by the CSF1R gene) present on cells of the myelo-monocytic lineage including macrophages , monocytes, hematopoietic stem cells and hematopoietic progenitor cells such as MPP, CMP, GMP, MDP and MoP. An agonist of the M-CSF receptor can stimulate in such progenitors the production of myeloid cells, including neutrophils and cells of the mononuclear phagocyte system including monocytes, macrophages and dendritic cells. In one embodiment, the CSFR1 agonist is a human polypeptide. In a preferred embodiment, the CSFR1 agonist is the M-CSF polypeptide or a M/CSF polypeptide fused to another polypeptide, such as a M-CSF/Fc fusion protein. In another preferred embodiment, the CSFR1 agonist is the IL34 polypeptide or a IL34 polypeptide fused to another polypeptide, such as a IL34-Fc fusion protein The CSFR1 agonist shows a certain specific activity in the activity assay below.

The "specific activity" of a CSF1R agonist can be determined by measuring the ED50 of the agonist in ng/ml in a proliferation assay ("activity assay") using mouse M-NSF-60 cells. The cell proliferation assay can be calibrated with the international standard for human M-CSF (NIBSC code 89/512). A CSF1R agonist induces concentration-dependent proliferation of M-NCF-60 cells (Mire-Sluis et al. Journal of Immunological Methods 179(1995) 141-151; and WHO Technical Report Series (1994) no. 840, p. 10). In the proliferation assay specificity of an agonist for CSF1R can be determined by the fact that the agonist-dependent proliferation can be blocked with an anti-CSFl receptor antibody and/or an CSFR1 antagonist. The ED50 of a CSFR1 agonist in this proliferation assay is typically in the range of 0.5 to 2 ng/ml, which corresponds for, e.g. M-CSF to a specific activity of 0.5 - 2 × 10⁶ units/mg. Preferably CSFR1 agonists for use in the present invention have a specific activity of at least 1 × 10³ units/mg, such as at least 1 × 10⁴ units/mg, for example at least 1 × 10⁵ units/mg.

The proliferation or activity assay is suitably performed with the M-NFS-60 cell line (American Type Culture Collection Accession No. CRL-1838, which is available from ATCC in Rockville, MD, USA, and which is derived from a myelogenous leukemia induced with the Cas-Br-MuLV wild mouse ecotropic retrovirous. The M-NFS-60 cell line is responsive to both interleukin 3 and M-CSF and contains a truncated c-myb proto-oncogene caused by the integration of a retrovirus.

The proliferation of M-NFS-60 requires an agonist of the CSF1R, such as active M-CSF, in a dose dependent fashion. In the assay, M-NFS-60 cells are washed and plated in RPMI1640 medium with 10% FBS and the CSF1R-agonist, for example 3000 U/ml of M-CSF, and 1% Pen/Strep. Starting cell numbers are determined, cells are incubated at 37 °C and under 5% CO₂ atmosphere for 72 hours before resulting cell numbers are then quantified. A CSF1R-agonist enables cell expansion in a CSF1R-specific manner. The typical result of a proliferation assay is, for example, shown in product leaflets of commercial M-CSF suppliers, such as in the BioLegend product leaflet as online in May 2022 (https://www.biolegend.com/en-us/products/recombinant-human-m-csf-carrier-free-7717).

"ED50" is defined as half maximal effective dose and refers to the concentration of a CSFR1 agonist which induces a response halfway between the baseline and maximum after a specified exposure time in the activity assay. More simply, ED50 can be defined as the concentration of a CSFR1 agonist required to obtain a 50% effect in the activity assay. ED50 is a measure of concentration, expressed in molar units (M), where 1 M is equivalent to 1 mol/l.

As used herein, the terms "macrophage colony stimulating factor polypeptide" or "M-CSF polypeptide" (also known as CSF-1, for "colony stimulating factor 1 polypeptide") refer to any native or variant (whether native or synthetic) cytokine which controls the production, differentiation, and function of macrophages. The term includes naturally occurring M-CSF variants and modified forms thereof. Thus, three distinct variant M-CSF isoforms produced through alternative mRNA splicing have been described, respectively a M-CSF[alpha] variant which refers to a protein of 256 amino acids provided in the UniProt Uniparc database under accession number UPI0000D61F83, a M-CSF[beta] variant which refers to a protein of 554 amino acids provided in the GenPept database under accession number NP_000748.3 and is encoded by the nucleic acid sequence provided in the GenBank database under accession number NM_000757.5, and a M-CSF[gamma] variant which refers to a protein of 438 amino acids provided in the GenPept database under accession number NP_757349.1 and is encoded by the nucleic acid sequence provided in the GenBank database under accession number NM_172210.2.

In one embodiment, the M-CSF polypeptide is the human isoform M-CSF[alpha] of 256 amino acids provided in the UniProt/Uniparc database under accession number UPI0000D61F83 and is shown as follows (SEQ ID NO: 1) or a polypeptide having a sequence at least 80%, 85%, 90%, 95%, 96%, 97%, 98% or 99% identical to the sequence SEQ ID NO: 1 :

MTAPGAAGRCPPTTWLGSLLLLVCLLASRSITEEVSEYCSHMIGSGHLQSLQRLIDSQ
METSCQITFEFVDQEQLKDPVCYLKKAFLLVQDIMEDTMRFRDNTPNAIAIVQLQELS
LRLKSCFTKDYEEHDKACVRTFYETPLQLLEKVKNVFNETKNLLDKDWNIFSKNCNN
SFAECSSQGHERQSEGSFSPQLQESVFHLLVPSVILVLLAVGGLLFYRWRRRSHQEPQ
RADSPLEQPEGSPLTQDDRQVELPV

In one embodiment, the M-CSF polypeptide is the human isoform M-CSF[beta] of 554 amino acids provided in the GenBank database under accession number NP_000748.3 and is shown as follows (SEQ ID NO: 2) or a polypeptide having a sequence at least 80%, 85%, 90%, 95%, 96%, 97%, 98% or 99% identical to the sequence SEQ ID NO: 2:

MTAPGAAGRCPPTTWLGSLLLLVCLLASRSITEEVSEYCSHMIGSGHLQSLQRLIDSQ
METSCQITFEFVDQEQLKDPVCYLKKAFLLVQDIMEDTMRFRDNTPNAIAIVQLQELS
LRLKSCFTKDYEEHDKACVRTFYETPLQLLEKVKNVFNETKNLLDKDWNIFSKNCNN
SFAECSSQDVVTKPDCNCLYPKAIPSSDPASVSPHQPLAPSMAPVAGLTWEDSEGTEG

SSLLPGEQPLHTVDPGSAKQRPPRSTCQSFEPPETPVVKDSTIGGSPQPRPSVGAFNPG
MEDILDSAMGTNWVPEEASGEASEIPVPQGTELSPSRPGGGSMQTEPARPSNFLSASS
PLPASAKGQQPADVTGTALPRVGPVRPTGQDWNHTPQKTDHPSALLRDPPEPGSPRIS
SLRPQGLSNPSTLSAQPQLSRSHSSGSVLPLGELEGRRSTRDRRSPAEPEGGPASEGAA
RPLPRFNSVPLTDTGHERQSEGSSSPQLQESVFHLLVPSVILVLLAVGGLLFYRWRRRS
HQEPQRADSPLEQPEGSPLTQDDRQVELPV

In one embodiment, the M-CSF polypeptide is the human isoform M-CSF[gamma] of 438 amino acids provided in the GenBank database under accession number NP_757349.1_and is shown as follows (SEQ ID NO: 3) or a polypeptide having a sequence at least 80%, 85%, 90%, 95%, 96%, 97%, 98% or 99% identical to the sequence SEQ ID NO: 3 :

MTAPGAAGRCPPTTWLGSLLLLVCLLASRSITEEVSEYCSHMIGSGHLQSLQRLIDSQ
METSCQITFEFVDQEQLKDPVCYLKKAFLLVQDIMEDTMRFRDNTPNAIAIVQLQELS
LRLKSCFTKDYEEHDKACVRTFYETPLQLLEKVKNVFNETKNLLDKDWNIFSKNCNN
SFAECSSQDVVTKPDCNCLYPKAIPSSDPASVSPHQPLAPSMAPVAGLTWEDSEGTEG
SSLLPGEQPLHTVDPGSAKQRPPRSTCQSFEPPETPVVKDSTIGGSPQPRPSVGAFNPG
MEDILDSAMGTNWVPEEASGEASEIPVPQGTELSPSRPGGGSMQTEPARPSNFLSASS
PLPASAKGQQPADVTGHERQSEGSSSPQLQESVFHLLVPSVILVLLAVGGLLFYRWRR
RSHQEPQRADSPLEQPEGSPLTQDDRQVELPV

As used herein, the term "polypeptide" refers to a polymer of amino acid residues joined by peptide bonds, whether produced naturally or synthetically, having no specific length. Thus, peptides, oligopeptides and proteins are included in the definition of polypeptide and these terms are used interchangeably throughout the specification, as well as in the claims. The term polypeptide does not exclude post-translational modifications that include but are not limited to phosphorylation, acetylation, glycosylation and the like. The term also applies to amino acid polymers in which one or more amino acid residue is an artificial chemical mimetic of a corresponding naturally occurring amino acid, as well as to naturally occurring amino acid polymers and non-naturally occurring amino acid polymer.

The term "M-CSF polypeptide" is herein defined as including the naturally occurring human polypeptide M-CSF and naturally-occurring allelic variations of the polypeptide. Allelic variations are naturally-occurring base changes in the species population which may or may not result in an amino acid change in a polypeptide or protein. Additionally, the M-CSF polypeptides according to the invention not only encompass polypeptides comprising or consisting of full-length M-CSF and variants thereof, but also polypeptides consisting of fragments thereof, provided the fragments are biologically active. Additionally included in this definition are both recombinant and synthetic versions of the polypeptide M-CSF, which may contain induced modifications in the polypeptide and DNA sequences thereof. Accordingly, the term M-CSF polypeptide intends to encompass the functional equivalents of the M-CSF polypeptides encoded by the sequence SEQ ID NO: 1, SEQ ID NO: 2 or SEQ ID NO: 3. As used herein, a "functional equivalent" refers to a molecule (e.g. a recombinant polypeptide) that retains the biological activity and the specificity of the parent polypeptide. Therefore, the term "functional equivalent of the M-CSF polypeptide" includes variants and fragments of the polypeptide to which it refers (i.e. the M-CSF polypeptide) provided that the functional equivalents exhibit at least one, preferably all, of the biological activities of the reference polypeptide, as described below.

A polypeptide "variant" refers to a biologically active polypeptide having at least about 80% amino acid sequence identity with the native sequence polypeptide. Such variants include, for instance, polypeptides wherein one or more amino acid residues are added, or deleted, at the Nor C-terminus of the polypeptide. Ordinarily, a variant will have at least about 80% amino acid sequence identity, more preferably at least about 90% amino acid sequence identity, and even more preferably at least about 95% amino acid sequence identity with the native sequence polypeptide. By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% (5 of 100) of the amino acid residues in the subject sequence may be inserted, deleted, or substituted with another amino acid. In the frame of the present application, the percentage of identity is calculated using a global alignment (i.e., the two sequences are compared over their entire length). Methods for comparing the identity and homology of two or more sequences are well known in the art. The "needle" program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is for example available on the ebi.ac.uk world wide web site. The percentage of identity in accordance with the invention is preferably calculated using the EMBOSS: :needle (global) program with a "Gap Open" parameter equal to 10.0, a "Gap Extend" parameter equal to 0.5, and a Blosum62 matrix.

Polypeptides consisting of an amino acid sequence "at least 80%, 85%, 90%, 95%, 96%, 97%, 98% or 99% identical" to a reference sequence may comprise mutations such as deletions, insertions and/or substitutions compared to the reference sequence. The polypeptide consisting of an amino acid sequence at least 80%, 85%, 90%, 95%, 96%, 97%, 98% or 99% identical to a reference sequence may correspond to an allelic variant of the reference sequence. It may for example only comprise substitutions compared to the reference sequence. The substitutions preferably correspond to conservative substitutions as indicated in the table below.

| **Conservative substitutions** | **Type of Amino Acid** |
|---|---|
| Ala, Val, Leu, He, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

A polypeptide "fragment", as used herein, refers to a biologically active polypeptide that is shorter than a reference polypeptide (e.g. a fragment of the M-CSF polypeptide). Thus, the polypeptide according to the invention encompasses polypeptides comprising or consisting of fragments of M-CSF, provided the fragments are biologically active.

In the frame of the invention, the biologically active fragment may for example comprise at least 150, 175, 200, 225, 250, 275, 300, 325, 350, 375, 400, 425, 450, 475, 500, 525 or 550 consecutive amino acids of the M-CSF polypeptide.

As described above the three M-CSF isoforms are synthesized from a full-length and a truncated precursor. It should be further noted that the N-terminal 150 amino acids of precursors are identical and sufficient for in vitro biological activity as described in Pixley et al. 2004 Trends Cell Biol. 2004 Nov;14(11):628-38). Indeed, amino acids 1-150 of all three mature forms of M-CSF are identical and are believed to contain sequences essential for biological activity of M-CSF. Accordingly, a biologically active fragment of the MCSF- polypeptide is a N-terminal fragment comprising at least 150 amino acids.

In one embodiment, the M-CSF polypeptide is a recombinant 156 amino acid polypeptide of murine M-CSF and is shown as follows (SEQ ID NO: 4):

MKEVSEHCSHMIGNGHLKVLQQLIDSQMETSCQIAFEFVDQEQLDDPVCYLKKAFFL
VQDIIDETMRFKDNTPNANATERLQELSNNLNSCFTKDYEEQNKACVRTFHETPLQL
LEKIKNFFNETKNLLEKDWNIFTKNCNNSFAKCSSRDVVTKP

In one particular embodiment, the M-CSF polypeptide comprises or consists of a 150 amino acid polypeptide of human M-CSF and is shown as follows (SEQ ID NO: 5):

MTAPGAAGRCPPTTWLGSLLLLVCLLASRSITEEVSEYCSHMIGSGHLQSLQRLIDSQ
METSCQITFEFVDQEQLKDPVCYLKKAFLLVQDIMEDTMRFRDNTPNAIAIVQLQELS
LRLKSCFTKDYEEHDKACVRTFYETPLQLLEKVK

As used herein, the terms "Interleukin-34 polypeptide" or "IL-34 polypeptide" are well known in the art and refer to a cytokine that promotes the proliferation, survival and differentiation of monocytes and macrophages. The naturally occurring human IL-34 protein has an amino acid sequence of 242 amino acids provided in the UniProt database under accession number Q6ZMJ4 and is shown as follows (SEQ ID NO: 6) or a polypeptide having a sequence at least 80%, 85%, 90%, 95%, 96%, 97%, 98% or 99% identical to the sequence SEQ ID NO: 6 :

MPRGFTWLRYLGIFLGVALGNEPLEMWPLTQNEECTVTGFLRDKLQYRSRLQYMKH
YFPINYKISVPYEGVFRIANVTRLQRAQVSERELRYLWVLVSLSATESVQDVLLEGHP
SWKYLQEVETLLLNVQQGLTDVEVSPKVESVLSLLNAPGPNLKLVRPKALLDNCFRV
MELLYCSCCKQSSVLNWQDCEVPSPQSCSPEPSLQYAATQLYPPPPWSPSSPPHSTGS
VRPVRAQGEGLLP

As previously described for the M-CSF polypeptide, the term IL-34 polypeptide intends to encompass the functional equivalents of the IL-34 polypeptide encoded by the sequence SEQ ID NO: 6.

In one embodiment, the polypeptides of the invention may comprise a tag. A tag is an epitope-containing sequence which can be useful for the purification of the polypeptides. It is attached to by a variety of techniques such as affinity chromatography, for the localization of said polypeptide within a cell or a tissue sample using immuno labeling techniques, the detection of said polypeptide by immunoblotting etc. Examples of tags commonly employed in the art are the GST (glutathion-S-transferase)-tag, the FLAG^{™}-tag, the Strep-tag^{™}, V5 tag, myc tag, His tag (which typically consists of six histidine residues), etc.

In one embodiment, the polypeptides of the invention may be part of a larger polypetide comprising a fusion partner, such as the polypeptide sequence of the Fc-fragment of an antibody.

In another embodiment, the polypeptides of the invention may comprise chemical modifications improving their stability and/or their biodisponibility. Such chemical modifications aim at obtaining polypeptides with increased protection of the polypeptides against enzymatic degradation in vivo, and/or increased capacity to cross membrane barriers, thus increasing its half-life and maintaining or improving its biological activity. Any chemical modification known in the art can be employed according to the present invention.

The polypeptides of the invention may be produced by any suitable means, as will be apparent to those of skill in the art. In order to produce sufficient amounts of M-CSF polypeptides or IL-34 polypeptides for use in accordance with the invention, expression may conveniently be achieved by culturing under appropriate conditions recombinant host cells containing the polypeptide of the invention. Preferably, the polypeptide is produced by recombinant means, by expression from an encoding nucleic acid molecule. Systems for cloning and expression of a polypeptide in a variety of different host cells are well known.

When expressed in recombinant form, the polypeptide is preferably generated by expression from an encoding nucleic acid in a host cell. Any host cell may be used, depending upon the individual requirements of a particular system. Suitable host cells include bacteria mammalian cells, plant cells, yeast and baculovirus systems. Mammalian cell lines available in the art for expression of a heterologous polypeptide include Chinese hamster ovary cells. HeLa cells, baby hamster kidney cells and many others. Bacteria are also preferred hosts for the production of recombinant protein, due to the ease with which bacteria may be manipulated and grown. A common, preferred bacterial host is *E coli.* Production of recombinant active human M-CSF from bacterial cell culture is, for example, described in the United States Patent 4929700. Production of glycosylated human M-CSF from eukaryotic cells is, for example, described in WO87/06954.

Moreover, it should be noted that the majority of protein-based biopharmaceuticals bare some form of post-translational modification which can profoundly affect protein properties relevant to their therapeutic application. Protein glycosylation represents the most common modification (about 50% of human proteins are glycosylated). Glycosylation can introduce considerable heterogeneity into a protein composition through the generation of different glycan structures on the proteins within the composition. Such glycan structures are made by the action of diverse enzymes of the glycosylation machinery as the glycoprotein transits the Endoplasmatic Reticulum (ER) and the Golgi-Complex (glycosylation cascade). The nature of the glycan structure(s) of a protein has impact on the protein's folding, stability, life time, trafficking, pharmaco-dynamics, pharmacokinetics and immunogenicity. The glycan structure has great impact on the protein's primary functional activity. Glycosylation can affect local protein structure and may help to direct the folding of the polypeptide chain. One important kind of glycan structures are the so called N-glycans. They are generated by covalent linkage of an oligosaccharide to the amino (N)-group of asparagin residues in the consensus sequence NXS/T of the nascent polypeptide chain. N-glycans may further participate in the sorting or directing of a protein to its final target: the N-glycan of an antibody, for example, may interact with complement components. N-glycans also serve to stabilize a glycoprotein, for example, by enhancing its solubility, shielding hydrophobic patches on its surface, protecting from proteolysis, and directing intra-chain stabilizing interactions. Glycosylation may regulate protein half-life, for example, in humans the presence of terminal sialic acids in N-glycans may increase the half-life of proteins, circulating in the blood stream. As used herein, the term "glycoprotein" refers to any protein having one or more N- glycans attached thereto. Thus, the term refers both to proteins that are generally recognized in the art as a glycoprotein and to proteins which have been genetically engineered to contain one or more N-linked glycosylation sites. As used herein, the terms "N-glycan" and "glycoform" are used interchangeably and refer to an N-linked oligosaccharide, for example, one that is attached by an asparagine-N-acetylglucosamine linkage to an asparagine residue of a polypeptide. N-linked glycoproteins contain an N-acetylglucosamine residue linked to the amide nitrogen of an asparagine residue in the protein. The predominant sugars found on glycoproteins are glucose, galactose, mannose, fucose, N-acetylgalactosamine (GalNAc), N- acetylglucosamine (GlcNAc) and sialic acid (e.g., N- acetyl-neuraminic acid (NANA)). The processing of the sugar groups occurs co-translationally in the lumen of the ER and continues post-translationally in the Golgi apparatus for N-linked glycoproteins. A number of yeasts, for example, *Pichia pastoris, Yarrowia lipolytica* and *Saccharomyces cerevisiae* are recently under development to use the advantages of such systems but to eliminate the disadvantages in respect to glycosylation. Several strains are under genetical development to produce defined, human-like glycan structures on a protein. Methods for genetically engineering yeast to produce human-like N- glycans are described in U.S. Patent Nos. 7,029,872 and 7,449,308 along with methods described in U.S. Published Application Nos. 20040230042, 20050208617, 20040171826, 20050208617, and 20060286637. These methods have been used to construct recombinant yeast that can produce therapeutic glycoproteins that have predominantly human-like complex or hybrid N- glycans thereon instead of yeast type N-glycans. As previously described, human-like glycosylation is primarily characterized by "complex" N-glycan structures containing N-acetylglusosamine, galactose, fucose and/or N-acetylneuraminic acid. Thus, several strains of yeasts have been genetically engineered to produce glycoproteins comprising one or more human-like complex or human-like hybrid N-glycans such as GlcNAcMan3GlcNAc2.

The specific activity of the M-CSF polypeptide or IL-34 polypeptide may vary depending on the method that was used for their preparation. In particular, the glycosylation status may vary between M-CSF polypeptides or IL-34 polypeptides that were isolated from a human subject, compared to M-CSF polypeptides or IL-34 polypeptides that were produced recombinantly in human or non-human host cells. The terms "M-CSF polypeptide" and "IL-34 polypeptide" also include recombinant polypeptides comprising varying glycosylation. For the purpose of the present invention, the amount or dose of a M-CSF polypeptide or IL-34 polypeptide that is administered to a subject, has to be adapted in each case to the amount of M-CSF or IL-34 that was extracted from a human source, such as urine. The necessary dose adaption can be performed based on the specific activity by measuring the ED50 of the respective the proliferation assay described above.

IKZF1 (IKAROS family zinc finger 1) as used herein is described in detail as gene ID 10320 in the NCBI Gene database. This gene encodes a transcription factor that belongs to the family of zinc-finger DNA-binding proteins associated with chromatin remodeling. The expression of this protein is restricted to the fetal and adult hemo-lymphopoietic system, and it functions as a regulator of lymphocyte differentiation. The corresponding murine gene is *Ikaros.*

ID2 (inhibitor of DNA binding 2) as used herein is described in detail as gene ID 3398 in the NCBI Gene database. The protein encoded by this gene belongs to the inhibitor of DNA binding family, members of which are transcriptional regulators that contain a helix-loop-helix (HLH) domain but not a basic domain. Members of the inhibitor of DNA binding family inhibit the functions of basic helix-loop-helix transcription factors in a dominant-negative manner by suppressing their heterodimerization partners through the HLH domains. This protein may play a role in negatively regulating cell differentiation. The corresponding murine gene is *Id2.*

RUNX3 (RUNX family transcription factor 3) as used herein is described in detail as gene ID 864 in the NCBI Gene database. This gene encodes a member of the runt domain-containing family of transcription factors. A heterodimer of this protein and a beta subunit forms a complex that binds to the core DNA sequence 5'-PYGPYGGT-3' found in a number of enhancers and promoters, and can either activate or suppress transcription. It also interacts with other transcription factors. It functions as a tumor suppressor, and the gene is frequently deleted or transcriptionally silenced in cancer. The corresponding murine gene is *Runx3.*

GATA3 (GATA binding protein 3) as used herein is described in detail as gene ID 2625 in the NCBI Gene database. This gene encodes a protein which belongs to the GATA family of transcription factors. The protein contains two GATA-type zinc fingers and is an important regulator of T-cell development and plays an important role in endothelial cell biology. Defects in this gene are the cause of hypoparathyroidism with sensorineural deafness and renal dysplasia. The corresponding murine gene is *Gata3.*

TBX21 (T-box transcription factor 21) as used herein is described in detail as gene ID 30009 in the NCBI Gene database. This gene is a member of a phylogenetically conserved family of genes that share a common DNA-binding domain, the T-box. T-box genes encode transcription factors involved in the regulation of developmental processes. This gene is the human ortholog of mouse *Tbx21*/*Tbet* gene. Studies in mouse show that Tbx21 protein is a Th1 cell-specific transcription factor that controls the expression of the hallmark Th1 cytokine, interferon-gamma (IFNG). Expression of the human ortholog also correlates with IFNG expression in Th1 and natural killer cells, suggesting a role for this gene in initiating Th1 lineage development from naive Th precursor cells.

EOMES (eomesodermin) as used herein is described in detail as gene ID 8320 in the NCBI Gene database. This gene belongs to the TBR1 (T-box brain protein 1) sub-family of T-box genes that share the common DNA-binding T-box domain. The encoded protein is a transcription factor which is crucial for embryonic development of mesoderm and the central nervous system in vertebrates. The protein may also be necessary for the differentiation of effector CD8+ T cells which are involved in defense against viral infections. A similar *Eomes* gene disrupted in mice is shown to be essential during trophoblast development and gastrulation.

IFNG (interferon gamma) as used herein is described in detail as gene ID 3458 in the NCBI Gene database. This gene encodes a soluble cytokine that is a member of the type II interferon class. The encoded protein is secreted by cells of both the innate and adaptive immune systems. The active protein is a homodimer that binds to the interferon gamma receptor which triggers a cellular response to viral and microbial infections. Mutations in this gene are associated with an increased susceptibility to viral, bacterial and parasitic infections and to several autoimmune diseases. The corresponding murine gene is *Infg.*

GZMB (granzyme B) as used herein is described in detail as gene ID 3002 in the NCBI Gene database. This gene encodes a member of the granzyme subfamily of proteins, part of the peptidase S1 family of serine proteases. The encoded preproprotein is secreted by natural killer (NK) cells and cytotoxic T lymphocytes (CTLs) and proteolytically processed to generate the active protease, which induces target cell apoptosis. This protein also processes cytokines and degrades extracellular matrix proteins, and these roles are implicated in chronic inflammation and wound healing. Expression of this gene may be elevated in human patients with cardiac fibrosis. The corresponding murine gene is *Gzmb.*

PRF1 (perforin 1) as used herein is described in detail as gene ID 5551 in the NCBI Gene database. This gene encodes a protein with structural similarities to complement component C9 that is important in immunity. This protein forms membrane pores that allow the release of granzymes and subsequent cytolysis of target cells. Whether pore formation occurs in the plasma membrane of target cells or in an endosomal membrane inside target cells is subject to debate. Mutations in this gene are associated with a variety of human disease including diabetes, multiple sclerosis, lymphomas, autoimmune lymphoproliferative syndrome (ALPS), aplastic anemia, and familial hemophagocytic lymphohistiocytosis type 2 (FHL2), a rare and lethal autosomal recessive disorder of early childhood. The corresponding murine gene is *Prf1.*

CEBPA (CCAAT enhancer binding protein alpha) as used herein is described in detail as gene ID 1050 in the NCBI Gene database. This intronless gene encodes a transcription factor that contains a basic leucine zipper (bZIP) domain and recognizes the CCAAT motif in the promoters of target genes. The encoded protein functions in homodimers and also heterodimers with CCAAT/enhancer-binding proteins beta and gamma. Activity of this protein can modulate the expression of genes involved in cell cycle regulation as well as in body weight homeostasis. Mutation of this gene is associated with acute myeloid leukemia. The corresponding murine gene is *Cebpa.*

MITF (melanocyte inducing transcription factor) as used herein is described in detail as gene ID 4286 in the NCBI Gene database. The protein encoded by this gene is a transcription factor that contains both basic helix-loop-helix and leucine zipper structural features. The encoded protein regulates melanocyte development and is responsible for pigment cell-specific transcription of the melanogenesis enzyme genes. The corresponding murine gene is *Mitf.*

XCL1 (X-C motif chemokine ligand 1) as used herein is described in detail as gene ID 6375 in the NCBI Gene database. This antimicrobial gene encodes a member of the chemokine superfamily. Chemokines function in inflammatory and immunological responses, inducing leukocyte migration and activation. The encoded protein is a member of the C-chemokine subfamily, retaining only two of four cysteines conserved in other chemokines, and is thought to be specifically chemotactic for T cells. This gene and a closely related family member are located on the long arm of chromosome 1. The corresponding murine gene is *Xcl1.*

STATB5 (signal transducer and activator of transcription 5B) as used herein is described in detail as gene ID 6777 in the NCBI Gene database. The protein encoded by this gene is a member of the STAT family of transcription factors. In response to cytokines and growth factors, STAT family members are phosphorylated by the receptor associated kinases, and then form homo- or heterodimers that translocate to the cell nucleus where they act as transcription activators. This protein mediates the signal transduction triggered by various cell ligands, such as IL2, IL4, CSF1, and different growth hormones. It has been shown to be involved in diverse biological processes, such as TCR signaling, apoptosis, adult mammary gland development, and sexual dimorphism of liver gene expression. The corresponding murine gene is *Tstat5b.*

JAK3 (Janus kinase 3) as used herein is described in detail as gene ID 3718 in the NCBI Gene database. The protein encoded by this gene is a member of the Janus kinase (JAK) family of tyrosine kinases involved in cytokine receptor-mediated intracellular signal transduction. It is predominantly expressed in immune cells and transduces a signal in response to its activation via tyrosine phosphorylation by interleukin receptors. Mutations in this gene are associated with autosomal SCID (severe combined immunodeficiency disease). The corresponding murine gene is *Jak3.*

IFNB1 (interferon beta 1) as used herein is described in detail as gene ID 3456 in the NCBI Gene database. This gene encodes a cytokine that belongs to the interferon family of signaling proteins, which are released as part of the innate immune response to pathogens. The protein encoded by this gene belongs to the type I class of interferons, which are important for defense against viral infections. In addition, type I interferons are involved in cell differentiation and anti-tumor defenses. Following secretion in response to a pathogen, type I interferons bind a homologous receptor complex and induce transcription of genes such as those encoding inflammatory cytokines and chemokines. Overactivation of type I interferon secretion is linked to autoimmune diseases. Mice deficient for this gene display several phenotypes including defects in B cell maturation and increased susceptibility to viral infection. The corresponding murine gene is *Ifnb1.*

E2F1 (E2F transcription factor 1) as used herein is described in detail as gene ID 1869 in the NCBI Gene database. The protein encoded by this gene is a member of the E2F family of transcription factors. The E2F family plays a crucial role in the control of cell cycle and action of tumor suppressor proteins and is also a target of the transforming proteins of small DNA tumor viruses. The E2F proteins contain several evolutionally conserved domains found in most members of the family. These domains include a DNA binding domain, a dimerization domain which determines interaction with the differentiation regulated transcription factor proteins (DP), a transactivation domain enriched in acidic amino acids, and a tumor suppressor protein association domain which is embedded within the transactivation domain. This protein and another 2 members, E2F2 and E2F3, have an additional cyclin binding domain. This protein binds preferentially to retinoblastoma protein pRB in a cell-cycle dependent manner. It can mediate both cell proliferation and p53-dependent/independent apoptosis. The corresponding murine gene is *E2f1.*

IL15RA (interleukin 15 receptor subunit alpha) as used herein is described in detail as gene ID 3601 in the NCBI Gene database. This gene encodes a cytokine receptor that specifically binds interleukin 15 (IL15) with high affinity. The receptors of IL15 and IL2 share two subunits, IL2R beta and IL2R gamma. This forms the basis of many overlapping biological activities of IL15 and IL2. The protein encoded by this gene is structurally related to IL2R alpha, an additional IL2-specific alpha subunit necessary for high affinity IL2 binding. Unlike IL2RA, IL15RA is capable of binding IL15 with high affinity independent of other subunits, which suggests distinct roles between IL15 and IL2. This receptor is reported to enhance cell proliferation and expression of apoptosis inhibitor BCL2L1/BCL2-XL and BCL2. The corresponding murine gene is *Il15ra.*

IL2RB (interleukin-2 receptor subunit beta, also known as CD122 or IL15RB) as used herein is described in detail as gene ID 3560 in the NCBI Gene database. The interleukin 2 receptor, which is involved in T cell-mediated immune responses, is present in 3 forms with respect to ability to bind interleukin 2. The low affinity form is a monomer of the alpha subunit and is not involved in signal transduction. The intermediate affinity form consists of an alpha/beta subunit heterodimer, while the high affinity form consists of an alpha/beta/gamma subunit heterotrimer. Both the intermediate and high affinity forms of the receptor are involved in receptor-mediated endocytosis and transduction of mitogenic signals from interleukin 2. The protein encoded by this gene represents the beta subunit and is a type I membrane protein. The use of alternative promoters results in multiple transcript variants encoding the same protein. The protein is primarily expressed in the hematopoietic system. The corresponding murine gene is *Il2rb (Il15rb).*

IL-15 (interleukin 15) as used herein is described in detail as gene ID 3600 in the NCBI Gene database. The protein encoded by this gene is a cytokine that regulates T and natural killer cell activation and proliferation. This cytokine and interleukine 2 share many biological activities. They are found to bind common hematopoietin receptor subunits, and may compete for the same receptor, and thus negatively regulate each other's activity. The number of CD8+ memory cells is shown to be controlled by a balance between this cytokine and IL2. This cytokine induces the activation of JAK kinases, as well as the phosphorylation and activation of transcription activators STAT3, STAT5, and STAT6. Studies of the mouse counterpart *Il15*suggested that this cytokine may increase the expression of apoptosis inhibitor BCL2L1BCL-x(L), possibly through the transcription activation activity of STAT6, and thus prevent apoptosis.

IL-34 (interleukin 34) as used herein is described in detail as gene ID 146433 in the NCBI Gene database. Interleukin-34 is a cytokine that promotes the differentiation and viability of monocytes and macrophages through the colony-stimulating factor-1 receptor (CSF1R). The corresponding murine gene is *Il34.*

A "myeloid cell" as used herein is a cell of hematopoietic origin that is not lymphoid and not erythro-megakaryocytic and not a multi-lineage progenitor with more than myeloid lineage potential.

A "monocyte" is a mononuclear phagocyte of the peripheral blood. Peripheral blood monocytes are phenotypically and functionally heterogeneous with an international nomenclature describing three subsets, based on cell surface expression of CD14 and CD16 (Blood 2010, PMID:20628149). Classical CD14+, CD16- monocytes represent roughly 85% of total human monocytes, express high level of the chemokine receptor CCR2, show a strong inflammatory response to lipopolysaccharides, and are rapidly recruited to inflamed and infected tissues. Their half-life in the peripheral blood is estimated to be one day (Patel et al., 2017). Most of them migrate into tissues to generate macrophages or other cell types (Guilliams A et al, 2018). These classical monocytes are distinct from CD14+, CD16+ intermediate monocytes whose half-life is 3-4 days and CD14low, CD16+ nonclassical monocytes, which express higher levels of CX3CR1 receptor, and remain longer in the blood (half-life 7 days) to patrol on the luminal part of the endothelium wall (Cros J et al, 2010).

A "dendritic cell" (DC) is an antigen presenting cell existing in vivo, in vitro, ex vivo, or in a host or subject, or which can be derived from a hematopoietic stem cell, a hematopoietic progenitor or a monocyte. Dendritic cells and their precursors can be isolated from a variety of lymphoid organs, e.g., spleen, lymph nodes, as well as from bone marrow and peripheral blood. The DC has a characteristic morphology with thin sheets (lamellipodia) extending in multiple directions away from the dendritic cell body. DCs express constitutively both MHC class I and class II molecules, which present peptide antigens to CD8+ and CD4+ T cells respectively and can activate naive T-cells. In addition, human skin and mucosal DCs also express the CD1 gene family, MHC class I-related molecules that present microbial lipid or glycolipid antigens. The DC membrane is also rich in molecules that allow adhesion of T cells (e.g. intercellular adhesion molecule 1 or CD54) or that co-stimulate T-cell activation such as B7-1 and B7-2 (also known as CD80 and CD86 respectively). Generally, DCs express CD85, CD180, CD187 CD205 CD281, CD282, CD284, CD286 and in a subset manner CD206, CD207, CD208 and CD209. Unless specifically excluded, "dendritic cells" are comprised by the term "macrophage" as used herein.

Plasmacytoid dendritic cells (pDCs) as used herein are a relatively recently discovered cell type that was first characterized in the 1990s. The earliest *in vitro* studies found that these cells were developmentally and functionally related to DCs. However, pDCs lack dendrite-like projections and instead have the rounded shape of plasma cells. Their name thus derives from the combination of their DC-like function with their plasma cell-like morphology. pDCs are mainly found in the lymphoid organs and tissues where they remain resident for relatively long periods. These cells express a narrow range of PRRs that are focused on the recognition of viral nucleic acids. In response to engagement of these PRRs by viral RNA or DNA, pDCs secrete large quantities of IFNα and IFNβ, cytokines that have potent antiviral properties. The capacity of pDCs for antigen presentation is more limited than that of DCs, and pDCs appear to be less important for the activation of naive T cells.

"Natural killer cells", also known as "NK cells" or large granular lymphocytes (LGL), are a type of cytotoxic lymphocyte critical to the innate immune system that belong to the rapidly expanding family of innate lymphoid cells (ILC) and represent 5-20% of all circulating lymphocytes in humans. The role of NK cells is analogous to that of cytotoxic T cells in the vertebrate adaptive immune response. NK cells provide rapid responses to virus-infected cell and other intracellular pathogens acting at around 3 days after infection, and respond to tumor formation. Typically, immune cells detect the major histocompatibility complex (MHC) presented on infected cell surfaces, triggering cytokine release, causing the death of the infected cell by lysis or apoptosis. NK cells are unique, however, as they have the ability to recognize and kill stressed cells in the absence of antibodies and MHC, allowing for a much faster immune reaction. They were named "natural killers" because of the notion that they do not require activation to kill cells that are missing "self" markers of MHC class 1.This role is especially important because harmful cells that are missing MHC I markers cannot be detected and destroyed by other immune cells, such as T lymphocyte cells.

NK cells can be identified by the presence of CD56 and the absence of CD3 (CD56+, CD3-). NK cells (belonging to the group of innate lymphoid cells) are one of the three kinds of cells differentiated from the common lymphoid progenitor, the other two being B and T lymphocytes. NK cells are known to differentiate and mature in the bone marrow, lymph nodes, spleen, tonsils, and thymus, where they then enter into the circulation. NK cells differ from natural killer T cells (NKTs) phenotypically, by origin and by respective effector functions; often, NKT cell activity promotes NK cell activity by secreting interferon gamma. In contrast to NKT cells, NK cells do not express T-cell antigen receptors (TCR) or pan T marker CD3 or surface immunoglobulins (Ig) B cell receptors, but they usually express the surface markers CD16 (FcyRIII) and CD57 in humans, NK1.1 or NK1.2 in C57BL/6 mice. The NKp46 cell surface marker constitutes, at the moment, another NK cell marker of preference being expressed in both humans, several strains of mice (including BALB/c mice) and in three common monkey species.

In addition to natural killer cells being effectors of innate immunity, both activating and inhibitory NK cell receptors play important functional roles, including self-tolerance and the sustaining of NK cell activity. NK cells also play a role in the adaptive immune response: numerous experiments have demonstrated their ability to readily adjust to the immediate environment and formulate antigen-specific immunological memory, fundamental for responding to secondary infections with the same antigen. The role of NK cells in both the innate and adaptive immune responses is becoming increasingly important in research using NK cell activity as a potential cancer therapy.

A "surface marker" is a molecule, typically a protein or a carbohydrate structure, that is present and accessible on the exterior of the plasma membrane of a cell and that is specific for a particular cell type or a limited number of cell types, thereby being a "marker" for these cell types

A cell is "positive" for a surface marker if staining with a surface-marker-specific antibody creates a specific fluorescence signal in a FACS experiment. The principles of FACS are explained in detail in the book "practical flow cytometry", 4th edition by Howard M. Shapiro. In a FACS experiment a collection of cells is typically stained with several fluorescent antibodies, each one selectively binding a different surface marker and having a different fluorochrome. This allows the selection of particular cell types within a heterogeneous collection of cells by appropriate gating strategies in a FACS experiment. A specific fluorescence signal by the surface-marker-specific antibody is typically then verified in a one-dimensional histogram plot by comparing the histograms for the staining with all antibodies with the histogram for the staining with the mix of antibodies where only the surface-marker-specific antibody has been omitted (so called "FMO" or "fluorescence minus one" signal). If the two histograms are different such that the staining with the mix of all antibodies produces more fluorescence than the FMO control, then the tested collection of cells is positive for the tested cell surface marker. In terms of visual appearance of the histogram this means that the peak of fluorescence for the staining with the mix of all antibodies is shifted to higher fluorescence values when compared to the FMO control. Preferably the two histograms - all antibodies on the one hand and FMO on the other - overlap by at most 70 area% (area under the curve), such as at most 50 area%, for example by at most 25 area %.

"Secretion" of a cytokine, such as IL15, can be determined by qualitatively and/or quantitatively measuring the appearance of said cytokine in the supernatant of a cell culture with immunobiochemical methods, for example by ELISA-based methods or a bead-based multiplex assay, such as the Luminex technology, to name but two examples. Briefly, the medium used for cell growth is analyzed with regard to the presence of a particular cytokine to-be-tested BEFORE being added to a collection of cells and AFTER the cells, which are to be tested for cytokine secretion, have been cultured in it. A cytokine is "secreted" by the cells which were cultured in the medium if the concentration of the cytokine in the supernatant has increased during the cultivation period and if this increase in cytokine concentration can be confirmed in three consecutive independent measurements. IL6 can be detected at or even below a concentration of 0.1pg/ml, for example by the meso scale discovery immunoassay "V-PLEX Human IL-6 Kit" of Meso Scale Diagnostics. CXCL10 can be detected at or even below a concentration of 5pg/ml, for example by the LANCE Ultra human CXCL10 detection kit of Perkin Elmer.

The term "progenitor cell" as used herein relates to cells which are descendants of stem cells and which can further differentiate to create specialized cell types. There are many types of progenitor cells throughout the human body. Each progenitor cell is only capable of differentiating into cells that belong to the same tissue or organ. Some progenitor cells have one final target cell that they differentiate to, while others have the potential to terminate in more than one cell type. Progenitor cells are thus an intermediary cell type involved in the creation of mature cells in human tissues and organs, the blood, and the central nervous system. Hematopoietic progenitor cells are an intermediate cell type in blood cell development. They are immature cells that develop from hematopoietic stem cells and eventually differentiate into one of more than ten different types of mature blood cells.

The term "ex-vivo" as used herein means outside of a living body.

The term "in-vitro" as used herein means outside of a living body and within a laboratory environment. For example, cells which are cultured "in-vitro" are cultured in controlled, and often artificial, culture media.

As used herein, the term "pharmaceutically acceptable" embraces both human and veterinary use: for example the term "pharmaceutically acceptable" embraces a veterinary acceptable compound or a compound acceptable in human medicine and health care.

### Pharmaceutically acceptable salts:

In view of the close relationship between the CSFR1 agonists in their form as free compounds and the compounds in the form of their salts or solvates, whenever a compound is referred to in this context, a corresponding salt or solvate is also intended, provided such is possible or appropriate under the circumstances.

Salts and solvates of the CSFR1 agonists of the present invention and physiologically functional derivatives thereof which are suitable for use in medicine are those wherein the counter-ion or associated solvent is pharmaceutically acceptable. However, salts and solvates having non-pharmaceutically acceptable counter-ions or associated solvents are within the scope of the present invention, for example, for use as intermediates in the preparation of other compounds and their pharmaceutically acceptable salts and solvates.

Examples of solvates include hydrates.

All numerical designations, e.g., pH, temperature, time, concentration, and molecular weight, including ranges, are approximations which are varied (+) or (-) by increments of 0.1. It is to be understood, although not always explicitly stated that all numerical designations are preceded by the term "about." It also is to be understood, although not always explicitly stated, that the reagents described herein are merely examples and that equivalents of such are known in the art.

It is to be understood that this invention is not limited to the particular materials and methods described herein. It is also to be understood that the terminology used herein is for the purpose of describing particular embodiments and is not intended to limit the scope of the present invention, which will be limited only by the appended claims. As used herein, the singular forms "a", "an", and "the" include plural reference unless the context clearly indicates otherwise. Unless defined otherwise, all technical and scientific terms used herein have the same meanings as commonly understood by one of ordinary skill in the art to which this invention belongs. The following references provide one of skill with a general definition of many of the terms used in this invention - unless defined otherwise herein - and ranked in increasing order of priority: Singleton et al., Dictionary of Microbiology and Molecular Biology (3rd ed. 2006); The Glossary of Genomics Terms (JAMA. 2013; 309(14): 1533-1535), Janeway's Immunobiology, 9th edition and "Practical Flow Cytometry", 4th edition by H.M. Shapiro.

All publications mentioned herein are cited for the purpose of describing and disclosing the cell lines, protocols, reagents and vectors which are reported in the publications and which might be used in connection with the invention. Nothing herein is to be construed as an admission that the invention is not entitled to antedate such disclosure by virtue of prior invention.

### Preferred embodiments of the invention

In a first aspect, the invention provides an agonist of the colony stimulating factor 1 receptor (CSF1R) for use in the prophylaxis and/or treatment of viral infections in states of immunosuppression in a subject.

In a physiological setting, the CSF-1 receptor (CSF1R, also known as M-CSFR or CD115) is activated by the homodimeric growth factors colony-stimulating factor-1 (CSF-1, also called macrophage colony-stimulating factor, M-CSF) or interleukin-34 (IL-34). It plays important roles in development, innate immunity, tissue homeostasis and regeneration by regulating the development, survival, proliferation and function of monocytes, their bone marrow progenitors including hematopoietic stem cells, as well as monocyte derived macrophages. It is also expressed on tissue resident macrophages derived from embryonic progenitors or fetal monocytes. These monocyte- or embryonic progenitor-derived tissue macrophages are present in essentially every tissue and include specialized tissue macrophages such as osteoclasts, Langerhans cells of the skin, brain microglia and other brain macrophages. Owing to this specific expression pattern in macrophages and its progenitors and the broad presence of macrophages in essentially all tissues, it plays a central role in neoplastic, inflammatory, infectious, fibrotic and neurological diseases. It can also be aberrantly expressed in tumor cells and has been claimed in the past to play functional roles in Paneth cells, neural progenitors, oocytes and trophoblastic cells in the female reproductive tract (Stanley and Chitu), but these observations have not been substantiated by more recent approaches and might be due to secondary effects of tissue resident macrophages.

The evolution, structure, and regulation of expression of the CSF1R gene, the structures of CSF-1, IL-34, and the CSF1R as well as the mechanism of ligand binding to and activation of the receptor are known. There are pathways regulating macrophage function, survival, proliferation, differentiation, and chemotaxis downstream from the CSF1R

In one embodiment, the CSF1R agonist according to the invention is selected from natural and synthetic (artificial) ligands, e.g., cytokines, cytokine variants, analogues, muteins, and binding compositions derived from antibodies; small molecules, e.g., chemically produced active pharmaceutical ingredients, peptide mimetics of cytokines, peptide mimetics of antibodies, nucleic acids and nucleic acid mimetics.

The invention is based on the surprising finding that in a model of HCT, M-CSF/CSF-1 as a key cytokine for myelo-monocytic differentiation promoted rapid antiviral activity and protection from CMV viremia by stimulating coordinated myeloid and NK cell differentiation culminating in increased NK cell numbers and activation.

Therefore, the CSF1R agonists according to the invention is preferably selected from the ligands M-CSF and IL-34. In a most preferred embodiment, the CSF1R agonist is M-CSF. In a further most preferred embodiment of the invention, the CSF1R agonist is IL-34.

The genes for the two CSF1R ligands, CSF-1 and IL-34, are found on different chromosomes (chromosome 1 and 16 in humans) and are expressed *in vivo* from distinct promoters. The biological activities of homodimeric glycoprotein IL-34 resemble those of the secreted glycoprotein isoform of CSF-1. Although there are significant differences in their signaling through the CSF-1R, it is primarily the differential expression of IL-34 and CSF-1 that results in their differential spatiotemporal regulation through the CSF-1R *in vivo.* Circulating CSF-1 shows humoral regulation. In contrast, IL-34 is not detectable in the circulation of healthy individuals and thus IL-34 actions are likely to be restricted to the local microenvironments in which they are expressed. Through their different spatiotemporal expression, the two ligands play complementary roles in regulating the development, maintenance, and activity of specific macrophage populations like Langerhans cells, microglia and osteoclasts as well as potentially the regulation of cells of the female reproductive tract (Stanley and Chitu).

Said subject is preferably a mammal, most preferably a human.

Viral infections that can be treated with CSF1R agonists according to the invention are not restricted to CMV infections, but are selected from infections with any type of viruses that are capable of attacking animals. Preferably, the viral infections that can be treated with CSF1R agonists are selected from infections with
- dsDNA viruses (e.g. Adenoviruses, Herpesviruses, Poxviruses),
- ssDNA viruses (+ strand or "sense") DNA (e.g. Parvoviruses),
- dsRNA viruses (e.g. Reoviruses),
- (+)ssRNA viruses (+ strand or sense) RNA (e.g. Coronaviruses, Picornaviruses, Togaviruses),
- (-)ssRNA viruses (- strand or antisense) RNA (e.g. Orthomyxoviruses, Rhabdoviruses),
- ssRNA-RT viruses (+ strand or sense) RNA with DNA intermediate in life-cycle (e.g. Retroviruses),
- dsDNA-RT viruses DNA with RNA intermediate in life-cycle (e.g. Hepadnaviruses), wherein the abbreviations have the meaning as follows:
   - ds double strand,
   - ss single strand,
   - RT reverse transcriptase,
   - (-) negative sense,
   - (+) positive sense.

These types of viruses are known to the person skilled in the art.

More preferably, the viral infections that can be prevented and/or treated with CSF1R agonists are selected from viral infections that are associated with a state of immunosuppression in a subj ect.

Viruses are obligate intracellular parasites, relying to a major extent on the host cell for replication. An active replication of the viral genome results in the release of new progeny virus particles from the host cell by membrane budding, membrane perforation or the lysis of the host cell (lytic infection). Another mode of virus infection is a latent infection, where the virus is persisting in cells without or little replication. Virus can be maintained for example by integration into the host DNA (retroviruses) or episomally (Herpesviridiae like CMV). Latent viral persistence can be seen as an immune evasion mechanism, permitting the virus to survive an effective immune response. A combination of stages, where virus replication involves both silent and productive infection without rapidly killing or even producing excessive damage to the host cells, falls under the umbrella of a persistent or chronic infection. Reactivation is the process by which a virus switches from a latent to an active phase of replication. Reactivation may be provoked by a combination of external and/or internal cellular stimuli, in particular a weakened, failing or absent immune response as in an immunosuppressed subject.

"Associated" with a state of immunosuppression in a subject comprises new infections with one or more viruses as well as the reactivation of latent viruses.

In a preferred embodiment, said viral infection is activation of a latent virus in said subject. In particular, when said subject had been diagnosed as having a latent virus infection prior to induction of an immunosuppressed state in said subject, the use of the agonist of the CSF1-receptor according to the invention is a prophylactic use. An immunosuppressed state is, for example, induced prior to HCT when the recipient is conditioned for stem cell transplantation by chemotherapy and/or irradiation. An immunosuppressed state is, for example, also induced by chemotherapy and/or irradiation in, for example, cancer patients. In such cases, when the induction of an immunosuppressed state in a subject is intended in a subject, the present invention suggests that such subjects are examined for the presence of a latent virus infection prior to induction of the immunosuppressed state, and that those subjects that have been diagnosed as having a latent virus infection, will then receive treatment with the CSF1R agonist, such as treatment with M-CSF, after induction of the immunosuppressed state, e.g. after chemotherapy and/or irradiation, but prior to reactivation of the latent virus infection.

Even more preferably the viral infections that can be prevented and/or treated with CSF1R agonists in a state of immunosuppression in a subject are selected from viral infections that are associated with HCT, in particular in a subject which had been diagnosed as having a latent virus infection prior to induction of an immunosuppressed state in said subject.

Most preferably, the viral infections that can be prevented and/or treated with CSF1R agonists in a state of immunosuppression in a subject are selected from viral infections with CMV, HMPV, HBoV, HCoV-NL63, HCoV-HKU1, HHV-6, HHV-7, BK virus, JC virus, KI virus, WU virus, Epstein-Barr virus, adenovirus (ADV), in particular in a subject which had been diagnosed as having a latent virus infection prior to induction of an immunosuppressed state in said subject.

In a further preferred embodiment, said viral infection is an accidental infection, such as infection with a respiratory virus, and preferably the use of the agonist of the CSF 1-receptor is prophylactic use.

Said prophylactic use of the agonist of the CSF 1-receptor is suitably indicated for a period of increased risk of accidential infection with a respiratory virus, such as the respective "flu season" of the northern or southern hemisphere, or the months with increased rates of respiratory virus infections, for example the period from October to March in the northern hemisphere.

Further suitably, said prophylactic use is indicated in a country of increased risk of accidental infection with a respiratory virus, such as in a country comprising a region having a C, D or E climate according to the Köppen-Geiger classification, in particular indicated for the above period of increased risk of accidental infection with a respiratory virus. The Köppen-Geiger climate classification is e.g. described on the https://www.nationalgeographic.org/encyclopedia/koppen-climate-classification-system/ web page. Taking subjects intended for HCT or cancer subjects intended for chemotherapy as an example, the present invention suggests that such subjects should receive prophylactic treatment with an agonist of the CSF1-receptor, such as with M-CSF, after induction of the immunosuppressed state, e.g. after chemotherapy and/or irradiation, in order to avoid accidental infection with a respiratory virus country comprising a region having a C, D or E climate according to the Köppen-Geiger classification, such as the USA or Germany, and/or at a time of high risk, such from October to March.

In a further embodiment said prophylactic use is indicated during a pandemic or endemic, for example of a virus such for example SARS-CoV2, wherein it is preferably indicated for an immunosuppressed subject belonging to a high risk group for said pandemic or endemic virus, and/or belonging to the group of unvaccinated subjects.

In a further preferred embodiment, said viral infection is a viral infection that is derived from the transfer of donor material to said subject. Said donor material is e.g. selected from blood, plasma or cells. Preferably, said donor material are cells, most preferably hematopoietic stem cells, for example in the case of a recipient subject receiving HCT.

For example if the donor of the material intended to be administered to a subject where an immunosuppressed state is to be induced, for example a subject receiving HCT, had been diagnosed as having a latent virus infection, then the receiving subject should receive prophylactic treatment with an agonist of the CSF1-receptor, such as with M-CSF, after induction of the immunosuppressed state, e.g. after chemotherapy and/or irradiation, in order to avoid infection with a latent virus coming from the donor material. Preferably, when the donor had been diagnosed as having a latent virus infection, the use of the agonist of the CSF1-receptor is a prophylactic use.

Said latent virus in the donor is, e.g. selected from CMV, HMPV, HBoV, HCoV-NL63, HCoV-HKU1, HHV-6, HHV-7, BK virus, JC virus, KI virus, WU virus, Epstein-Barr virus and adenovirus (ADV). More preferably, said latent virus in the donor is CMV, Eppstein-Barr virus, adenovirus, HHV-6, BK virus or JC virus.

More preferably, the state of immunosuppression is hematopoietic stem cell transplantation.

Most preferably, the viral infection that can be prevented and/or treated with CSF1R agonists is CMV viremia after HCT, most preferably during leukopenia after HCT. The subject is preferably a mammalian subject, most preferably a human subject.

The major histocompatibility complex (MHC) class II, for example as detectable on CD14+ monocytes by expression of human leukocyte antigen receptors (HLA-DR), is essential for the immunological synapse between innate and adaptive immune cells mediating an immune response in infectious disease. Its reduced expression is associated with a high risk of secondary infections in septic patients. In another example, it has been shown that coronavirus disease (COVID-19) induces an alteration of Interferon (IFN) genes expression potentially responsible for the observed low HLA-DR expression in circulating monocytes (mHLA-DR) (Dickel et al.).

M-CSF was used as a drug (Leucoprol ^{®}) in cancer treatment / cancer management in connection with chemotherapy. When the state of immunosuppression in a subject is associated with the disorder "cancer", the present invention preferably relates to a situation, where said viral infection is activation of a latent virus in said subject and wherein said subject had been diagnosed as having a latent virus infection prior to induction of an immunosuppressed state in said subject, and wherein the use of the agonist of the CSF1-receptor is a prophylactic use.

M-CSF was used as a drug (Leucoprol ^{®}) in HCT and/or bone marrow transplantation. When the state of immunosuppression in a subject is associated with HCT and/or bone marrow transplantation, the present invention preferably relates to a situation, where said viral infection is activation of a latent virus in said subject and wherein said subject had been diagnosed as having a latent virus infection prior to induction of an immunosuppressed state in said subject, and wherein the use of the agonist of the CSF1-receptor is a prophylactic use.

As already mentioned hereinabove, the invention is based on the surprising finding that in a model of HCT, M-CSF/CSF-1 as a key cytokine for myelo-monocytic differentiation promoted rapid antiviral activity and protection from CMV viremia by stimulating coordinated myeloid and NK cell differentiation culminating in increased NK cell numbers and activation.

It has further been found that said NK cell number increase is both due to donor-derived cells and host-derived cells with a major proportion coming from donor-derived cells, wherein said CSF1R agonist treatment increases the number of donor- and host-derived CD122⁺CD27⁺ CD11b- NK progenitor cells. Thus, in a further embodiment, the present invention provides said CSF1R agonist for the use of the invention, characterized in that said NK cell number increase is due to donor- and/or host-derived cells, wherein said CSF1R agonist treatment increases the number of donor and/or host-derived CD122⁺CD27⁺ NK progenitor cells.

Moreover, said CSF1R agonist treatment increases the number of donor- and host- derived NK1.1+, NKp46+, CD122+, CD27+ , CD11b- immature NK cells as well as mature NK1.1-, NKp46-, CD122+, CD27+, CD11b- M1 and NK1.1-, NKp46-, CD122+, CD27-, CD11b+ M2 NK cells. Accordingly, in a further embodiment, the present invention provides said CSF1R agonist for the use of the invention, characterized in that said CSF1R agonist treatment increases the number of donor- and/or host-derived immature as well as mature M1 and M2 NK cells.

Said NK cells, the number of which is increased by CSF1R agonist treatment, were shown to be NK1.1+ cells, preferably from spleen, which show increased expression of the immature NK cell transcription factors *IKZF1, ID2, RUNX3, GATA3* and *TBX21* as well as the mature NK cell factor *EOMES.* Accordingly, the invention provides in a further embodiment said CSF1R agonist for the use of the invention, characterized in that said NK cells are selected from spleen NK1.1+ cells, wherein said spleen NK1.1+ cells show increased expression of the immature NK cell transcription factors *IKZF1, ID2, RUX3, GATA3* and *TBX21* as well as the mature NK cell factor *EOMES.*

Moreover, it has been found that the treatment with a CSF1R agonist increases the number of IFN-γ and granzyme B producing NK cells, preferably in the spleen. Accordingly, the invention provides in a further embodiment said CSF1R agonist for the use of the invention, characterized in that said CSF1R agonist treatment increases the number of IFN-γ and granzyme B producing NK cells, preferably in the spleen.

The inventors could further show that the treatment with a CSF1R agonist increases the mRNA levels encoding the genes *IFNG, GZMB* and *PRF1* as well as the maturation and activation marker genes *CEBPA, MITF* and *XCL1* in the spleen. Thus, in a further embodiment, the invention provides said CSF1R agonist for the use of the invention, characterized in that said CSF1R agonist treatment increases the mRNA levels encoding the genes *IFNG, GZMB* and *PRF1* as well as the maturation and activation marker genes *CEBPA, MITF* and *XCL1* in the spleen.

As already mentioned hereinabove, the invention is based on the surprising finding that in a model of HCT, M-CSF/CSF-1 as a key cytokine for myelo-monocytic differentiation promoted rapid antiviral activity and protection from CMV viremia by stimulating coordinated myeloid and NK cell differentiation culminating in increased NK cell numbers and activation. This NK cell response was dependent on M-CSF-induced myelopoiesis and IL-15 production in monocytes. Furthermore, M-CSF also induced differentiation of plasmacytoid dendritic cells producing I-IFN, which was required for IL-15 mediated protection.

Accordingly, the invention provides said CSF1R agonist for the use of the invention, wherein said CSF1R agonist treatment increases donor myelopiesis, characterized in that said CSF1R agonist treatment increases the number of myeloid cells selected from granulocyte-monocyte progenitor (GMP) cells, granulocytes, mononuclear phagocytes, plasmacytoid dendritic cells (pDC) and/or classical dendritic cells (cDC).

In another embodiment, the invention provides said CSF1R agonist for the use of the invention, characterized in that said CSF1R agonist treatment increases IL-15 production, for example in the spleen.

Said increased IL-15 production is accompanied by increased expression of IL-15Rα on monocytes, a molecule that is required for 11-15 presentation to target cells. Therefore, the invention provides in a further embodiment said CSF1R agonist for the use of the invention, characterized in that said increased IL-15 production is accompanied by induction and presentation of IL-15Rα on said monocytes.

Said increased IL-15 production and Il15Rα presentation in said monocytes leads to increased expression of *IL15RB, STAT5B, JAK3* and *E2F1* (*E2f1-6* in mice) in NK cells after signal transmission from the IL-15/L-15Rα complex on the presenting monocytes, through IL-15Rβ on the NK cells. The invention thus provides in a further embodiment said CSF1R agonist for the use of the invention, characterized in that said increased IL-15 production and Il15Rα presentation in said monocytes leads to increased expression of *IL15RB, STAT5B,* JAK3 and E2F1 (*E2f1-6* in mice) in NK cells after signal transmission from the IL-15/L-15Rα complex on the presenting monocytes, through IL-15Rβ on the NK cells.

The CSF1R agonist treatment further induces *IFNB1* gene expression and IFN-I production in pDCs, wherein said IFN-I stimulates IL-15 production in myeloid cells. Accordingly, the invention provides said CSF1R agonist for the use of the invention, characterized in that said CSF1R agonist treatment further induces *IFNB1* gene expression and IFN-I production in pDCs, wherein said IFN-I stimulates IL-15 production in myeloid cells.

Taken together, the inventors could show for the first time that CSF1R agonist treatment, such as M-CSF treatment, induces an integrated multistep differentiation cascade that culminated in increased NK cell generation and activation protecting graft recipients from a viral infection, such as a CMV infection. The experimental results as described in the working examples below, identify a mechanism by which M-CSF-induced myelopoiesis can rapidly reconstitute antiviral activity under immunosuppressed conditions, which provides a general paradigm to boost innate antiviral immune response.

In a further embodiment, the invention provides the use of an agonist of the colony stimulating factor 1 receptor (CSF1R) as described herein for the preparation of a medicament for the prophylaxis and/or treatment of viral infections in states of immunosuppression in a subject. In particular, the invention provides the use of an agonist of the colony stimulating factor 1 receptor (CSF1R), as described herein, such as M-CSF, for the preparation of a medicament for the prophylaxis of activation of a latent virus in a subject, wherein said subject is diagnosed as carrying a latent virus and wherein a state of immunosuppression is induced in said subject, for example for HCT.

In a further embodiment, the invention provides a method of prophylaxis and/or treatment of viral infections in states of immunosuppression in a subject, said method comprising the administering of a therapeutically effective amount of an agonist of the colony stimulating factor 1 receptor (CSF1R) as described herein, to a subject in need thereof. In particular the invention provides a method of prophylaxis of activation of a latent virus in a subject in induced states of immunosuppression in a subject, said method comprising the diagnosis of the subject as being the carrier of a latent virus and the administering of a therapeutically effective amount of an agonist of the colony stimulating factor 1 receptor (CSF1R) as described herein, to a subject in need thereof.

In a second aspect the invention provides a pharmaceutical composition comprising the CSF1R agonist as described herein, for use in the prophylaxis and/or treatment of viral infections in states of immunosuppression in a subject. In particular, the invention provides a pharmaceutical composition comprising the CSF1R agonist as described herein, such as comprising M-CSF, for use in the prophylaxis of activation of a latent virus in a subject, wherein said subject is diagnosed as carrying a latent virus and wherein a state of immunosuppression is induced in said subject, for example for HCT.

The CSF1R agonists of the invention may be formulated for administration to a subject using techniques known to the skilled artisan. Formulations comprising CSF1R agonists may include pharmaceutically acceptable excipient(s). Examples of generally used excipients include, without limitation: saline, buffered saline, dextrose, water- for- infection, glycerol, ethanol, and combinations thereof, stabilizing agents, solubilizing agents and surfactants, buffers and preservatives, tonicity agents, bulking agents, and lubricating agents. The formulations comprising CSF1R agonists will typically have been prepared in the absence of any non-human components, such as animal serum (e.g., bovine serum albumin).

The choice of formulation, i.e. type of pharmaceutical composition for administering CSF1R agonists for a given application will depend on a variety of factors. Prominent among these will be the species of subject, the nature of the disorder, dysfunction, or disease being treated and its state and distribution in the subject, the nature of other therapies and agents that are being administered, the optimum route for administration of the CSF1R agonists, the dosing regimen, and other factors that will be apparent to those skilled in the art. In particular, for instance, the choice of suitable carriers and other additives will depend on the exact route of administration and the nature of the particular dosage form, for example, liquid dosage form (e.g., whether the composition is to be formulated into a solution, a suspension, gel or another liquid form, such as a time release form or liquid-filled form).

Examples of compositions comprising CSF1R agonists include liquid preparations, including suspensions and preparations for intramuscular or intravenous administration (e.g., injectable administration), such as sterile emulsions. Such compositions may comprise an admixture of CSF1R agonists with a suitable carrier, diluent, or excipient such as sterile water, physiological saline, glucose, dextrose, or the like. The compositions can contain auxiliary substances such as wetting or emulsifying agents, pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE," 17th edition, 1985, incorporated herein by reference, may be consulted to prepare suitable preparations, without undue experimentation.

Various additives often will be included to enhance the stability, sterility, and isotonicity of the compositions, such as antimicrobial preservatives, antioxidants, chelating agents, and buffers, among others. Prevention of the action of microorganisms can be ensured by various antibacterial and antifungal agents, which are known to the person skilled in the art. In many cases, it will be desirable to include isotonic agents, for example, sugars, sodium chloride, and the like. Prolonged absorption of the injectable pharmaceutical form can be brought about by the use of agents that delay absorption, for example, aluminum monostearate, and gelatin.

According to the present invention, however, any vehicle, diluent, or additive used would have to be compatible with the nature of the CSF1R agonists (i.e. peptide vs. small molecule).

Typically, the compositions will be isotonic, i.e., they will have the same osmotic pressure as blood and lacrimal fluid when properly prepared for administration.

The desired isotonicity of the compositions of this invention maybe accomplished using sodium chloride, or other pharmaceutically acceptable agents such as dextrose, boric acid, sodium tartrate, propylene glycol, or other inorganic or organic solutes. Sodium chloride is preferred particularly for buffers containing sodium ions.

Viscosity of the compositions, if desired, can be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the agent selected. The important point is to use an amount, which will achieve the selected viscosity. Viscous compositions are normally prepared from solutions by the addition of such thickening agents.

Those skilled in the art will recognize that the components of the compositions should be chemically inert. This will present no problem to those skilled in chemical and pharmaceutical principles. Problems can be readily avoided by reference to standard texts or by simple experiments (not involving undue experimentation) using information provided by the disclosure, the documents cited herein, and generally available in the art.

Sterile injectable solutions can be prepared by incorporating the CSF1R agonists utilized in practicing the present invention in the required amount of the appropriate solvent with various amounts of the other ingredients, as desired.

In some embodiments, CSF1R agonists are formulated in a unit dosage injectable form, such as a solution, suspension, or emulsion. Pharmaceutical formulations suitable for injection of CSF1R agonists typically are sterile aqueous solutions and dispersions. Carriers for injectable formulations can be a solvent or dispersing medium containing, for example, water, saline, phosphate buffered saline, polyol (for example, glycerol, propylene glycol, liquid polyethylene glycol, and the like), and suitable mixtures thereof.

The skilled person can readily determine the amount of CSF1R agonists and optional additives, vehicles, and/or carrier in compositions to be administered in methods of the invention. Typically, any additives (in addition to the CSF1R agonists) are present in an amount of 0.001 to 50 wt % in solution, such as in phosphate buffered saline. The active ingredient is present in the order of micrograms to milligrams, such as about 0.0001 to about 5 wt %, preferably about 0.0001 to about 1 wt %, most preferably about 0.0001 to about 0.05 wt % or about 0.001 to about 20 wt %, preferably about 0.01 to about 10 wt %, and most preferably about 0.05 to about 5 wt %.

Order of administration, formulations, doses, frequency of dosing, and routes of administration of CSF1R agonists generally will vary with the disorder or disease being treated, its severity, the subject, other therapies that are being administered, the stage of the disorder or disease, and prognostic factors, among others. General regimens that have been established for other treatments provide a framework for determining appropriate dosing in CSF1R agonist-mediated direct or adjunctive therapy. These, together with the additional information provided herein, will enable the skilled artisan to determine appropriate administration procedures in accordance with embodiments of the invention, without undue experimentation.

For therapeutic applications, CSF1R agonists can be administered to a subject by any of a variety of routes known to those skilled in the art that may be used to administer CSF1R agonists to a subject. Among methods that may be used in this regard in embodiments of the invention are methods for administering CSF1R agonists by a parenteral route. Parenteral routes of administration useful in various embodiments of the invention include, among others, administration by intravenous, intraarterial, intracardiac, intra- articular (joint), intraspinal, intrathecal (spinal fluids), intraosseous, intraarticular, intrasynovial (joint fluid area), intracutaneous, intradermal, subcutaneous (s.c, s.q., sub-Q, Hypo), and/or intramuscular injection. Any known device useful for parenteral injection or infusion of the formulations can be used to effect such administration. Injections can be performed as bulk injections or continuous flow injections. In some embodiments intravenous, intraarterial, intracutaneous, intradermal, subcutaneous and/or intramuscular injection are used. In some embodiments intravenous, intraarterial, intracutaneous, subcutaneous, and/or intramuscular injection are used. In various embodiments of the invention CSF1R agonists are administered by systemic injection. Systemic injection, such as intravenous injection, offers one of the simplest and least invasive routes for administering CSF1R agonists. In a variety of embodiments CSF1R agonists may be administered by targeted and/or localized injections to ensure optimum effect at the target sites.

CSF1R agonists may be administered to the subject through a hypodermic needle by a syringe in some embodiments of the invention. In various embodiments, CSF1R agonists are administered to the subject through a catheter. In a variety of embodiments, CSF1R agonists are administered by surgical implantation.

In additional embodiments of the invention, CSF1R agonists are suitably formulated for oral, rectal, epicutaneous, intraocular, nasal, and/or pulmonary, delivery and are administered accordingly.

Preferably, the pharmaceutical composition comprising a CSF1R agonists of the invention is an injectable. More preferably, the pharmaceutical composition comprising a CSF1R agonists of the invention is administered parenterally. Most preferably, the pharmaceutical composition comprising a CSF1R agonists of the invention is administered subcutaneously or intravenously.

Compositions can be administered in dosages and by techniques well known to those skilled in the medical and veterinary arts taking into consideration such factors as the age, sex, weight, and condition of the particular patient, and the formulation that will be administered (e.g., solid vs. liquid). Doses for humans or other mammals can be determined without undue experimentation by the skilled artisan, from this disclosure, the documents cited herein, and the knowledge in the art. The optimal dose of CSF1R agonists for some embodiments will be in the range of doses used for antiviral immunotherapy.

It is to be appreciated that a single dose may be delivered all at once, fractionally, or continuously over a period of time. The entire dose also may be delivered to a single location or spread fractionally over several locations. In various embodiments, CSF1R agonists may be administered in an initial dose, and thereafter maintained by further administration of CSF1R agonists. CSF1R agonists may be administered by one method initially, and thereafter administered by the same method or one or more different methods. The subject's levels of CSF1R agonists can be maintained by the ongoing administration of the CSF1R agonists. Various embodiments administer the CSF1R agonists either initially or to maintain their level in the subject or both by intravenous injection. In a variety of embodiments, other forms of administration are used, dependent upon the patient's condition and other factors, discussed elsewhere herein. In some embodiments CSF1R agonists are administered to a subject in one dose. In others CSF1R agonists are administered to a subject in a series of two or more doses in succession. In some other embodiments wherein CSF1R agonists are administered in a single dose, in two doses, and/or more than two doses, the doses may be the same or different, and they are administered with equal or with unequal intervals between them. In some embodiments, CSF1R agonists are administered over a period of less than one day. In other embodiment they are administered over two, three, four, five, or six days. In some embodiments CSF1R agonists are administered one or more times per week, over a period of weeks. In other embodiments they are administered over a period of weeks for one to several months. In various embodiments they may be administered over a period of months. In others they may be administered over a period of one or more years.

In a preferred embodiment, the CSF1R agonist is M-CSF or IL-34 or functional equivalent thereof. M-CSF, also known as millimostim or miromistim, is the active ingredient in the prescribed drug Leucoprol ^{®}. Leucoprol ^{®} is prescribed for the promotion of the increase in granulocyte count after bone marrow transplantation, in ovarian cancer and acute myeloid leukemia after repeated administration of antineoplastic agents and

Leucoprol ^{®} contains the following incredients:

| | |
|---|---|
| M-CSF (Millimostim) | 8 million units (extract from human urine) |
| Human serum albumin | 32 mg |
| Disodiumhydrogenphosphate hydrate | 118.4 mg |
| Sodium dihydrogen phosphate | 11.2 mg |
| D-mannitol | 400 mg |

In a preferred embodiment, the CSF1R agonist of the invention is provided as active ingredient in a pharmaceutical composition as described for Leucoprol ^{®}. The CSF1R agonist as active ingredient in this pharmaceutical composition is preferably either M-CSF or IL-34 or a functional derivative thereof, wherein said pharmaceutical composition comprises said agonist of CSF1R in an amount that has a specific activity comparable to 6 to 10 million units of M-CSF, preferably comparable to 8 million units of M-CSF from human urine..

In a more preferred embodiment, the pharmaceutical composition according to the invention comprises 6 to 10 million units of a M-CSF polypeptide, preferably 8 million units of a M-CSF polypeptide, in an amount that has a specific activity comparable to 6 to 10 million units of M-CSF, preferably comparable to 8 million units of M-CSF from human urine.

Most preferably, the pharmaceutical composition according to the invention comprises 6 to 10 million units of M-CSF, preferably 8 million units of M-CSF, wherein said M-CSF had been extracted from human urine.

For use, this pharmaceutical composition is typically dissolved in an appropriate amount of physiological saline solution, diluted to 200 to 500 ml of infusion solution, and intravenously infused over 30 minutes per 100 ml.

In general, for adults, 8 million units of M-CSF are administered once daily, for example in the case of HCT preferably on the day prior to, on the day of and on the day after HCT. In either case, the dose may be adjusted according to the patient's age and symptoms.

Accordingly, the invention provides a CSF1R agonist or a pharmaceutical composition comprising the same for use, characterized in that 8 million units of said CSF1R agonist, when extracted from human urine, or an equivalent to 8 million units M-CSF, preferably with a comparable efficacy, are administered to a subject once daily, for example in the case of HCT preferably on the day prior to, on the day of and on the day after HCT. This pharmaceutical composition can further comprise another agent, such as an immunosuppressive drug or an antiviral drug, as discussed above.

In a further preferred embodiment, the pharmaceutical composition of the invention is administered to the subject as infusion.

The pharmaceutical composition according to the invention is, for example, for prevention of the activation of a latent virus in a subject undergoing hematopoietic stem cell transplantation (HCT) which has been tested positive for a latent virus, preferably wherein the pharmaceutical composition is administered once daily, for example in the case of HCT preferably on the day prior to, on the day of and on the day after HCT.

The pharmaceutical composition according to the invention is, for prevention of a new viral infection, preferably wherein the pharmaceutical composition is administered once daily, for example in the case of HCT preferably on the day prior to, on the day of and on the day after HCT.

The pharmaceutical composition according to the invention is, for treatment of a new viral infection, administered to a subject that had received a HCT, preferably once daily, for example for a period of up to two weeks after diagnosing said new viral infection in said subject.

The invention further relates to a method of prophylactic treatment of viral infections in states of immunosuppression in a subject, comprising administering an agonist of CSF1R as described herein, or a pharmaceutical composition comprising an agonist of CSF1R as described herein, in a therapeutically effective amount to a subject in need thereof.

The invention further relates to the use of an agonist of the CSF1-receptor as described herein, or of a pharmaceutical composition comprising an agonist of CSF1R as described herein for the preparation of a medicament for the prophylaxis and/or treatment of viral infections in states of immunosuppression in a subject.

CSF1R agonists may be administered after pretreatment with immunosuppressive agents to prevent virus infection and graft rejection at the same time, such as a corticosteroid, cyclosporin A, a cyclosporin-like immunosuppressive agent, cyclophosphamide, antithymocyte globulin, azathioprine, rapamycin (also known as Sirolimus), FK-506 (also known as Tacrolimus), and a macrolide-like immunosuppressive agent other than FK-506 and rapamycin. Immunosuppressive agents in accordance with the foregoing may be the only such additional agents or may be combined with other agents, such as other agents noted herein. Other immunosuppressive agents include Mycophenolate mofetil.

Such other agents also include antibiotic agents, antifungal agents, and antiviral agents, to name just a few other pharmacologically active substances and compositions that maybe used in accordance with embodiments of the invention. Preferred according to the invention is a combination of the CSF1R agonists with antiviral agents to improve the antiviral activity.

Antiviral agents are preferably selected from letermovir, acyclovir, cidofovir, ganciclovir, idoxuridine, penciclovir, valganciclovir, valacyclovir, vidarabine, amantadine, rimantadine, zanamivir, fomivirsen, imiquimod, ribavirin, nucleoside reverse transcriptase inhibitors (e. g., zidovudine, didanosine, stavudine, zalcitabine, lamividudine ), non-nucleoside reverse transcriptase inhibitors (e.g., nevirapine, efavirenz, delvirudine ), and protease inhibitors (e.g., saquinivir, indinavir, ritonavir, nelfinavir, amprenavir, and lopinavir).

Anti-fungal compounds may be a systemic antifungal agent. One useful antifungal compound of this type is amphotericin B from the family of polyene macrolide antibiotics. Another antifungal compound is flucytosine, a fluorinated pyrimidine. Deamination of flucytosine by the fungus generates 5-flurouracil, an anti-metabolite and DNA synthesis inhibitor. Flucytosine is typically used for infections of cryptococcus and candiadosis. Although used alone, flucytosine is generally used in combination with amphotericin B. Imidazoles and triazoles represent a broad class of azole based antifungal compounds. Exemplary azole antifungals include, among others, ketoconzaole, itracanazole, fluconazole, econazole, voriconazole, and tercanozole.

Anti-bacterial compounds may be antibiotics suitable for the particular bacterial pathogen. These include both wide spectrum antibiotics and more targeted anti-bacterial compounds. Various classes of anti-bacterial compounds are, by way of example and not limitation, quinolones and fluoroquinolones, [beta]-lactam antibiotics, aminoglycosides, tetracycline, macrolides, and various cogeners thereof Exemplary quinolone compounds include ciprofloxacin, ofloxacin, sparfloxacin, lomefloxacin, and moxifioxacin. Exemplary [beta]-lactam antibiotics include penicillins (e.g., penicillin G, penicillin V), ampicillin, carbenicillin, methicillin, carbapenem, and cephalosporins (e.g., cephalothin, cefamandole, cefaclor, cefonicid, cefotetan, cefatoxime, ceftazidime, ceftizoxime, cefepime ). Exemplary aminoglycosides include neomycin, streptomycin, kanamycin, gentamicin, tobramycin, amikacin, and netilmicin. Exemplary macrolides include erythromycin, clarithromycin, and azithromycin.

The invention further relates to the following embodiments:
1. An agonist of the CSF1-receptor for use in the prophylaxis and/or treatment of viral infections in a disorder characterized by a state of immunosuppression in a subject.
2. The agonist of the CSF1-receptor for use according to embodiment 1, wherein said viral infection is activation of a latent virus in said subject.
3. The agonist of the CSF1-receptor for use according to embodiment 2, wherein said subject had been diagnosed as having a latent virus infection and wherein the use of the agonist of the CSF 1-receptor is prophylactic use.
4. The agonist of the CSF1-receptor for use according to embodiment 2 or 3, wherein said latent virus is selected from CMV, HMPV, HBoV, HCoV-NL63, HCoV-HKU1, HHV-6, HHV-7, BK virus, JC virus, KI virus, WU virus, Epstein-Barr virus and adenovirus (ADV).
5. The agonist of the CSF1-receptor for use according to embodiment 4, wherein said latent virus is CMV, Eppstein-Barr virus, adenovirus, HHV-6, BK virus or JC virus.
6. The agonist of the CSF1-receptor for use according to any one of embodiments 2 to 5, wherein the state of immunosuppression is induced.
7. The agonist of the CSF1-receptor for use according to embodiment 6, wherein the subject has been diagnosed as being a carrier of a latent virus.
8. The agonist of the CSF1-receptor for use according to embodiment 7, wherein the subject has been diagnosed as being a carrier of a latent virus prior to the induction of immunosuppression.
9. The agonist of the CSF1-receptor for use according to embodiment 8, wherein the disorder is hematopoietic stem cell transplantation.
10. The agonist of the CSF1-receptor for use according to embodiment 8, wherein the induced state of immunosuppression is chemotherapy.
11. The agonist of the CSF 1-receptor for use according to any one of embodiments 7 to 10, wherein the latent virus is CMV.
12. The agonist of the CSF1-receptor for use according to embodiment 1, wherein said viral infection is an accidential infection.
13. The agonist of the CSF1-receptor for use according to embodiment 12, wherein said accidental viral infection is infection with a respiratory and/or intestinal virus.
14. The agonist of the CSF1-receptor for use according to embodiment 13, wherein said respiratory virus is selected from respiratory syncytial virus (RSV), parainfluenza virus, rhinovirus, and influenza virus.
15. The agonist of the CSF1-receptor for use according to embodiments 13 or 14, wherein the state of immunosuppression is hematopoietic stem cell transplantation and wherein the use of the agonist of the CSF 1-receptor is prophylactic use.
16. The agonist of the CSF1-receptor for use according to embodiment 15, wherein the prophylactic use is indicated for a period of increased risk of accidential infection with a respiratory virus.
17. The agonist of the CSF1-receptor for use according to embodiments 15 or 16, wherein the prophylactic use is indicated in a country of increased risk of accidential infection with a respiratory virus.
18. The agonist of the CSF1-receptor for use according to embodiment 17, wherein the country of increased risk of accidential infection with a respiratory virus is a country comprising a region having a C, D or E climate according to the Koppen-Geiger classification.
19. The agonist of the CSF1-receptor for use according to embodiment 1, wherein said viral infection is derived from the transfer of donor material to said subject.
20. The agonist of the CSF1-receptor for use according to embodiment 19, wherein said donor material is blood, plasma or cells.
21. The agonist of the CSF1-receptor for use according to embodiment 20, wherein the cells are hematopoietic stem cells and wherein the disorder is hematopoietic stem cell transplantation.
22. The agonist of the CSF1-receptor for use according to embodiment 21, wherein the donor had been diagnosed as having a latent virus infection.
23. The agonist of the CSF1-receptor for use according to embodiment 22, wherein the use of the agonist of the CSF 1-receptor is prophylactic use.
24. The agonist of the CSF1-receptor for use according to embodiments 22 or 23, wherein said latent virus is selected from CMV, HMPV, HBoV, HCoV-NL63, HCoV-HKU1, HHV-6, HHV-7, BK virus, JC virus, KI virus, WU virus, Epstein-Barr virus and adenovirus (ADV).
25. The agonist of the CSF1-receptor for use according to embodiment 24, wherein the virus is CMV, Eppstein-Barr virus, adenovirus, HHV-6, BK virus or JC virus.
26. The agonist of the CSF1-receptor for use according to any one of embodiments 1 to 25, wherein the subject is a human subject.
27. The agonist of the CSF1-receptor for use according to any one of embodiments 1 to 25, wherein the agonist is a M-CSF polypeptide, IL-34 polypeptide or a functional equivalent of said M-CSF polypeptide or IL-34 polypeptide.
28. The agonist of the CSF1-receptor for use according to embodiment 27, wherein said M-CSF polypeptide or IL-34 polypeptide is naturally occurring, or a synthetic or recombinant variant thereof, or a modified form thereof, or a biologically active fragment thereof.
29. The agonist of the CSF1-receptor for use according to embodiment 27 or 28, wherein said M-CSF polypeptide is isolated from a bacterial or eukaryotic cell culture.
30. The agonist of the CSF1-receptor for use according to embodiment 28 or 29, wherein said M-CSF polypeptide is an allelic form or isoform of M-CSF.
31. The agonist of the CSF1-receptor for use according to any one of embodiments 27 to 30, wherein said M-CSF polypeptide a polypeptide comprising or consisting of an amino acid sequence selected from SEQ ID NO. 1, 2, 3, 4 or 5 or a polypeptide with at least 80 % sequence identity to a polypeptide of SEQ ID NO. 1, 2, 3, 4 or 5.
32. The agonist of the CSF1-receptor for use according to any one of embodiments 27 to 31, wherein said M-CSF polypeptide is of human origin.
33. The agonist of the CSF1-receptor for use according to any one of embodiment 27 or 28, wherein said IL-34 polypeptide is an allelic form or isoform of IL-34.
34. The agonist of the CSF1-receptor for use according to embodiment 33, wherein said IL-34 polypeptide is a polypeptide comprising or consisting of an amino acid sequence of SEQ ID NO. 6 or a polypeptide with at least 80 % sequence identity to a polypeptide of SEQ ID NO. 6.
35. The agonist of the CSF1-receptor for use according to embodiment 33 or 34, wherein said IL-34 polypeptide is of human origin.
36. The agonist of the CSF1-receptor for use according to any one of the preceding embodiments, wherein said agonist of the CSF1-receptor shows a specific activity with an ED50 in the range of 0.5 to 2 ng/ml in a proliferation assay using mouse M-NSF-60 cells.
37. The agonist of the CSF1-receptor for use according to any one of the preceding embodiments, wherein said agonist of the CSF1-receptor shows a specific activity of at least 1 × 10³ units (hM-CSF equivalent)/mg(agonist), such as at least 1 × 10⁴ units/mg, for example at least 1 × 10⁵ units/mg, in a proliferation assay using mouse M-NSF-60 cells.
38. The agonist of the CSF1-receptor for use according to any one of the preceding embodiments, wherein said CSF1R agonist treatment stimulates a NK cell differentiation resulting in increased NK cell numbers and activation.
39. The agonist of the CSF1-receptor for use according to any one of the preceding embodiments, wherein said CSF1R agonist treatment increases the number of donor-derived CD122⁺CD27⁺ NK progenitor cells.
40. The agonist of the CSF1-receptor for use according to any one of the preceding embodiments, wherein said CSF1R agonist treatment increases the number of donor-derived immature as well as mature M1 and M2 NK cells.
41. The agonist of the CSF1-receptor for use according to any one of the preceding embodiments, wherein said CSF1R agonist treatment further increases the number of resident host NK cells.
42. The agonist of the CSF1R for use according to any one of embodiments 38 to 41, wherein said NK cells are selected from spleen NK1.1+ cells wherein said spleen NK1.1+ cells show increased expression of the immature NK cell transcription factors *IKZF1, ID2, RUX3, GATA3* and *TBX21* as well as the mature NK cell factor *EOMES.*
43. The agonist of the CSF1R for use according to any one of embodiments 38 to 41, wherein said CSF1R agonist treatment increases the number of IFN-γ and granzyme B producing NK cells, preferably in the spleen.
44. The agonist of the CSF1R for use according to any one of embodiments 38 to 43, wherein said CSF1R agonist treatment increases the mRNA levels encoding the genes *IFNG, GZMB* and *PRF1* as well as the maturation and activation marker genes *CEBPA, MITF* and *XCL1* in NK cells, preferably in the spleen.
45. The agonist of the CSF1R for use according to any one of embodiments 38 to 44, wherein said CSF1R agonist treatment increases donor myelopiesis, characterized in that said CSF1R agonist treatment increases the number of myeloid cells selected from granulocyte-monocyte progenitor (GMP) cells, granulocytes, mononuclear phagocytes, plasmacytoid dendritic cells (pDC) and/or classical dendritic cells (cDC).
46. The agonist of the CSF1R for use according to any one of embodiments 38 to 45, wherein said CSF1R agonist treatment increases IL-15 production.
47. The agonist of CSF1R for use according to embodiment 46, wherein said CSF1R agonist treatment leads to an induction of IL-15Rα and 11-15 presentation on said monocytes.
48. The agonist of CSF1R for use according to embodiment 46 or 47, wherein said increased of IL-15Rα production and IL-15 presentation in said monocytes leads to increased expression of at least one of *Il15rb, STAT5B, JAK3* and E2f1-6 in NK cells after signal transmission from the IL-15/L-15Rα complex on the presenting monocytes, through IL-15Rβ on the NK cells.
49. The agonist of CSF1R for use according to any one of the preceding embodiments, wherein said CSF1R agonist treatment further induces *IFNB1* gene expression and IFN-I production in pDCs, wherein said IFN-I stimulates IL-15 production in myeloid cells.
50. A pharmaceutical composition comprising the agonist of the CSF1-receptor according to any one of the preceding embodiments, for use in the prophylaxis and/or treatment of viral infections in a disorder characterized by a state of immunosuppression in a subject.
51. The pharmaceutical composition according to embodiment 50, wherein the pharmaceutical composition is an injectable.
52. The pharmaceutical composition according to embodiment 50 or 51, wherein the pharmaceutical composition is administered parenterally.
53. The pharmaceutical composition according to any one of embodiments 50 to 52, wherein the pharmaceutical composition is administered subcutaneously or intravenously.
54. The pharmaceutical composition according to any one of embodiments 50 to 53, comprising said agonist of CSF1R in an amount that has a specific activity comparable to 6 to 10 million units of human M-CSF, preferably to 8 million units of M-CSF that is extracted from human urine.
55. The pharmaceutical composition according to any one of embodiments 50 to 54, comprising 6 to 10 million units of M-CSF, preferably 8 million units of M-CSF, wherein said M-CSF is obtainable from a bacterial or eukaryotic cell culture.
56. The pharmaceutical composition according to any one of embodiments 50 to 55, further comprising human serum albumin, disodiumhydrogenphosphate hydrate, sodium dihydrogen phosphate and D-mannitol.
57. The pharmaceutical composition according to any one of embodiments 50 to 56, comprising

| | |
|---|---|
| Human M-CSF | 8 million units, |
| Human serum albumin | 32 mg, |
| Disodiumhydrogenphosphate hydrate | 118.4 mg, |
| Sodium dihydrogen phosphate | 11.2 mg, and |
| D-mannitol | 400 mg. |

58. The pharmaceutical composition according to any one of embodiments 50 to 57, further comprising 200 to 500 ml of an aqueous liquid.
59. The pharmaceutical composition according to embodiment 58, wherein said aqueous liquid is physiological saline solution.
60. The pharmaceutical composition according to any one of embodiments 50 to 59, consisting of

| | |
|---|---|
| Human M-CSF | 8 million units, |
| Human serum albumin | 32 mg, |
| Disodiumhydrogenphosphate hydrate | 118.4 mg, |
| Sodium dihydrogen phosphate | 11.2 mg, |
| D-mannitol | 400 mg, and |
| Physiological saline solution | 200 to 500 ml. |

61. The pharmaceutical composition according to any one of embodiments 58 to 60, wherein said pharmaceutical composition has a pH in the range of 6.8 to 7.8.
62. The pharmaceutical composition according to any one of embodiments 58 to 61, wherein the pharmaceutical composition is administered by intravenous infusion.
63. The pharmaceutical composition according to any one of embodiments 60 to 62, wherein said pharmaceutical composition is administered by intravenous infusion over 30 minutes per 100 ml.
64. The pharmaceutical composition according to any one of embodiments 60 to 61, wherein said pharmaceutical composition is administered once daily.
65. The pharmaceutical composition according to any one of embodiments 60 to 64, wherein said pharmaceutical composition is administered once daily for a period of up to two weeks.
66. The pharmaceutical composition according to any one of embodiments 50 to 65, wherein said pharmaceutical composition is for prevention of activation of a latent virus according to any one of embodiments 2 to 11.
67. The pharmaceutical composition according to any one of embodiments 50 to 65, wherein said pharmaceutical composition is for prevention or treatment of an accidental viral infection according to any one of embodiments 12 to 18.
68. The pharmaceutical composition according to any one of embodiments 50 to 65, wherein said pharmaceutical composition is for prevention or treatment of a viral infection, wherein said viral infection is derived from the transfer of donor material to said subject, according to any one of embodiments 19 to 25.
69. The pharmaceutical composition according to any one of embodiments 50 to 68, wherein the pharmaceutical composition is to be administered at least once during the state of immunosuppression.
70. The pharmaceutical composition according to embodiment 69, wherein the pharmaceutical composition is to be administered during the state of immunosuppression.
71. The pharmaceutical composition for use according to any one of embodiments 50 to 70, wherein said pharmaceutical composition further comprises at least one additional agent, selected from an antiviral compound and an immunosuppressive agent.
72. The pharmaceutical composition for use according to embodiment 71, wherein said antiviral compound is selected from letermovir, acyclovir, cidofovir, ganciclovir, idoxuridine, penciclovir, valganciclovir, valacyclovir, vidarabine, amantadine, rimantadine, zanamivir, fomivirsen, imiquimod, ribavirin, nucleoside reverse transcriptase inhibitors (e. g., zidovudine, didanosine, stavudine, zalcitabine, lamividudine ), non-nucleoside reverse transcriptase inhibitors (e.g., nevirapine, efavirenz, delvirudine ), and protease inhibitors (e.g., saquinivir, indinavir, ritonavir, nelfinavir, amprenavir, and lopinavir).
73. The pharmaceutical composition for use according to embodiment 72, wherein said immunosuppressive agent is selected from a corticosteroid, cyclosporin A, a cyclosporin-like immunosuppressive agent, cyclophosphamide, antithymocyte globulin, azathioprine, rapamycin, FK-506, a macrolide-like immunosuppressive agent other than FK-506 and rapamycin; and Mycophenolate mofetil.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Handbook of Experimental Immunology" (Weir, 1997); "Short Protocols in Molecular Biology" (Ausubel, 2002); "Polymerase Chain Reaction: Principles, Applications and Troubleshooting", (Babar, 2011); "Current Protocols in Immunology" (Coligan, 2002). These techniques may be considered in making and practicing the invention.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practicing the present invention and are not intended to limit the scope of the invention.

### Examples of the invention

### Materials and Methods

**Mice and *in vivo* treatments.** CD45.1 and C57BL/6 mice were obtained from Charles River. 8-14 weeks old sex-matched CD45.2 recipients were reconstituted as described (Mossadegh-Keller et al., 2013; Sarrazin et al., 2009) with bone marrow derived KSL (c-Kit (CD117)+, Sca1+, Lin-) HCT isolated from 6-8 weeks old CD45.1 donors. For in vivo injections, the indicated concentrations of M-CSF and/or sorted cells were injected in 100-200µl of PBS into the retro-orbital sinus. For HCT transplantation, 3,000 KLS HCT were sorted from CD45.1 mice and mixed with 100,000 to 200,000 cKit-, Ter119+ carrier CD45.2 cells prior to injection into lethally irradiated (160 kV, 25 mA, 6.9 Gy) CD45.2 recipient mice. After irradiation, all mice were given antibiotics (Bactrim) in the drinking water to reduce the chance of opportunistic bacterial infections. Monocytes and Macrophages, NK cells were depleted in vivo by intraperitoneal delivery of 100 µg anti-CD115 mAB or 100 µg anti NK1.1 mAb. Anti-CD115 antibody was injected d-2, d-1 for myeloid cell depletion; Anti-NK1.1 antibody was injected d-1 before MCMV infection, followed by injections on d1, 3 and 5 for NK cell depletion. Control mice were treated with Rat IgG. Cell depletion efficiency was assessed by flow cytometry. GMPs from WT or *Il15ra*-KO or *Ifnar1*-KO mice were FACS sorted and injected on day 10 after HCT transplantation. All mouse experiments were performed under specific pathogen-free conditions in accordance with institutional guidelines under permit number: APAFIS#17258-2018102318448168-v5.

**Isolation of hematopoietic stem and progenitor cells (HCT), cKit⁻ Ter119⁺ cells and GMPs.** Total bone marrow cells were depleted of mature cells by staining with biotinylated rat anti-mouse lineage antibody cocktail, followed by streptavidin immuno-magnetic micro beads (Miltenyi Biotec). Lineage negative cells were stained with HCT markers: anti-CD117-BV605 (clone 2B8, BD Biosciences), anti-Sca-1-PerCP-Cy5.5 (clone D7, Bio legend), Streptavidin-APC (eBioscience) or with GMP markers on CD117+ Sca-1- Cells: anti- CD34-Alexa 700 (BD), anti-CD16/32-PE (BD) and LIVE/DEAD Fixable Violet Dead cell dye (Invitrogen) as viability marker. HCT and GMPs were sorted using FACSAriaIII equipment. For isolating cKit⁻ Ter119⁺ carrier cells from whole bone marrow, first cKit- cells were selected by depleting cKit⁺ cells by staining with biotinylated anti-mouse CD117 (clone 2B8, BioLegend), and then stained with biotinylated antimouse Ter119 (clone TER-119, BD biosciences), followed by streptavidin immunomagnetic micro beads (Miltenyi Biotec).

**M-CSF treatment.** Each mouse received indicated number of i.v. injections of 10 µg of each cytokine: 1h before HCT transplantation, 5h and 18h post-transplantation. Mouse recombinant M-CSF expressed in baculovirus (Wang et al.) or human-M-CSF from Chiron/Novartis was used for the study.

**Infection with *mouse cytomegalovirus (MCMV) and scoring of severe viremia.*** Two-weeks post-HCT transplantation, mice were challenged by intra-peritoneal inoculation of 5,000 PFU of the MCMV K181 v70 strain (Cocita et al.) in 200 µL of sterile PBS. Infected mice were monitored daily for signs of morbidity (weight loss, piloerection, hunched posture and lethargy). Imminent death was defined as loss of 20% initial body weight or development of severe lethargy (unresponsiveness to touch), as established in a preliminary experiment using death as the endpoint. In all other experiments, the animals were then sacrificed if moribund from severe viremia, in accordance with animal license guidelines (APAFIS#17258-2018102318448168-v5).

**Spleen preparation and flow cytometry analysis.** Spleen leukocytes suspensions were prepared using DNAse I and collagenase D (Baranek et al.) and stained with respective antibodies for different populations. For FACS sorting and analysis, described staining protocols (Kandalla et al.; Mossadegh-Keller et al.) and published stem and progenitor cell definitions (Bryder et al.) were used. The following antibodies were used : B220-APC (Mossadegh-Keller et al.), CD11b-PerCP (M1/70), CD11c-A700 (HL3), CD19-APC (6D5), CD19-FITC (1D3), CD19-PE (1D3), CD3ε-PE (145-2c11), CD4-PE (RM4-5), CD4-PerCP (RM4-5), CD8α-PB (53-6.7), CD8α-PE (53-6.7), CD8α-PerCP (53-6.7), CD8β (H35-17.2), DX5-APC (DX5), Granzyme B-APC (GB11), IFN-β-FITC (RMMB1), IFN-γ-A700 (XMG1.2), IL-12-APC (C15-6), Ki67-PB (B56), NK-1.1-BV510 (PK136), NK-1.1-APC (PK136), NKp46-PE (29A1.4), Streptavidine-PECy7 (BD Biosciences), Purified NK1.1 (PK136), purified polyclonal rat IgG (Jackson ImmunoResearch), CD117-BV605 (2B8), Sca-1-PerCP-Cy5.5 (D7), CD34-A700 (RAM34), CD16/32-PE (2.4G2), CD11b-PECF594 (M1/70), Ly6G-FITC (1A8), Ly6C (HK1.4), purified CD115 (AFS98), CD45.2 (104), CD45.1 (A20), anti-Terl 19 (TER-119) and anti-CD71 (R17217). LIVE/DEAD Fixable Violet Dead cell dye (Invitrogen) was used as viability marker. Antibodies were purchased from BD biosciences, eBioscience and Biolegend. Extracellular staining was performed at 4°C in PBS, EDTA 2mM, SVF2%, and intracellular staining with a Cytofix/Cytoperm^{™} Fixation/Permeabilization Solution Kit from BD biosciences. For FACS sorting and analysis, FACSCanto, LSRII and FACSAriaIII equipment and DIVA software (Becton Dickinson) were used, analyzing only populations with at least 200 events.

**Titration of viral loads and histopathology.** Viral loads were measured as absolute levels of expression of the *Ie1* gene (Baranek et al.), by RT-qPCR on mRNA extracted from frozen tissues or sorted cells 36-40h (1.5 days) or 72h (3 days) after MCMV infection as indicated, using previously reported protocols (Cocita et al.). Fragments of the liver were fixed in 4% paraformaldehyde and embedded in paraffin. Hematoxylin and eosin (H&E)-stained sections were coded and analyzed and scored by a pathologist blinded to sample identity to determine the presence and severity of MCMV hepatitis.

**Immunofluorescence and confocal imaging.** Freshly extracted livers were harvested and embedded in optimal cutting temperature (OCT) freezing media (Sakura Finetek). 8 µm frozen sections were cut with Leica CM3050 S cryostat and fixed in cold acetone for 10 min before saturation with PBS 2% BSA during 30 min. The following antibodies were used: NK-1.1-FITC (PK136,100) and IE-1 (168YPHFMPTNL176, 1:100). Sections were mounted with Prolong Gold anti-fade reagent (Invitrogen) and confocal microscopy acquisition was performed on a LSM780 Carl Zeiss microscope.

**mRNA preparation, Gene expression analysis by microfluidic real-time PCR.** Total mRNA was extracted from FACS-sorted bulk populations of 50,000 cells. RNA extraction and cDNA synthesis were performed with a µMACS one step T7 template kit (Miltenyi). Specific target gene expression was detected either according to Fluidigm protocols as previously described (Soucie et al.) or by SybrGreen method (Mossadegh-Keller et al.). In brief, after 10 cycles of cDNA preamplification, microfluidic real-time PCR was performed using Dynamic Array integrated fluidic circuits (Biomark; Fluidigm) with TaqMan gene expression assays (Lenac Rovis et al.) or primer assay in 96.96 Dynamic Arrays on a BioMark System (Fluidigm). Ct values were calculated from the system's software (BioMark Real-time PCR Analysis; Fluidigm). Relative gene expression was calculated with the ΔΔ Ct method using Hprt as housekeeping gene for normalization.

Primers used for microfluidic real-time PCR were: mouse *Hprt*: 5'-CTGATAAAATCTACAGTCATAGGAATGGA-3' and 5'-GGCCCTCTGTGTGCTCAAG-3 '; mouse *Ifng*: 5'-CCACGGCACAGTCATTGAAA-3' and 5'-GCCAGTTCCTCCAGATATCCAA-3'; mouse *Prf1*: 5'-GATGTGAACCCTAGGCCAGA-3' and 5'-AAAGAGGTGGCCATTTTGTG-3'; mouse *Cebpa*: 5'-CAAGAACAGCAACGAGTACCG-3' and 5'-GTCACTGGTCAACTCCAGCAC-3'; mouse *Mitf*: 5'-ACTTTCCCTTATCCCATCCACC-3' and 5'-TGAGATCCAGAGTTGTCGTACA-3'; mouse *Nkg2d*: 5'-ACGTTTCAGCCAGTATTGTGC-3' and 5'-GGAAGCTTGGCTCTGGTTC-3'; mouse *Irf3*: 5'-GAGAGCCGAACGAGGTTCAG-3' and 5'-CTTCCAGGTTGACACGTCCG-3'; mouse *Irf7*: 5'-CTTCCCTATTTTCCGTGGCTG-3' and 5'-TCCAGTTGATCCGCATAAGGT-3'; mouse *Ifnb1*: 5'-CAGCTCCAAGAAAGGACGAAC-3' and 5'-GGCAGTGTAACTCTTCTGCAT-3'; mouse *Il15rb:* 5'-TGGAGCCTGTCCCTCTACG-3' and 5'-TCCACATGCAAGAGACATTGG-3'; mouse *Jak3*: 5'-CCATCACGTTAGACTTTGCCA-3' and 5'-GGCGGAGAATATAGGTGCCTG-3'; mouse *Stat5b*: 5'-CGATGCCCTTCACCAGATG-3' and 5'-AGCTGGGTGGCCTTAATGTTC-3'; And the following for Fluidigm: *Ikaros* (Assay number: Mm00496114_m1); *Id2* (Mm00711781_m1); *Runx3* (Mm00490666_m1); *Gata3* (Mm00484683_m1); *Tbet* (Mm00450960_m1); *Eomes* (Mm01351985_m1); *E2f1* (Mm00432939_m1); *E2f2* (Mm00809102_s1); *E2f4* (Mm00514160_m1); *E2f6* (Mm00519030_m1).

### Example 1: M-CSF protects HCT graft recipients from CMV viremia and mortality

CMV infection is a serious problem in immunosuppressed patients and one of the principal causes of morbidity and mortality after hematopoietic cell transplantation (Arber et al.) (Ljungman et al.; Locatelli et al.). MCMV is a natural pathogen in mice that recapitulates the key immunological hallmarks of human CMV infection (Krmpotic et al.). In order to study the antiviral effects of M-CSF on MCMV under conditions of leukopenia, a mouse HCT transplantation protocol as a model (Kandalla et al.) was used. Mice were infected with a lethal dose of MCMV in the leukopenic period, two weeks after HCT transplantation and M-CSF or placebo treatment.

To test the effect of M-CSF on survival after viral infection, irradiated and HCT-transplanted mice received either four injections of 10µg of baculovirus-produced mouse recombinant M-CSF (Dominguez-Andres et al.) or PBS, for several days during and after HCT transplantation (-1h, +1d, +3d, +5d). Alternatively, mice received three injections of 10µg of M-CSF or PBS on the day of HCT transplantation (-1h, +5h, +18h). As shown in the scheme ofFig.1A, mice were then infected two weeks later with 5,000 PFU of MCMV, a dose that had been established before to cause 80-90% lethality in untreated transplant recipients (Sup. Fig.1A). Survival rates significantly and strongly increased in treated mice, from 25% in control to 83.3% or 81.8%, respectively, in the mice that had received four treatments over several days (Sup. Fig.1B) or three treatments of M-CSF on the day of transplant (Fig.1B). Mice that were treated with bacteria-produced recombinant human M-CSF similarly showed strongly improved survival rates (Sup. Fig.1C). Based on these findings, three treatments on the day of transplant (-1h, +5h, +18h) were used throughout the rest of the study. However, even a single dose of cytokine treatment five hours after transplantation was sufficient to protect from MCMV infection-induced mortality with improved survival rates from 12.5% in control to 66.6% in treated conditions (Sup. Fig.1D). Furthermore, in the M-CSF-treated groups, even the mice eventually succumbing to infection did so later than control mice. Together these results showed a surprising potency of M-CSF treatment in protecting HCT-transplanted mice from MCMV-induced lethality.

Improved survival of M-CSF treated mice was associated with less severe tissue damage. One of the first organs to be severely affected by CMV infection is the liver (Hsu et al.). M-CSF-treated mice showed only a tendency of slightly decreased numbers of inflammatory foci (Fig. 1C) but a significant reduction of apoptotic or necrotic hepatocytes (Fig. 1D) in the liver four days after MCMV infection and strongly decreased necrotic areas eight days after infection (Fig. 1E). Consistent with this, M-CSF-treated mice also showed a significant decrease in viral load as shown by reduced number of infected hepatocytes on histological sections (Fig. 1F), by immune-fluorescence staining for viral protein IE1 (Fig. 1G) and by RT-qPCR measured viral RNA copy numbers (Fig. 1H). Collectively these results demonstrate that M-CSF treatment protected HCT-transplanted recipients against MCMV infection and ensuing tissue damage.

### Example 2: M-CSF treatment increases NK cell production, differentiation and activation

Since NK cells are early antiviral effector cells, including during HCT (Ullah et al.), it was investigated whether M-CSF treatment had an effect on NK cells. Indeed, a strong increase in the absolute numbers of NK cells in the spleen was observed 2 weeks after M-CSF treatment (Fig. 2A). This increase was evident both in uninfected mice and 1.5 days after infection, and observed beyond the increased NK cell numbers seen in response to infection itself (Fig. 2A). Separate analysis of CD45.2 recipient and CD45.1 graft donor cells revealed that M-CSF augmented both recipient- and donor-derived NK cell numbers but that the majority of the NK cell increase was due to donor-derived cells (Sup. Fig. 2A). The analysis was therefore focused on donor-derived cells. Since M-CSF is short-lived (Koths) but resulted in increased NK cell numbers two weeks after application and enabled an even stronger increase after MCMV infection, it was hypothesized that it might augment also NK cell progenitor numbers and their differentiation. NK cell differentiation stages can be identified by their differential expression of specific surface markers and transcription factors (Huntington et al.; Serafini et al.; Vosshenrich and Di Santo) (Fig. 2B). For example, NK cell progenitors express CD122, CD27 and NKG2D but not the mature markers NK1.1 and NKp46. It was observed that M-CSF significantly increased the number of donor-derived CD122⁺ CD27⁺ NK cell progenitors both in uninfected and infected mice (Fig. 2C).

Then NK maturation and differentiation status were analyzed. Based on the expression of CD11b and CD27, different maturation and differentiation states of NK cells have been identified: CD11b- CD27+ immature NK cells, CD11b+ CD27+ mature M1 NK cells and CD11b+ CD27- mature M2 NK cells (Fig. 2B) (Chiossone et al.; Hayakawa and Smyth; Kim et al.). M-CSF treatment significantly increased donor-derived immature as well as mature M1 and M2 NK cells, with a more profound increase in infected mice (Fig. 2D). A similar, albeit much smaller, increase of progenitor and mature cells was also observed for recipient-derived NK cells (Sup. Fig. 2C), indicating that radioresistant resident host NK cells remained responsive to M-CSF-induced signals.

The observations from the surface marker analysis were further confirmed by gene expression analysis of stage-specific transcription factors (Fig.2A). NK1.1⁺ NK cells that were FACS sorted from the spleen 2 weeks after M-CSF stimulation showed increased expression of *Ikaros, Id2, Runx3, Gata3* and *Tbet,* transcription factors that are all expressed in immature NK cells (Fig.2E). An increased expression of the mature NK cell gene marker *Eomes* was also detected (Fig. 2E). Similar observations were made for host-derived NK cells (Sup. Fig. 2B). Whereas some transcription factor genes like *Ikaros* and *Gata3* were more strongly induced by M-CSF in uninfected mice, *Tbet* and *Eomes* were more strongly induced after infection (Fig. 2E). Importantly, however, MCMV infection alone was not sufficient for the observed inductions.

Together this indicated that M-CSF lead to an increased number of NK cell progenitors in the transplanted cells and their enhanced differentiation along the NK cell lineage as well as to increased maturation of resident NK cells.

### Example 3: NK cell activity is required for the antiviral effect of M-CSF

The major anti-viral activity of NK cells is mediated by the production of inflammatory cytokines such as IFN-γ and by direct killing of infected cells through perforin-dependent delivery to their cytosol of apoptosis-inducing enzymes such as granzyme B (Bukowski et al.). Interestingly, it was observed that M-CSF treatment increased the number of IFN-γ (Fig. 3A) and granzyme B producing NK cells (Fig. 3B) in the spleen of MCMV infected mice. These results were confirmed by gene expression analysis, showing higher mRNA levels principally in donor-derived NK cells of M-CSF treated MCMV-infected mice for *Ifng, Gzmb* and *Prf1* (Fig. 3C, Sup. Fig. 2D). They also showed increased levels of the maturation and activation marker genes *Cebpa, Mitf* and *Xcl1* (Fig. 3C, Sup. Fig. 2D). Consistent with this increased antiviral activity, NK cells accumulated at infectious foci in the livers of M-CSF-treated mice was associated with reduced numbers of MCMV-infected cells as early as 4 days after MCMV infection (Fig. 3D). In order to determine whether the antiviral NK cell effect was required for the protective effect of M-CSF against CMV infection, NK cells were depleted using anti-NK1.1 antibody in M-CSF-treated and MCMV-infected HCT recipients (Fig. 3E). It was observed that NK cell-depletion nearly abolished the increased survival of M-CSF-treated mice. This demonstrated that the major part of the protective effect of M-CSF against lethal MCMV infection was dependent on NK cells.

### Example 4: M-CSF-induced myelopoiesis is required for its antiviral effect

Since M-CSF has not been reported to act directly on the NK cell lineage, it was investigated whether M-CSF effects on the myeloid lineage could indirectly be important for NK cell-mediated anti-viral activity. It was reported before that M-CSF treatment of HCT-transplanted mice increased myeloid cell generation at time points relevant for defense against bacterial and fungal infections (Kandalla et al.). Here it was observed that M-CSF stimulation also resulted in increased donor-derived granulocyte-monocyte/macrophage progenitors (GMP) and granulocytes and mononuclear phagocytes (Fig. 4A, B), pDC and cDC (Sup. Fig. 3A) in the spleen two weeks after HCT transplantation and thus at the time of MCMV infection in the experimental protocol. In order to determine whether this M-CSF-induced increase in myeloid cells was relevant to the observed anti-viral effect of M-CSF, complementary loss and gain of function approaches were used. First a depletion approach with an anti-MCSFR (anti-CD 115) antibody was used, which selectively eliminates M-CSF-dependent myeloid cells and mononuclear phagocytes (Tagliani et al.). This antibody was injected 12 days after the HSC transplantation and M-CSF treatment protocol and thus well after the initially injected M-CSF has been cleared from the system. Therefore, this protocol scores the effect of much later developing M-CSF-dependent myeloid progenitors and monocytic cells. The effectiveness of this approach was confirmed and strongly reduced numbers of GMP, monocytes, cDCs and pDCs was observed 24 hours after the second anti-CD 115 antibody second treatment (Fig. 4D). It was observed that this myeloid cell-depletion completely abolished the protective effect of M-CSF treatment in MCMV-infected HCT transplant recipients (Fig. 4C), indicating that myeloid cells were required for M-CSF anti-viral activity. In the reciprocal gain-of-function experiment, GMP was transplanted into HCT-engrafted mice that were not treated with M-CSF, 10 days after hematopoietic cell transplantation, a time point when M-CSF treatment resulted in increased GMP levels (Fig.4A). It was observed that GMP transplantation resulted in a similar strong increase in survival of MCMV-infected HCT engrafted mice as M-CSF treatment (Fig. 4E). Together, these two approaches demonstrated that the anti-viral activity of M-CSF is dependent on an M-CSF-induced increase in myelopoiesis.

### Example 5: Myeloid IL-15 trans-presentation is required for protective antiviral activity of M-CSF

Since it was observed that both myelopoiesis and NK cell differentiation were required for the anti-viral effect of M-CSF, it was hypothesized that M-CSF-induced myeloid cell generation could indirectly affect NK-cell differentiation and anti-viral activity. Indeed, anti-CD 115-mediated depletion of myeloid cells also resulted in a strong reduction of immature and mature NK cell populations (Fig. 5A), suggesting that signals from myeloid cells affected NK cell differentiation and maturation. To identify such signals, it was first focused on IL-15, a key cytokine for NK cell differentiation and acquisition of cytotoxic effector functions (Nguyen et al.) (Boudreau et al.; Budagian et al.; Huntington et al.) that can be produced and trans-presented by IL-15Ra on myeloid cells (Castillo et al.; Huntington et al.; Lucas et al.; Patidar et al.) including during MCMV infection (Ghilas et al.) (Baranek et al.; Fehniger et al.).

Indeed, a much stronger increase of IL-15 was found 1.5 days after MCMV infection in the spleens of mice, if they had been treated with M-CSF during transplantation (Fig. 5B). To determine which cell type was responsible for the increased production of IL-15 in M-CSF treated mice, gene expression in spleen cDC and monocytes was analyzed (Fig. 5C), both of which have been reported to be capable of stimulating NK cells by IL-15 (Baranek et al.; Dominguez-Andres et al.; Lucas et al.). IL-15 signaling requires trans-presentation by the surface molecule IL-15RA/CD215. Therefore, the expressions levels of IL-15Rα and genes involved in induction of IL-15 and/or IL-15Rα expression (schematic representation in Fig. 7) were specifically analyzed. Upregulation of *Il15ra* and of the *Ifnb1* gene was observed that encodes one of the type I interferons (IFN-I), which are known to induce IL-15 and IL-15Rα expression (Baranek et al.; Ohteki et al.). Genes encoding transcription factors promoting the production of IFN-I (*Irf1, Irf3* and *Irf7*) or the response to these cytokines (*Irf1*) were also induced. The expression of these factors was more strongly increased in CD11b⁺ Ly6C⁺ monocytes than in cDCs, indicating that they are the primary cell type responsible for induction of IL-15 signaling in M-CSF-treated infected animals. These results were further confirmed on the protein level by FACS analysis showing that monocytes of M-CSF treated animals show a strong induction of IL-15Rα during infection, with a stronger effect observed in Ly6C^{hi} monocytes than Ly6C^{lo} monocytes (Fig. 5D). The strong M-CSF dependent induction of both IL-15 and IL-15Rα was most pronounced under infection conditions.

Next, the effect of increased IL-15 signals from monocytes on the expression of IL-15 response genes in NK target cells was analyzed. Consistent with the known presentation of IL-15 by IL-15Rα on stimulating cells and signal transmission through IL-15Rβ on target cells (Baranek et al.; Huntington et al.; Patidar et al.), M-CSF treatment increased *Il15rb* expression on NK cells (Fig. 5E). Furthermore, M-CSF treatment also increased expression of the *Stat5b* and *Jak3* genes encoding signaling molecules that are downstream of IL-15Rβ in NK cells. To check whether this myeloid cell communication to NK cells through IL-15 was important for antiviral activity, the ability of grafted *Il15ra*-KO GMP that are incapable of trans-presenting IL-15 and WT GMP were compared to protect HCT transplant recipients from lethal MCMV infection. As before, 80% survival in WT GMP-transplanted mice was observed after MCMV infection but no survival of *Il15ra*-KO GMP-transplanted mice, demonstrating that IL-15 signaling from myeloid cells was required for the antiviral effect of NK cells (Fig. 5F). Furthermore, M-CSF treatment resulted in no survival advantage in *Il15ra*-KO HCT-transplanted mice and was comparable to untreated WT HCT transplanted mice (Fig. 5G), indicating that IL-15 signaling was acting downstream of M-CSF. Together the data demonstrated that myeloid-derived IL-15-signaling was required for the antiviral effect of M-CSF-induced myelopoiesis.

### Example 6: M-CSF-induced IFN-I production stimulates IL-15-dependent antiviral effect

IFN-I release plays an important role in the early antiviral defense that precedes NK cell activation (Alexandre et al.; Baranek et al.; Cocita et al.; Degli-Esposti and Smyth; Liu et al.). Since in the sensitive post-transplantation period this rapid response mechanisms might be important to avoid fatal viremia, it was investigated whether M-CSF treatment also affected IFN-I production. As shown in Fig. 6A, mice that had been treated with M-CSF during transplantation indeed showed a strong increase of *Ifnb1* mRNA levels in the spleen at 1.5 days after CMV infection that persisted until 3 days at a weaker level. Plasmacytoid dendritic cells (pDC) are the principal source of IFN-I during MCMV infection (Dalod et al.; Zucchini et al.). Consistent with this, it was observed that pDC numbers in the spleen were strongly increased in M-CSF-treated mice 2 weeks after HCT transplantation (Sup. Fig. 3A). This advantage was also maintained after MCMV infection, with M-CSF-treated mice showing a larger increase in pDC numbers than control mice (Fig. 6B). M-CSF treatment also increased the number of IFN-I-positive pDC in MCMV infected mice (Fig. 6C). Monocytes but not classical DC also showed a strong increase in *Ifnb1* gene expression (Fig. 5C). Together this supported the notion that M-CSF treatment increased IFN-I production during MCMV infection of HCT-transplanted recipients by promoting a faster reconstitution of cellular sources of these cytokines, in particular monocytes and pDC. pDC can differentiate from myeloid progenitors and M-CSF can stimulate pDC development (Fancke et al.). Consistent with this, treatment of transplanted mice with anti-CD115 antibody also eliminated pDC (Fig. 4D). The observations that M-CSF can stimulate GMP production from HCT (Kandalla et al.; Mossadegh-Keller et al.) and that GMP transplantation can protect from viral infection (Fig. 4E, 5F), might therefore indicate that IFN-I production from pDC might also contribute to the anti-viral effect of M-CSF in HCT-transplanted mice.

Beyond its direct antiviral effects on infected cells, IFN-I can also indirectly affect the antiviral immune response by activating NK cells or by stimulating myeloid cells to produce IL-15 (Baranek et al.; Degli-Esposti and Smyth; Nguyen et al.). In order to dissect these possibilities and to investigate the relative importance of the IFN-I effect on myeloid cells during the antiviral immune response, it was investigated whether the protective effect of GMP transplantation against lethal viremia (Fig. 4E, 5F) was dependent on IFN-I activation. Therefore, *Ifnar1-*KO or WT GMP was injected at day 10 after HCT transplantation without M-CSF treatment. As observed before (Fig. 4E, 5F) GMP transplantation could mimic the protective effect of M-CSF induced myelopoiesis but *Ifnar1* deficiency completely abolished this effect (Fig. 6D). This indicated that IFN-I stimulation of myeloid cells was required for their antiviral effect. Since IFN-I can stimulate IL-15 production in myeloid cells (Baranek et al.) and it was observed that IL-15 production by myeloid cells was required for M-CSF induced anti-viral activity (Fig. 5F), it was investigated whether IL-15 could rescue *Ifnar1* deficiency. As shown in Fig. 6E, IL-15 treatment prior to MCMV infection could indeed partially compensate for the inability of *Ifnar1*-KO GMP to protect HCT transplanted mice from lethal viremia. Together this indicated the importance of IFN-I induction of IL-15 in myeloid cells for the antiviral response and indicated that its anti-viral effect was mainly due to its effect on the identified myeloid and NK cell differentiation cascade rather than direct effects on infected cells (Fig. 7).

### Summary

Within the present invention, previously unknown protective effects of M-CSF-induced myelopoiesis against viral infection during immunosuppression were identified. A coordinated differentiation cascade was identified between myeloid cells and NK cells that play a major role in reconstituting immune protection against viral infection and assigns a critical role to M-CSF-induced myelopoiesis to participate in antiviral activities.

### Discussion of the results

Immune compromised individuals show a strongly increased sensitivity not only to viremia, but also to infection-induced morbidity and mortality. Here it was used a mouse model of leukopenia after HSC transplantation to investigate the effects of M-CSF-induced myelopoiesis on MCMV infection. HSC transplantation is an important major therapeutic strategy against several malignant and non-malignant hematological pathologies. Since the procedure involves a conditioning that ablates the hematopoietic system, the patients encounter a serious immunodeficiency after HSC transplantation that leaves them highly vulnerable to opportunistic bacterial, fungal and viral infection, before the new donor bone marrow starts to produce sufficient new immune effector cells. Although improvements have been made in the management of infections, they are still the cause for 30-50% of deaths after transplantation. The development of new preventive strategies against infections based on a more rapid reconstitution of immune-competence would avoid these fatalities and could extend the utility of HSC transplantation to other pathologies. Cytomegalovirus (CMV) infection is a serious clinical complication of HSC transplantation and a major cause of post-transplantation morbidity and mortality (Boeckh and Ljungman; Ljungman et al.; Zaia). Unfortunately, available anti-viral therapies are associated with numerous drawbacks, such as poor bioavailability, development of anti-viral drug resistance (Ahmed; El Chaer et al.) and significant bone marrow toxicity impeding donor cell engraftment and hematopoietic reconstitution. For example, ganciclovir severely compromises myelopoiesis, and thus further aggravates susceptibility to secondary infections (Boeckh et al.; Goodrich et al.; Salzberger et al.). Universal prophylaxis with antiviral therefore remains problematic due to these risks of toxic side effects. Furthermore, CMV strains have developed that are resistant to the most common antiviral drugs such as ganciclovir and valganciclovir (El Chaer et al.).

Adoptive transfer protocols of lymphoid progenitors also has been proposed to improve recovery, and resulted in protection against lethal infections of MCMV in a mouse model of HCT (Arber et al.). Cell therapy approaches, however, require complex logistics and rigorous quality control, and their effectiveness and tolerance might be patient specific. Given the lack of satisfying treatment options, the development of new anti-viral therapies addresses a clear and urgent clinical need, for both acute and prophylactic treatments that might be addressed by M-CSF therapy.

Several properties of M-CSF make it an ideal candidate for accelerating immune competence recovery in HSC transplant recipients and present key advantages over other myeloid cytokines used in clinical practice. It was shown before that M-CSF directly engages HSC and early progenitors and thus intervenes at the earliest point of the differentiation hierarchy to initiate the production of innate immune cells (Kandalla et al.; Mossadegh-Keller et al.; Sarrazin et al.). This is important, because HCT are present from the earliest time points after transplantation in immuneosuppressed patients, long before more mature progenitors develop. M-CSF stimulation could therefore shorten the time of immune system reconstitution to reduce the risk of infections. Other cytokines, in particular G-CSF (marketed in the clinic as Neupogen, Granocyte), are also used to stimulate immune function. However, in contrast to M-CSF, G-CSF can only act on already existing mature or late myeloid progenitor cells to activate their immune function. Since these cells will only be produced with a significant lag phase, G-CSF will be ineffective early after transplantation. M-CSF treatment is based on a conceptually very different approach. By acting on the highest point of the hematopoietic differentiation hierarchy, it can stimulate myelopoiesis at a very early time point after myeloablation and can thus dramatically shorten the period when leukopenic patients are the most vulnerable to fatal infections. Consistent with this, it was shown previously that M-CSF but not G-CSF can stimulate the increased production of myeloid cells in hematopoietic stem and early progenitor cells and protect from bacterial and fungal infections (Kandalla et al.). Importantly, M-CSF-induced myelopoiesis also does not compromise stem cells numbers or activity (Sarrazin et al.), and does not come at the expense of the generation of other blood cell lineages like platelets that are important for restoring blood clotting activity (Kandalla et al.). Here it was found an additional advantage of M-CSF treatment by promoting rapid reconstitution of antiviral activity and protection from viral infection through a multistep myeloid and NK cell differentiation cascade. A significant advantage of the early action of M-CSF on hematopoietic stem and progenitor cells appears to be the simulation of a wide panel of innate immune cells that are all important to fight pathogens. Whereas G-CSF only stimulates granulocytes and their direct progenitors, M-CSF stimulates the production of i) granulocytes, mediating cytotoxic bacterial killing, ii) monocytes and macrophages, capable of pathogen control by phagocytosis and reactive oxygen production, and iii) dendritic cells with the strongest antigen presentation activity that alerts the adaptive immune system. The invention now shows that M-CSF also induced I-IFN producing pDC and indirectly stimulated NK cell differentiation and activation through induction of IL-15-producing monocytic cells, which together mediated strong antiviral activity.

Whereas the protective functions of NK cells and DCs during CMV infection are well described (Alexandre et al.; Brinkmann et al.), the role of macrophages and monocytes appears more ambivalent. On the one hand, they can be target cells for MCMV infection (Daley-Bauer et al.; Hanson et al.; Hokeness et al.) and serve as vehicles of CMV dissemination (Daley-Bauer et al.; Smith et al.). On the other hand, the observation that macrophage depletion with clodronate-loaded liposomes increased MCMV burden (Hanson et al.), also suggested a protective role of these cells during infection. Such dual role might be dependent on the context of infection or on the specific subpopulation. Whereas CX3CR1hi patrolling monocytes are involved in dissemination (Daley-Bauer et al.), Ly6C+ /CCR2+ inflammatory monocytes can activate antiviral responses in early CMV infection via direct or indirect mechanisms (Gawish et al.; Hokeness et al.; Lenac Rovis et al.; Salazar-Mather et al.; Soudja et al.). Ly6C+ CCR2+ inflammatory monocytes could initiate differentiation of memory CD8+ T and NK cells into antimicrobial effector cells (Soudja et al.) or showed direct iNOS mediated antiviral effects (Lenac Rovis et al.). IFN-I signaling is also important for recruitment of CCR2+ inflammatory monocytes via its ligand, the chemokine MCP-1/CCL2 (Salazar-Mather et al.). Mice deficient for MCP-1 or CCR2 thus showed a reduced accumulation of monocyte-derived macrophages and NK cells in liver, increased viral titers, widespread virus-induced liver pathology and reduced survival (Crane et al.; Hokeness et al.).

It has been shown previously that CD11chigh DC-derived IL-15 promoted NK cell priming (Lucas et al.) and that inflammatory monocyte-derived IL-15 could stimulate NK cells differentiation (Soudja et al.). In the leukopenic settings investigated here, Ly6Chigh monocytes appeared to be more important than DC for IL-15 presentation, since they expressed higher levels of IL-15Rα that is required for IL-15 cross-presentation to NK cells (Lucas et al.). Consistent with the cooperative role of IL-15 and IFN-I in NK cell activation (Lucas et al.; Nguyen et al.), it was observed that both *Il15ra-* and *Ifnar1*-deficiency in GMP-derived myeloid cells abolished their protective effect against MCMV, whereas ectopic IL-15 could rescue Ifnar1-deficiency. This suggested that IL-15 induction in monocytes required IFN-I that in turn was mainly produced by M-CSF-induced pDC. Together, the experiments revealed the surprising capacity of M-CSF to initiate a fully synchronized differentiation cascade and cytokine mediated cross talk between different myeloid and NK cell lineages to provide effective anti-viral protection in leukopenic immunosuppressed individuals (Fig. 7).

Given its multiple advantages, M-CSF therapy could not only be beneficial in hematopoietic cell transplantation protocols but also more generally in other settings where severe myeloablation occurs, for example after chemotherapy. Beyond this, the myeloid cell-mediated antiviral activity described here could also be more generally helpful to understand and fight other viral infections, such as the present aggressive pandemic of Coronavirus disease 2019 (COVID-19), a pulmonary inflammatory disease caused by severe acute respiratory syndrome coronavirus 2 (SARS-CoV-2). An effective anti-viral response involves effective innate and adaptive immune responses while at the same time avoiding cytokine storms. Individual M-CSF or IL-15 or a combinatorial therapy could be effective in inducing innate immunity in COVID-19 patients while mitigating cytokine storms response to SARS-CoV-2 infection that occur via the induction of several immune cells. IL-15 overexpression might promote innate immune responses via the induction of NK, CD8+ T and T regulatory cells that neutralize the induced Th2 cytokine storms, resulting in decreased levels of IL-4, IL-5 and IL-13 (Kandikattu et al.). These events might mitigate SARS-CoV-2 induced inflammation and fibrosis via IFN-γ and IL-10, while inhibiting viral replication and reducing viral load.

### References

Ahmed, A. 2011. Antiviral treatment of cytomegalovirus infection. Infect Disord Drug Targets 11:475-503.

Alexandre, Y.O., C.D. Cocita, S. Ghilas, and M. Dalod. 2014. Deciphering the role of DC subsets in MCMV infection to better understand immune protection against viral infections. Front Microbiol 5:378.

Arber, C., A. BitMansour, T.E. Sparer, J.P. Higgins, E.S. Mocarski, I.L. Weissman, J.A. Shizuru, and J.M. Brown. 2003. Common lymphoid progenitors rapidly engraft and protect against lethal murine cytomegalovirus infection after hematopoietic stem cell transplantation. Blood 102:421-428.

Baranek, T., T.P. Manh, Y. Alexandre, M.A. Maqbool, J.Z. Cabeza, E. Tomasello, K. Crozat, G. Bessou, N. Zucchini, S.H. Robbins, E. Vivier, U. Kalinke, P. Ferrier, and M. Dalod. 2012. Differential responses of immune cells to type I interferon contribute to host resistance to viral infection. Cell Host Microbe 12:571-584.

Boeckh, M., T.A. Gooley, D. Myerson, T. Cunningham, G. Schoch, and R.A. Bowden. 1996. Cytomegalovirus pp65 antigenemia-guided early treatment with ganciclovir versus ganciclovir at engraftment after allogeneic marrow transplantation: a randomized double-blind study. Blood 88:4063-4071.

Boeckh, M., and P. Ljungman. 2009. How we treat cytomegalovirus in hematopoietic cell transplant recipients. Blood 113:5711-5719.

Boettcher and Manz. 2017. Regulation of Inflammation- and Infection-driven Hematopoiesis. Trends Immunol. 38(5):345-357.

Boudreau, J.E., K.B. Stephenson, F. Wang, A.A. Ashkar, K.L. Mossman, L.L. Lenz, K.L. Rosenthal, J.L. Bramson, B.D. Lichty, and Y. Wan. 2011. IL-15 and type I interferon are required for activation of tumoricidal NK cells by virus-infected dendritic cells. Cancer Res 71:2497-2506.

Brinkmann, M.M., F. Dag, H. Hengel, M. Messerle, U. Kalinke, and L. Cicin-Sain. 2015. Cytomegalovirus immune evasion of myeloid lineage cells. Med Microbiol Immunol 204:367-382.

Bryder, D., D.J. Rossi, and I.L. Weissman. 2006. Hematopoietic stem cells: the paradigmatic tissue-specific stem cell. Am J Pathol 169:338-346.

Budagian, V., E. Bulanova, R. Paus, and S. Bulfone-Paus. 2006. IL-15/IL-15 receptor biology: a guided tour through an expanding universe. Cytokine Growth Factor Rev 17:259-280.

Bukowski, J.F., B.A. Woda, and R.M. Welsh. 1984. Pathogenesis of murine cytomegalovirus infection in natural killer cell-depleted mice. J Virol 52:119-128.

Castillo, E.F., S.W. Stonier, L. Frasca, and K.S. Schluns. 2009. Dendritic cells support the in vivo development and maintenance of NK cells via IL-15 trans-presentation. J Immunol 183:4948-4956.

D. Chatzidimitriou, D., E. Gavriilaki, I. Sakellari, and E. Diza. 2010. Hematopoietic cell transplantation and emerging viral infections. J. Med. Virol. 82:528-538

Cheers, C., and E.R. Stanley. 1988. Macrophage production during murine listeriosis: colony-stimulating factor 1 (CSF-1) and CSF-1-binding cells in genetically resistant and susceptible mice. Infect Immun 56:2972-2978.

Chiossone, L., J. Chaix, N. Fuseri, C. Roth, E. Vivier, and T. Walzer. 2009. Maturation of mouse NK cells is a 4-stage developmental program. Blood 113:5488-5496.

Cho, S.Y., D.G. Lee, and H.J. Kim. 2019. Cytomegalovirus Infections after Hematopoietic Stem Cell Transplantation: Current Status and Future Immunotherapy. Int J Mol Sci 20:

Cocita, C., R. Guiton, G. Bessou, L. Chasson, M. Boyron, K. Crozat, and M. Dalod. 2015. Natural Killer Cell Sensing of Infected Cells Compensates for MyD88 Deficiency but Not IFN-I Activity in Resistance to Mouse Cytomegalovirus. PLoS Pathog 11:e1004897.

Crane, M.J., K.L. Hokeness-Antonelli, and T.P. Salazar-Mather. 2009. Regulation of inflammatory monocyte/macrophage recruitment from the bone marrow during murine cytomegalovirus infection: role for type I interferons in localized induction of CCR2 ligands. J Immunol 183:2810-2817.

Cros J, Cagnard N, Woollard K, Patey N, Zhang SY, Senechal B, Puel A, Biswas SK, Moshous D, Picard C, Jais JP, D'Cruz D, Casanova JL, Trouillet C, Geissmann F. Immunity. 2010 Sep 24;33(3):375-86

Daley-Bauer, L.P., L.J. Roback, G.M. Wynn, and E.S. Mocarski. 2014. Cytomegalovirus hijacks CX3CR1(hi) patrolling monocytes as immune-privileged vehicles for dissemination in mice. Cell Host Microbe 15:351-362.

Daley-Bauer, L.P., G.M. Wynn, and E.S. Mocarski. 2012. Cytomegalovirus impairs antiviral CD8+ T cell immunity by recruiting inflammatory monocytes. Immunity 37:122-133.

Dalod, M., T. Hamilton, R. Salomon, T.P. Salazar-Mather, S.C. Henry, J.D. Hamilton, and C.A. Biron. 2003. Dendritic cell responses to early murine cytomegalovirus infection: subset functional specialization and differential regulation by interferon alpha/beta. J Exp Med 197:885-898.

Dalod, M., T.P. Salazar-Mather, L. Malmgaard, C. Lewis, C. Asselin-Paturel, F. Briere, G. Trinchieri, and C.A. Biron. 2002. Interferon alpha/beta and interleukin 12 responses to viral infections: pathways regulating dendritic cell cytokine expression in vivo. J Exp Med 195:517-528.

Degli-Esposti, M.A., and M.J. Smyth. 2005. Close encounters of different kinds: dendritic cells and NK cells take centre stage. Nat Rev Immunol 5:112-124.

Dickel S, et al. Case Report: Interferon-γ Restores Monocytic Human Leukocyte Antigen Receptor (mHLA-DR) in Severe COVID-19 With Acquired Immunosuppression Syndrome. Front Immunol. 2021 Apr 7;12:645124.

Dominguez-Andres, J., L. Feo-Lucas, M. Minguito de la Escalera, L. Gonzalez, M. Lopez-Bravo, and C. Ardavin. 2017. Inflammatory Ly6C(high) Monocytes Protect against Candidiasis through IL-15-Driven NK Cell/Neutrophil Activation. Immunity 46:1059-1072 e1054.

El Chaer, F., D.P. Shah, and R.F. Chemaly. 2016. How I treat resistant cytomegalovirus infection in hematopoietic cell transplantation recipients. Blood 128:2624-2636. Fancke, B., M. Suter, H. Hochrein, and M. O'Keeffe. 2008. M-CSF: a novel plasmacytoid and conventional dendritic cell poietin. Blood 111:150-159.

Fehniger, T.A., S.F. Cai, X. Cao, A.J. Bredemeyer, R.M. Presti, A.R. French, and T.J. Ley. 2007. Acquisition of murine NK cell cytotoxicity requires the translation of a pre-existing pool of granzyme B and perforin mRNAs. Immunity 26:798-811.

Gawish, R., T. Bulat, M. Biaggio, C. Lassnig, Z. Bago-Horvath, S. Macho-Maschler, A. Poelzl, N. Simonovic, M. Prchal-Murphy, R. Rom, L. Amenitsch, L. Ferrarese, J. Kornhoff, T. Lederer, J. Svinka, R. Eferl, M. Bosmann, U. Kalinke, D. Stoiber, V. Sexl, A. Krmpotic, S. Jonjic, M. Muller, and B. Strobl. 2019. Myeloid Cells Restrict MCMV and Drive Stress-Induced Extramedullary Hematopoiesis through STAT1. Cell Rep 26:2394-2406 e2395.

Ghilas, S., M. Ambrosini, J.C. Cancel, C. Brousse, M. Masse, H. Lelouard, M. Dalod, and K. Crozat. 2021. Natural killer cells and dendritic epidermal gammadelta T cells orchestrate type 1 conventional DC spatiotemporal repositioning toward CD8(+) T cells. iScience 24:103059.

Guilliams M, Mildner A, Yona S. Immunity. 2018 Oct 16;49(4):595-613.

Goodrich, J.M., R.A. Bowden, L. Fisher, C. Keller, G. Schoch, and J.D. Meyers. 1993. Ganciclovir prophylaxis to prevent cytomegalovirus disease after allogeneic marrow transplant. Ann Intern Med 118:173-178.

Hanson, L.K., J.S. Slater, Z. Karabekian, H.W.t. Virgin, C.A. Biron, M.C. Ruzek, N. van Rooijen, R.P. Ciavarra, R.M. Stenberg, and A.E. Campbell. 1999. Replication of murine cytomegalovirus in differentiated macrophages as a determinant of viral pathogenesis. J Virol 73:5970-5980.

Hayakawa, Y., and M.J. Smyth. 2006. CD27 dissects mature NK cells into two subsets with distinct responsiveness and migratory capacity. J Immunol 176:1517-1524.

Heuser, M., A. Ganser, C. Bokemeyer, O. American Society of Clinical, N. National Comprehensive Cancer, R. European Organization for, and C. Treatment of. 2007. Use of colony-stimulating factors for chemotherapy-associated neutropenia: review of current guidelines. Semin Hematol 44:148-156.

Hokeness, K.L., W.A. Kuziel, C.A. Biron, and T.P. Salazar-Mather. 2005. Monocyte chemoattractant protein-1 and CCR2 interactions are required for IFN-alpha/beta-induced inflammatory responses and antiviral defense in liver. J Immunol 174:1549-1556.

Hsu, K.M., J.R. Pratt, W.J. Akers, S.I. Achilefu, and W.M. Yokoyama. 2009. Murine cytomegalovirus displays selective infection of cells within hours after systemic administration. J Gen Virol 90:33-43.

Huntington, N.D., N. Legrand, N.L. Alves, B. Jaron, K. Weijer, A. Plet, E. Corcuff, E. Mortier, Y. Jacques, H. Spits, and J.P. Di Santo. 2009. IL-15 trans-presentation promotes human NK cell development and differentiation in vivo. J Exp Med 206:25-34.

Huntington, N.D., S.L. Nutt, and S. Carotta. 2013. Regulation of murine natural killer cell commitment. Front Immunol 4:14.

Jain, N. A. et al, Cytotherapy. 2014 Jul;16(7):927-33.

Kandalla, P.K., S. Sarrazin, K. Molawi, C. Berruyer, D. Redelberger, A. Favel, C. Bordi, S. de Bentzmann, and M.H. Sieweke. 2016. M-CSF improves protection against bacterial and fungal infections after hematopoietic stem/progenitor cell transplantation. J Exp Med 213:2269-2279.

Kandikattu, H.K., S.U. Venkateshaiah, S. Kumar, and A. Mishra. 2020. IL-15 immunotherapy is a viable strategy for COVID-19. Cytokine Growth Factor Rev 54:24-31.

Kim, S., K. Iizuka, H.S. Kang, A. Dokun, A.R. French, S. Greco, and W.M. Yokoyama. 2002. In vivo developmental stages in murine natural killer cell maturation. Nat Immunol 3:523-528.

Koths, K. 1997. Structure-function studies on human macrophage colony-stimulating factor (M-CSF). Mol Reprod Dev 46:31-37; discussion 37-38.

Krmpotic, A., I. Bubic, B. Polic, P. Lucin, and S. Jonjic. 2003. Pathogenesis of murine cytomegalovirus infection. Microbes Infect 5:1263-1277.

Kropp, K.A., K.A. Robertson, G. Sing, S. Rodriguez-Martin, M. Blanc, P. Lacaze, M.F. Hassim, M.R. Khondoker, A. Busche, P. Dickinson, T. Forster, B. Strobl, M. Mueller, S. Jonjic, A. Angulo, and P. Ghazal. 2011. Reversible inhibition of murine cytomegalovirus replication by gamma interferon (IFN-gamma) in primary macrophages involves a primed type I IFN-signaling subnetwork for full establishment of an immediate-early antiviral state. J Virol 85:10286-10299.

Lenac Rovis, T., P. Kucan Brlic, N. Kaynan, V. Juranic Lisnic, I. Brizic, S. Jordan, A. Tomic, D. Kvestak, M. Babic, P. Tsukerman, M. Colonna, U. Koszinowski, M. Messerle, O. Mandelboim, A. Krmpotic, and S. Jonjic. 2016. Inflammatory monocytes and NK cells play a crucial role in DNAM-1-dependent control of cytomegalovirus infection. J Exp Med 213:1835-1850.

Liu, J., X. Guan, and X. Ma. 2005. Interferon regulatory factor 1 is an essential and direct transcriptional activator for interferon {gamma}-induced RANTES/CCl5 expression in macrophages. J Biol Chem 280:24347-24355.

Ljungman, P., M. Boeckh, H.H. Hirsch, F. Josephson, J. Lundgren, G. Nichols, A. Pikis, R.R. Razonable, V. Miller, P.D. Griffiths, and F. Disease Definitions Working Group of the Cytomegalovirus Drug Development. 2017. Definitions of Cytomegalovirus Infection and Disease in Transplant Patients for Use in Clinical Trials. Clin Infect Dis 64:87-91.

Ljungman, P., P. Griffiths, and C. Paya. 2002. Definitions of cytomegalovirus infection and disease in transplant recipients. Clin Infect Dis 34:1094-1097.

Locatelli, F., A. Bertaina, V. Bertaina, and P. Merli. 2016. Cytomegalovirus in hematopoietic stem cell transplant recipients - management of infection. Expert Rev Hematol 9:1093-1105.

Lucas, M., W. Schachterle, K. Oberle, P. Aichele, and A. Diefenbach. 2007. Dendritic cells prime natural killer cells by trans-presenting interleukin 15. Immunity 26:503-517.

Metcalf, D. 2008. Hematopoietic cytokines. Blood 111:485-491.

Mossadegh-Keller, N., S. Sarrazin, P.K. Kandalla, L. Espinosa, E.R. Stanley, S.L. Nutt, J. Moore, and M.H. Sieweke. 2013. M-CSF instructs myeloid lineage fate in single haematopoietic stem cells. Nature 497:239-243.

Motoyoshi, K. 1998. Biological activities and clinical application of M-CSF. Int J Hematol 67:109-122.

Mire-Sluis AR, Gaines-Das R, Thorpe R. Journal of Immunological Methods 179(1995) 141-151.

Needleman SB, Wunsch CD. 1970 J. Mol. Biol. 48:443-453

Nguyen, K.B., T.P. Salazar-Mather, M.Y. Dalod, J.B. Van Deusen, X.Q. Wei, F.Y. Liew, M.A. Caligiuri, J.E. Durbin, and C.A. Biron. 2002. Coordinated and distinct roles for IFN-alpha beta, IL-12, and IL-15 regulation of NK cell responses to viral infection. J Immunol 169:4279-4287.

Ogonek J, Kralj Juric M, Ghimire S, Varanasi PR, Holler E, Greinix H and Weissinger E (2016) Immune Reconstitution after Allogeneic Hematopoietic Stem Cell Transplantation. Front. Immunol. 7:507.

Ohteki, T., H. Yoshida, T. Matsuyama, G.S. Duncan, T.W. Mak, and P.S. Ohashi. 1998. The transcription factor interferon regulatory factor 1 (IRF-1) is important during the maturation of natural killer 1.1+ T cell receptor-alpha/beta+ (NK1+ T) cells, natural killer cells, and intestinal intraepithelial T cells. J Exp Med 187:967-972.

Orange, J.S., and C.A. Biron. 1996. Characterization of early IL-12, IFN-alphabeta, and TNF effects on antiviral state and NK cell responses during murine cytomegalovirus infection. J Immunol 156:4746-4756.

Patel, A. A. et al., J Exp Med. 2017 Jul 3;214(7):1913-1923.

Patidar, M., N. Yadav, and S.K. Dalai. 2016. Interleukin 15: A key cytokine for immunotherapy. Cytokine Growth Factor Rev 31:49-59.

Pixley FJ, Stanley ER. 2004 Trends Cell Biol. 2004 Nov;14(ll):628-38).

Plotkin, S. 2015. The history of vaccination against cytomegalovirus. Med Microbiol Immunol 204:247-254.

Presti, R.M., D.L. Popkin, M. Connick, S. Paetzold, and H.W.t. Virgin. 2001. Novel cell type-specific antiviral mechanism of interferon gamma action in macrophages. J Exp Med 193:483-496.

Puttur, F., M. Francozo, G. Solmaz, C. Bueno, M. Lindenberg, M. Gohmert, M. Swallow, D. Tufa, R. Jacobs, S. Lienenklaus, A.A. Kuhl, L. Borkner, L. Cicin-Sain, B. Holzmann, H. Wagner, L. Berod, and T. Sparwasser. 2016. Conventional Dendritic Cells Confer Protection against Mouse Cytomegalovirus Infection via TLR9 and MyD88 Signaling. Cell Rep 17:1113-1127.

Roth, P., A. Bartocci, and E.R. Stanley. 1997. Lipopolysaccharide induces synthesis of mouse colony-stimulating factor-1 in vivo. J Immunol 158:3874-3880.

Salazar-Mather, T.P., C.A. Lewis, and C.A. Biron. 2002. Type I interferons regulate inflammatory cell trafficking and macrophage inflammatory protein 1alpha delivery to the liver. J Clin Invest 110:321-330.

Salzberger, B., R.A. Bowden, R.C. Hackman, C. Davis, and M. Boeckh. 1997. Neutropenia in allogeneic marrow transplant recipients receiving ganciclovir for prevention of cytomegalovirus disease: risk factors and outcome. Blood 90:2502-2508.

Sarrazin, S., T. Fukao, N. Mossadegh, A. Aziz, F. Mourcin, L. vanHille, L.M. Kelly, P. Kastner, S. Chan, E. Duprez, C. Otto, and M.H. Sieweke. 2009. MafB restricts M-CSF dependent myeloid commitment divisions of hematopoietic stem cells Cell 138:1-14.

Sarrazin, S., and M. Sieweke. 2011. Integration of cytokine and transcription factor signals in hematopoietic stem cell commitment. Semin Immunol 23:326-334.

Serafini, N., C.A. Vosshenrich, and J.P. Di Santo. 2015. Transcriptional regulation of innate lymphoid cell fate. Nat Rev Immunol 15:415-428.

Smith, L.M., J.N. Tonkin, M.A. Lawson, and G.R. Shellam. 2004. Isolates of cytomegalovirus (CMV) from the black rat Rattus rattus form a distinct group of rat CMV. J Gen Virol 85:1313-1317.

Soucie, E.L., Z. Weng, L. Geirsdottir, K. Molawi, J. Maurizio, R. Fenouil, N. Mossadegh-Keller, G. Gimenez, L. VanHille, M. Beniazza, J. Favret, C. Berruyer, P. Perrin, N. Hacohen, J.C. Andrau, P. Ferrier, P. Dubreuil, A. Sidow, and M.H. Sieweke. 2016. Lineage-specific enhancers activate self-renewal genes in macrophages and embryonic stem cells. Science 351:aad5510.

Soudja, S.M., A.L. Ruiz, J.C. Marie, and G. Lauvau. 2012. Inflammatory monocytes activate memory CD8(+) T and innate NK lymphocytes independent of cognate antigen during microbial pathogen invasion. Immunity 37:549-562.

Stanley, E.R. and Chitu, V.: Cold Spring Harb Perspect Biol 2014;6:a021857

Strobl, B., I. Bubic, U. Bruns, R. Steinborn, R. Lajko, T. Kolbe, M. Karaghiosoff, U. Kalinke, S. Jonjic, and M. Muller. 2005. Novel functions of tyrosine kinase 2 in the antiviral defense against murine cytomegalovirus. J Immunol 175:4000-4008.

Tabeta, K., P. Georgel, E. Janssen, X. Du, K. Hoebe, K. Crozat, S. Mudd, L. Shamel, S. Sovath, J. Goode, L. Alexopoulou, R.A. Flavell, and B. Beutler. 2004. Toll-like receptors 9 and 3 as essential components of innate immune defense against mouse cytomegalovirus infection. Proc Natl Acad Sci USA 101:3516-3521.

Tagliani, E., C. Shi, P. Nancy, C.S. Tay, E.G. Pamer, and A. Erlebacher. 2011. Coordinate regulation of tissue macrophage and dendritic cell population dynamics by CSF-1. JExp Med 208:1901-1916.

Ullah, M.A., G.R. Hill, and S.K. Tey. 2016. Functional Reconstitution of Natural Killer Cells in Allogeneic Hematopoietic Stem Cell Transplantation. Front Immunol 7:144.

Ushach, I., and A. Zlotnik. 2016. Biological role of granulocyte macrophage colony-stimulating factor (GM-CSF) and macrophage colony-stimulating factor (M-CSF) on cells of the myeloid lineage. J Leukoc Biol 100:481-489.

Vosshenrich, C.A., and J.P. Di Santo. 2013. Developmental programming of natural killer and innate lymphoid cells. Curr Opin Immunol 25:130-138.

Wang, C.Y., Q.S. Li, H.H. Zhang, J.J. Su, J.J. Zhang, H. Li, F.J. Jia, and L.Y. Ge. 1993. [Detection of CAg in CSF of 231 cerebral cysticercosis patients]. Zhongguo Ji Sheng Chong Xue Yu Ji Sheng Chong Bing Za Zhi 11:276-278.

WHO Technical Report Series (1994) no. 840, p. 10.

Zaia, J.A. 1990. Viral infections associated with bone marrow transplantation. Hematol Oncol Clin North Am 4:603-623.

Zucchini, N., G. Bessou, S.H. Robbins, L. Chasson, A. Raper, P.R. Crocker, and M. Dalod. 2008. Individual plasmacytoid dendritic cells are major contributors to the production of multiple innate cytokines in an organ-specific manner during viral infection. Int Immunol 20:45-56.

## Claims

1. An agonist of the CSF1-receptor for use in the prophylaxis and/or treatment of viral infections in a disorder **characterized by** a state of immunosuppression in a subject.

2. The agonist of the CSF1-receptor for use according to claim 1, wherein said viral infection is activation of a latent virus in said subject.

3. The agonist of the CSF1-receptor for use according to claim 2, wherein said subject had been diagnosed as having a latent virus infection and wherein the use of the agonist of the CSF 1-receptor is prophylactic use.

4. The agonist of the CSF1-receptor for use according to claims 2 or 3, wherein said latent virus is selected from CMV, HMPV, HBoV, HCoV-NL63, HCoV-HKU1, HHV-6, HHV-7, BK virus, JC virus, KI virus, WU virus, Epstein-Barr virus and adenovirus (ADV).

5. The agonist of the CSF1-receptor for use according to any one of claims 2 to 4, wherein the state of immunosuppression is induced.

6. The agonist of the CSF1-receptor for use according to claim 5, wherein the subject has been diagnosed as being a carrier of a latent virus.

7. The agonist of the CSF 1-receptor for use according to any one of claims 1 to 6, wherein the disorder is hematopoietic stem cell transplantation.

8. The agonist of the CSF 1-receptor for use according to any one of claims 2 to 7, wherein the latent virus is CMV.

9. The agonist of the CSF1-receptor for use according to claim 1, wherein said viral infection is an accidental infection.

10. The agonist of the CSF1-receptor for use according to claim 1, wherein said viral infection is derived from the transfer of donor material to said subject.

11. The agonist of the CSF 1-receptor for use according to claim 10, wherein the donor had been diagnosed as having a latent virus infection and wherein the use is prophylactic.

12. The agonist of the CSF1-receptor for use according to any one of claims 1 to 11, wherein the agonist is a M-CSF polypeptide, IL-34 polypeptide or a functional equivalent of said M-CSF polypeptide or IL-34 polypeptide.

13. The agonist of the CSF1-receptor for use according to any one of the preceding claims, wherein said agonist of the CSF1-receptor shows a specific activity with an ED50 in the range of 0.5 to 2 ng/ml in a proliferation assay using mouse M-NSF-60 cells.

14. A pharmaceutical composition comprising an agonist of the CSF1-receptor, for use in the prophylaxis and/or treatment of viral infections in a disorder **characterized by** a state of immunosuppression in a subject.

15. The pharmaceutical composition according to claim 14, wherein the pharmaceutical composition is to be administered during the state of immune suppression.
